# EUROPEAN PATENT SPECIFICATION

(11) **EP 3 415 637 B1**
(45) Date of publication and mention of the grant of the patent: **10.05.2023**
(21) Application number: 17750348.9
(22) Date of filing: 10.02.2017
(51) Int. Cl.: C12Q 1/6886, C12N 15/09

(54) **METHOD FOR DIFFERENTIATING CONTRACTION OF ESOPHAGEAL BASALOID CARCINOMA**
VERFAHREN ZUR UNTERSCHEIDUNG DER KONTRAKTION EINES ÖSOPHAGEALEN BASALOIDEN KARZINOMS
PROCÉDÉ D'IDENTIFICATION DE DÉVELOPPEMENT DE CARCINOME BASALOÏDE CELLULAIRE DE L' SOPHAGE

(30) Priority: 10.02.2016 JP 2016024129
(43) Date of publication of application: 19.12.2018
(73) Proprietor: Fukushima Medical University, Fukushima 960-1295 (JP); Nippon Gene Co., Ltd, Chiyoda-ku, Tokyo 101-0054 (JP); Medicrome, Inc., Fukushima-shi, Fukushima 960-8031 (JP)
(72) Inventor: WATANABE, Shinya, Fukushima-shi Fukushima 960-1295 (JP); IMAI, Jun-ichi, Fukushima-shi Fukushima 960-1295 (JP); ITO, Emi, Fukushima-shi Fukushima 960-1295 (JP); MORISAWA, Gaku, Fukushima-shi Fukushima 960-1295 (JP); TADA, Takeshi, Fukushima-shi Fukushima 960-1295 (JP); GOTO, Mitsukazu, Fukushima-shi Fukushima 960-1295 (JP); KOGURE, Michihiko, Fukushima-shi Fukushima 960-1295 (JP); TOGASHI, Reiko, Tokyo 151-0051 (JP); NISHIKAWA, Akira, Toyama-shi Toyama 930-0834 (JP); MATSUKURA, Susumu, Yokohama-shi Kanagawa 230-0046 (JP)
(74) Representative: D Young & Co LLP
(86) International application number: PCT/JP2017/004870
(87) International publication number: WO 2017/138627

(56) References cited:
- WO-A1-2006/118308
- WO-A1-2009/142257
- MARIO SARBIA ET AL: "Short Communication Expression of Bcl-2 and Amplification of c-myc Are Frequent in Basaloid Squamous Cell Carcinomas of the Esophagus", AM. J. PATHOL., vol. 155, no. 4, 1 October 1999 (1999-10-01), pages 1027-1032, XP055620128,
- Anonymous: "Data Sheet. GeneChip? Human Genome U95 Set", , 1 January 2003 (2003-01-01), pages 1-2, XP055381769, Retrieved from the Internet: URL:http://tools.thermofisher.com/content/ sfs/brochures/hgu95_datasheet.pdf [retrieved on 2017-06-14]
- "Array Design for the GeneChip - HUMAN GENOME U133 SET", INTERNET CITATION, 1 January 2001 (2001-01-01), pages 1-8, XP008116589, Retrieved from the Internet: URL:www.affymetrix.com [retrieved on 1077-01-01]
- TAKESHI TADA ET AL: "A novel gene expression scoring system for accurate diagnosis of basaloid squamous cell carcinoma of the esophagus", INTERNATIONAL JOURNAL OF ONCOLOGY, vol. 51, no. 3, 19 September 2017 (2017-09-19), pages 877-886, XP055619644, GR ISSN: 1019-6439, DOI: 10.3892/ijo.2017.4075
- TAKESHI TADA: "Morateki Idenshi Hatsugen Kaiseki o Mochiita Shokudo Ruikitei Saibogan Tokuiteki Idenshi no Tansaku", Japan Society of Clinical Oncology, 2015, pages 13-1, XP9513107,
- TAKESHI TADA et al.: "Morateki Idenshi Hatsugen Kaiseki o Katsuyo shita Shokudo Ruikitei Saibogan no Shindan Seido no Kojo", Journal of Japan Surgical Society, vol. 115, no. 2, Spec. Issue, 2014, page 679, XP009513172, ISSN: 0301-4894
- TAKESHI TADA et al.: "2. Shokudo Ruikitei Saibogan ni Okeru Tokuiteki Idenshi no Tansaku", Fukushima Igaku Zasshi = Fukushima Medical Journal, vol. 65, no. 3, 2015, page 117, XP009513169,
- SCHAEFER I.M. et al.: "Common Genomic Aberrations in Basaloid Squamous Cell Carcinoma and Carcinosarcoma of the Esophagus Detected by CGH and Array CGH", Am J Clin Pathol, vol. 135, no. 4, 2011, pages 579-586, XP055406529,
- SATO-KUWABARA Y. et al.: "Comparative analysis of basaloid and conventional squamous cell carcinomas of the esophagus: prognostic relevance of clinicopathological features and protein expression.", Tumor Biol, vol. 37, 9 December 2015 (2015-12-09), pages 6691-6699, XP036218984,
- BELLIZZI A.M. et al.: "Basaloid Squamous Cell Carcinoma of the Esophagus: Assessment for High-Risk Human Papillomavirus and Related Molecular Markers", Am J Surg Pathol, vol. 33, no. 11, 2009, pages 1608-1614, XP009512509, ISSN: 0147-5185, DOI: 10.1097/PAS.0b013e3181b46fd4
- IMAMHASAN A. et al.: "Immunohistochemical and oncogenetic analyses of the esophageal basaloid squamous cell carcinoma in comparison with conventional squamous cell carcinomas", Human Pathology, vol. 43, 2012, pages 2012-2023, XP055406572,
- AKAGI I. et al.: "Basaloid Squamous Cell Carcinoma of the Esophagus : Report of Two Cases", J Nippon Med Sch, vol. 75, no. 6, 2008, pages 354-360, XP055406574,
- TAKESHI TADA et al.: "Idenshi Hatsugen Scoring System o Mochiita Shokudo Ruikitei Saibogan no Kanbetsu Shindanho no Kochiku", Fukushima Medical University Repository, 24 March 2016 (2016-03-24), XP055406582, Retrieved from the Internet: URL:http://ir.fmu.ac.jp/dspace/handle/ 123456789/545 [retrieved on 2017-03-23]

## Description

### Technical Field

The present invention relates to a method for differentiating the presence of basaloid squamous cell carcinoma of the esophagus.

### Background Art

Basaloid squamous cell carcinoma (BSC) is a tumor in the laryngeal pharynx region which was first reported by Wain et al. (Non Patent Literature 1). In addition to the laryngeal pharynx region, the development of the tumor in the esophagus, lung, anus, uterine cervix, penis, urinary bladder, etc. has been reported.

Basaloid squamous cell carcinoma of the esophagus (hereinafter often referred to as"BSCE") is a relatively rarecancer which is classified into special histopathologic type esophageal cancers excluding esophageal squamous cell carcinoma and esophageal adenocarcinoma among esophageal cancers. Reportedly, BSCE accounts for 1.0% to 8.7% in Japan (Non Patent Literature 2 to 11) and 0.4% to 11.3% in foreign countries (Non Patent Literature 12 to 20) with respect to all esophageal cancers. Histopathological characteristics of BSCE are noted as solid nest with comedo-type necrosis, cribriform pattern and pseudoacini formation, ductal differentiation, small nests with a microcystic and/or trabecular pattern, hyaline-like material deposition, coexistence of invasive SCC component, etc. However, since BSCE has various histological types, it is usually difficult to provide a differential diagnosis. In particular, it is necessary to correctly differentiate BSCE from adenoid cystic carcinoma, small cell carcinoma, poorly differentiated squamous cell carcinoma, and adenosquamous carcinoma. In addition, it is said that making a diagnosis of BSCE by preoperative endoscopic biopsy is very difficult, resulting in a diagnostic accuracy rate of only 0% to 10%. In the past, there have been reports about a BSCE diagnosis by immunostaining (Non Patent Literature 3, 6, 10, 12, 14, and 15) and PCR (Non Patent Literature 21 or 22). However, specificity was not shown in either case.

The prognosis of BSCE is still controversial. Meanwhile, it is said that proliferative capacity and the cytological malignancy of BSCE are higher than those of esophageal squamous cell carcinoma. It has also been reported that progressive BSCE has poor prognosis (Non Patent Literature 23). It is considered necessary to establish a new multidisciplinary therapy, in addition to surgery and radiation therapy, in order to improve the prognosis. However, due to the rarity and difficulty of preoperative diagnosis of BSCE, no characteristic therapy can be selected. At present, BSCE is treated in accordance with the treatment for a common type of esophageal squamous cell carcinoma.

Therefore, although it is indispensable to improve the preoperative diagnostic accuracy of BSCE, the existing methods cannot solve the above problems. There is a demand for the establishment of a BSCE diagnosis method with high accuracy.

### Citation List

### Non Patent Literature

Non Patent Literature 1: Wain S.L., et al., 1986, Hum Pathol 17:1158-66
Non Patent Literature 2: Takubo K., et al., 1991, ActaPathologica Japonica 41:59-64
Non Patent Literature 3: Abe K., et al., 1996, American Journal of Surgical Pathology 20:453-461
Non Patent Literature 4: Koide N., et al., 1997, Surgery Today 27:685-691
Non Patent Literature 5: Kawahara K., et al., 2001, Report of a case. Surgery Today 31:655-659
Non Patent Literature 6: Ohashi K., et al., 2003, Pathology Research and Practice 199:713-721
Non Patent Literature 7: Yoshioka S., et al., 2004, Japanese Journal of Gastroenterological Surgery 37:290-295
Non Patent Literature 8: Kobayashi Y., et al., 2009, Diseases of the Esophagus 22:231-238
Non Patent Literature 9: Saito S., et al., 2009, Esophagus 6:177-181
Non Patent Literature 10: Imamhasan A., et al., 2012, Human Pathology 43:2012-2023
Non Patent Literature 11: Tachimori Y., et al., 2008, Esophagus 12:130-157
Non Patent Literature 12: Sarbia M., et al., 1997, Cancer 79:1871-1878
Non Patent Literature 13: Zhang X. H., et al., 1998, World Journal of Gastroenterology 4:397-403
Non Patent Literature 14: Cho K.J., et al., 2000, Histopathology 36:331-340
Non Patent Literature 15: Huang Z., et al., 2001, Chinese medical journal 114:1084-1088
Non Patent Literature 16: Lam K.Y., et al., 2001, Journal of Pathology 195:435-442
Non Patent Literature 17: Klaase J.M., et al., 2003, Annals of Surgical Oncology 10:261-267
Non Patent Literature 18: Li T.J., et al., 2004, Archives of Pathology and Laboratory Medicine 128:1124-1130
Non Patent Literature 19: Chen S.B., et al., 2012, Journal of Cancer Research and Clinical Oncology 138:1165-1171
Non Patent Literature 20: Zhang B.H., et al., 2013, Asian Pacific Journal of Cancer Prevention 14:1889-1894
Non Patent Literature 21: Sarbia M., et al., 1999, American Journal of Pathology 155:1027-1032
Non Patent Literature 22: Bellizzi A.M., et al., 2009, American Journal of Surgical Pathology 33:1608-1614
Non Patent Literature 23: Arai T., et al., 2011, Esophagus 8:169-177

### Summary of Invention

The present invention relates to a method for assisting differentiating contraction of basaloid squamous cell carcinoma of the esophagus, comprising:
a measurement step of measuring expression levels of 5 genes comprising nucleotide sequences shown in SEQ ID NOs: 141 to 145, respectively, per unit amount of samples collected from a test subject and a healthy subject or a group of healthy subjects, thereby obtaining measurement values thereof;
a calculation step of, based on the measurement values obtained in the measurement step, calculating expression ratios for each gene based on the measurement values of the test subject and the healthy subject or the group of healthy subjects, thereby obtaining a sum of the expression ratios, or calculating an average measurement value from the respective measurement values of all of the genes measured in the measurement step for the test subject and the group of healthy subjects; and
a determination step of, based on the values obtained in the calculation step, determining that the test subject is highly likely to be affected with basaloid squamous cell carcinoma of the esophagus in a case in which the sum exceeds a given cutoff value resulting from an ROC curve, or in a case in which the average measurement value of the test subject is statistically significantly larger than the average measurement value of the group of healthy subjects.

The present invention also relates to a reagent for detecting basaloid squamous cell carcinoma of the esophagus, wherein said reagent contains a group of probes consisting of nucleotide sequences shown in SEQ ID NOs: 141 to 145.

The present invention further relates to a kit for detecting basaloid squamous cell carcinoma of the esophagus, wherein said kit contains the reagent for detecting basaloid squamous cell carcinoma of the esophagus according to the invention.

### Technical Problem

An object of the present invention is to develop and provide a method for readily and accurately differentiating an esophageal cancer patient's tumor between BSCE or another esophageal cancer.

### Solution to Problem

In order to attain the above object, the present inventors obtained gene expression profiles for cancer tissues and normal tissues excised from a plurality of esophageal cancer patients using a comprehensive gene expression analysis technique and found a group of 70 kinds of genes having expression levels that significantly differ between BSCE and other esophageal cancers or normal sites. Accordingly, the present inventors developed a method whereby it is possible to readily and accurately differentiate BSCE from other esophageal cancers by determining the expression levels of the above genes in samples from test subjects and healthy subjects. The present invention is based on the above findings.

### Advantageous Effects of Invention

According to the method of the invention for differentiating contraction of BSCE using the BSCE marker, it is possible to correctly differentiate a tumor of an esophageal cancer patient as BSCE or other esophageal cancer.

### Brief Description of Drawings

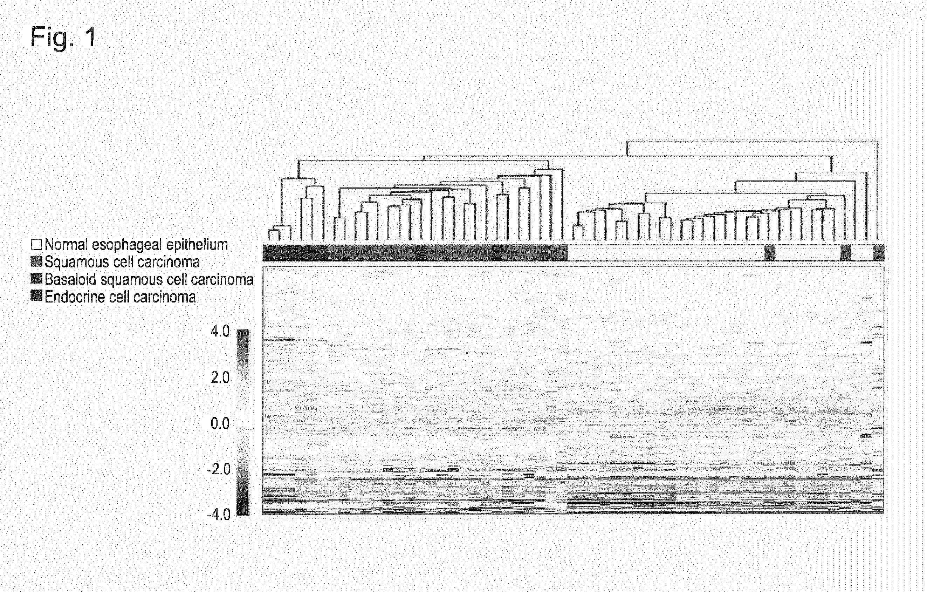

[Figure 1] Figure 1 shows the results of classification by cluster analysis based on the expression profiles of the group of 70 kinds of genes for differentiating BSCE (BSCE markers) of the present invention. The dendrogram in the top was created according to the non-similarity coefficient based on the gene expression patterns of 70 kinds of BSCE markers, and the bar in the middle indicates distinctions of cancer types of individual specimens (including normal tissues). In addition, the diagram (hereinafter referred to as "heat map chart") in the bottom show gene expression patterns.

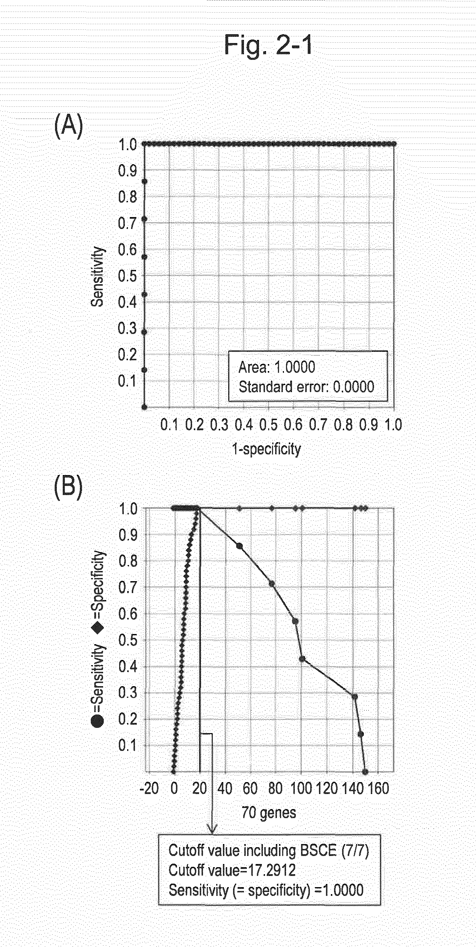

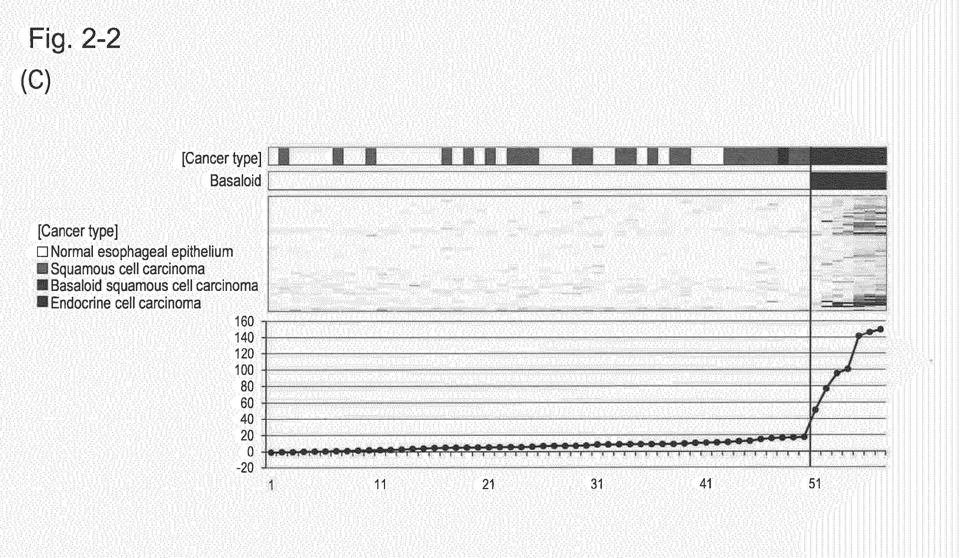

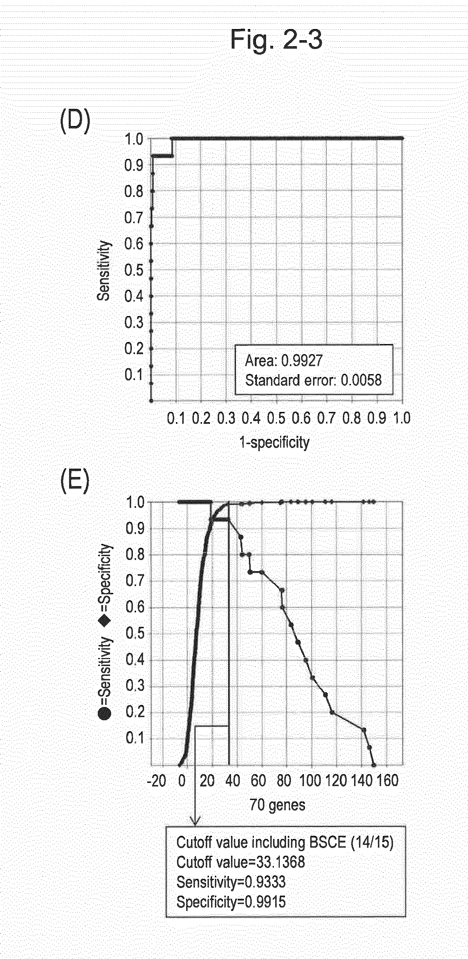

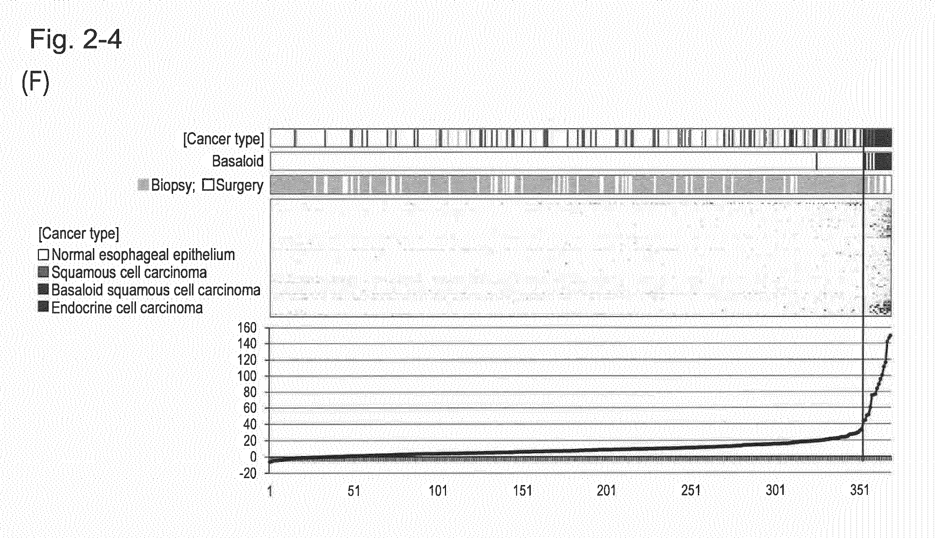

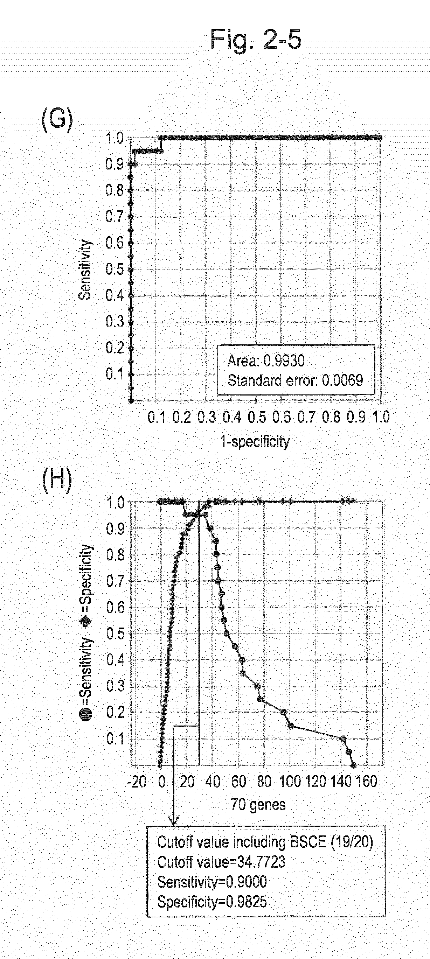

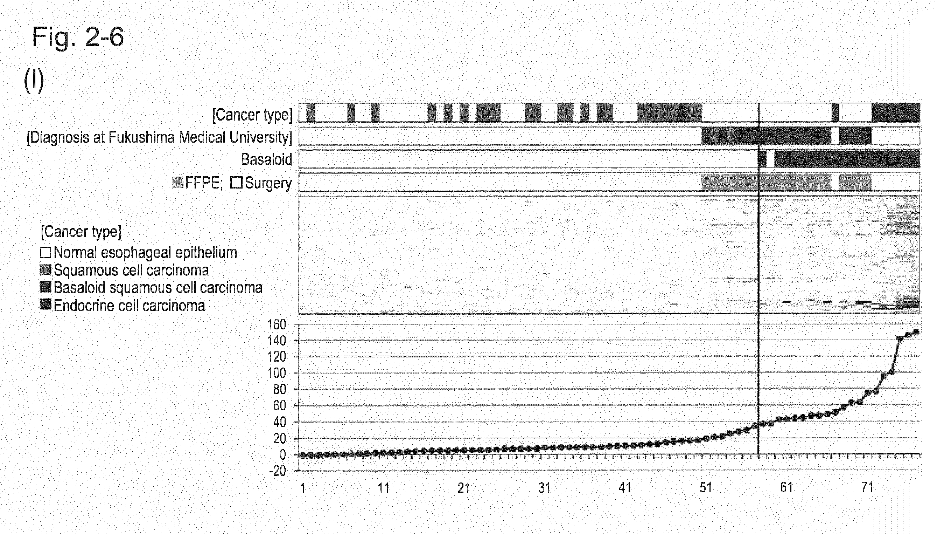

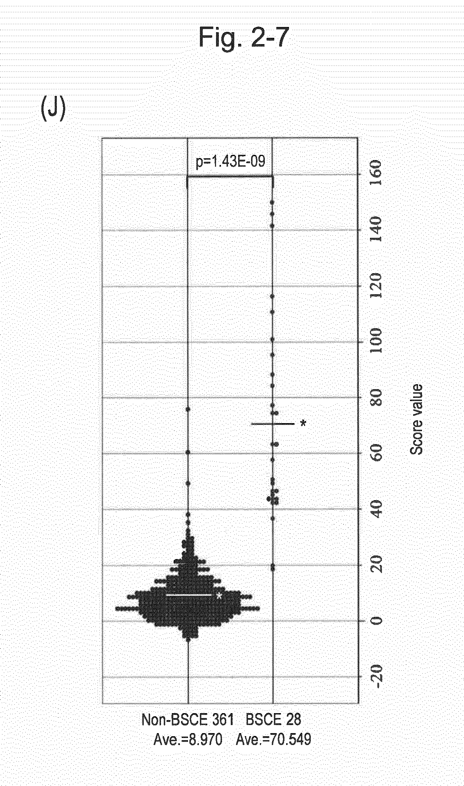

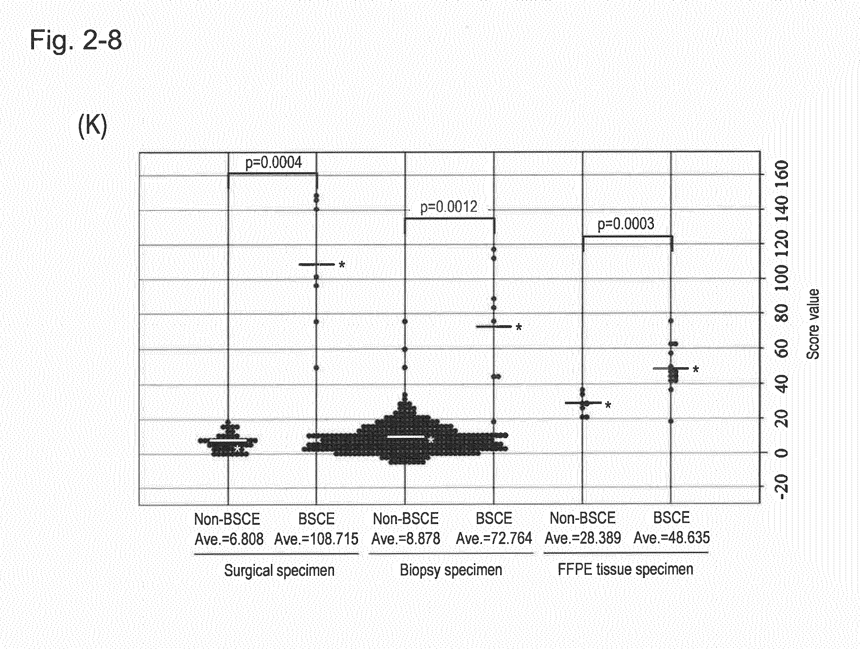

[Figure 2-1] Figure 2-1 (A) shows an ROC curve of 70 kinds of BSCE markers. The vertical axis represents "sensitivity" and the horizontal axis represents "1-specificity" (the same applies hereinafter to figures showing ROC curves). Figure 2-1 (B) shows a sensitivity-specificity curve obtained with the use of BSCE markers of 70 genes. The vertical axis represents "sensitivity" or "specificity" and the horizontal axis represents the sum of logarithmic transformed relative expression ratios of the 70 kinds of BSCE markers (expression scores of 70 genes).
[Figure 2-2] Figure 2-2 (C) shows a heat map chart obtained by aligning the specimens in the ascending order of the expression scores of 70 genes in each specimen (middle) and a graph plotting the values of the expression scores of 70 genes (bottom). The vertical line in the figure represents a cutoff value. In addition, of two bars in the top, the upper bar denoted by "Cancer type" indicates distinctions of cancer types (including normal tissues) and the lower bar denoted by "Basaloid" indicates distinction between BSCE and non-BSCE (colored portions correspond to BSCE).
[Figure 2-3] Figure 2-3 (D) shows an ROC curve created by combining the expression scores of 70 genes for 57 surgical specimens and the expression scores of 70 genes for 312 biopsy specimens obtained in Example 1. Figure 2-3 (E) shows a sensitivity-specificity curve from 57 surgical specimens and 312 biopsy specimens obtained with the use of the 70 kinds of BSCE markers. The vertical axis represents "sensitivity" or "specificity" and the horizontal axis represents the "expression scores of 70 genes."
[Figure 2-4] Figure 2-4 (F) shows a heat map chart obtained by aligning 312 biopsy specimens and 57 surgical specimens together in the ascending order of the expression scores of 70 genes (middle) and a graph plotting the values of the expression scores of 70 genes (bottom). The vertical line in the figure indicates a cutoff value. In addition, of three bars in the top, the first bar denoted by "Cancer type" indicates distinctions of cancer types (including normal tissues), the second bar denoted by "Basaloid" indicates distinction between BSCE and non-BSCE (colored portions correspond to BSCE), and the third bar denoted by "Biopsy; Surgery" indicates distinction between surgical specimens and biopsy specimens.
[Figure 2-5] Figure 2-5 (G) shows an ROC curve created by combining the expression scores of 70 genes for 57 surgical specimens and the expression scores of 70 genes for 20 FFPE tissue specimens. Figure 2-5 (H) shows a sensitivity-specificity curve from 57 surgical specimens and 20 FFPE tissue specimens obtained with the use of the 70 kinds of BSCE markers. The vertical axis represents "sensitivity" or "specificity" and the horizontal axis represents the "expression score of 70 genes."
[Figure 2-6] Figure 2-6 (I) shows a heat map chart obtained by aligning 20 FFPE tissue specimens and 57 surgical specimens together in the ascending order of the expression scores of 70 genes (middle) and a graph plotting the values of the expression scores of 70 genes (bottom). The vertical line in the figure represents a cutoff value. In addition, of four bars in the top, the top bar denoted by "Cancer type" indicates distinctions of cancer types (including normal tissues), the second bar denoted by "Diagnosis at Fukushima Medical University" indicates distinctions of cancer types as a result of pathological diagnosis of FFPE tissue specimens at Fukushima Medical University, the third bar denoted by "Basaloid" indicates distinction between BSCE and non-BSCE (colored portions correspond to BSCE), and the bottom bar denoted by "FFPE; Surgery" indicates distinction between surgical specimens and FFPE tissue specimens.
[Figure 2-7] Figure 2-7 (J) is a group scatter diagram according to the expression scores of 70 genes for BSCE markers when surgical specimens, biopsy specimens, and FFPE tissue specimens are all combined. In the figure, the horizontal line with an asterisk represents an average value (the same applies to the figures described below).
[Figure 2-8] Figure 2-8 (K) is a group scatter diagram created separately for each specimen type.

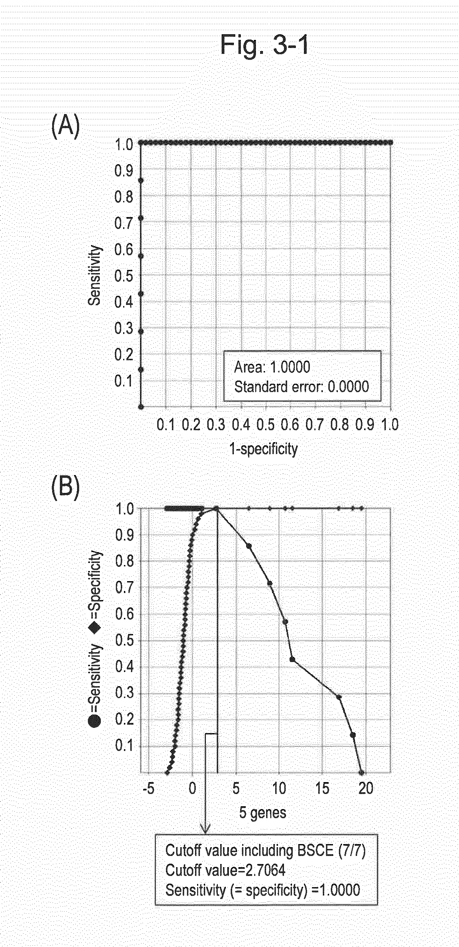

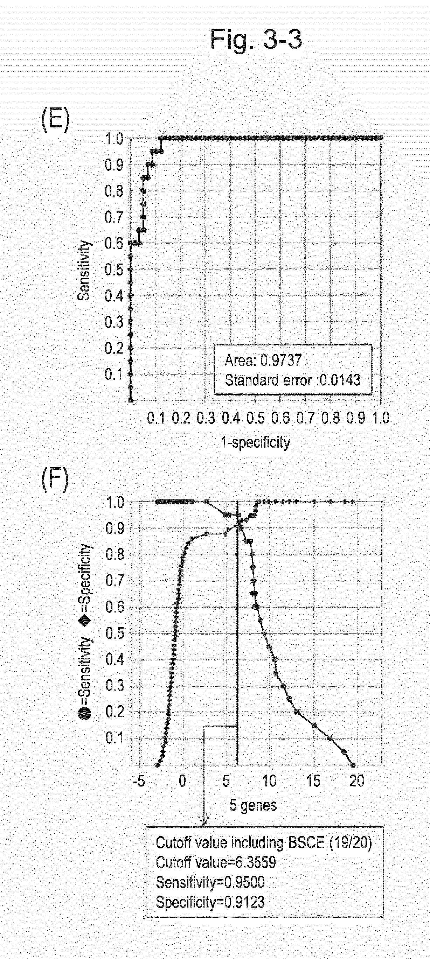

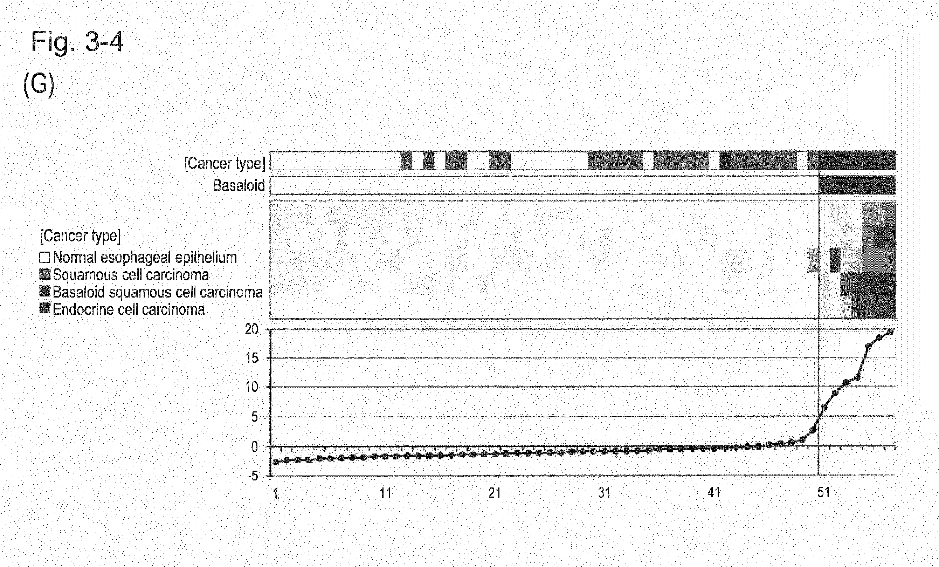

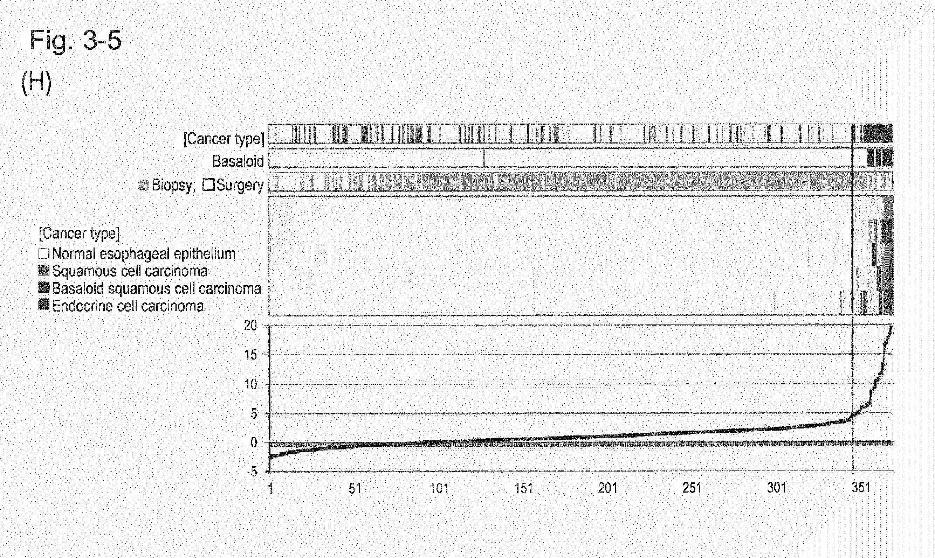

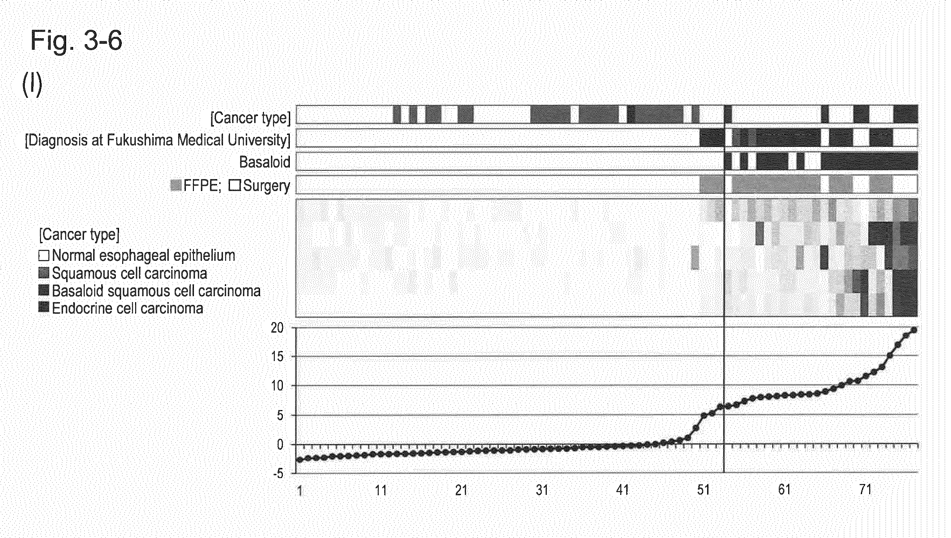

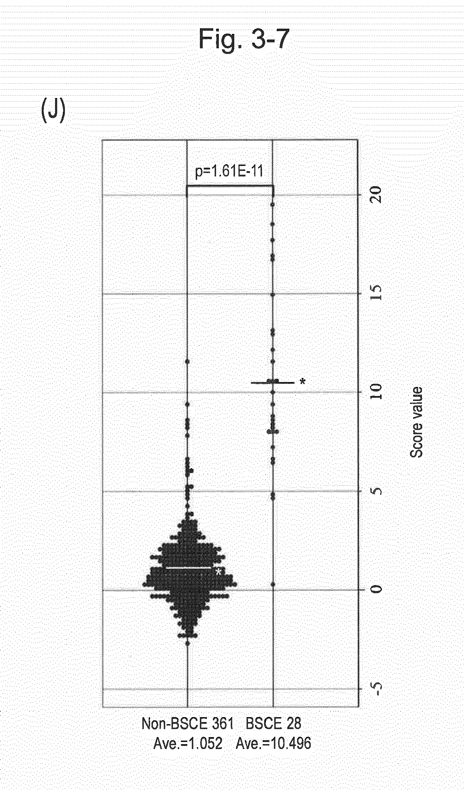

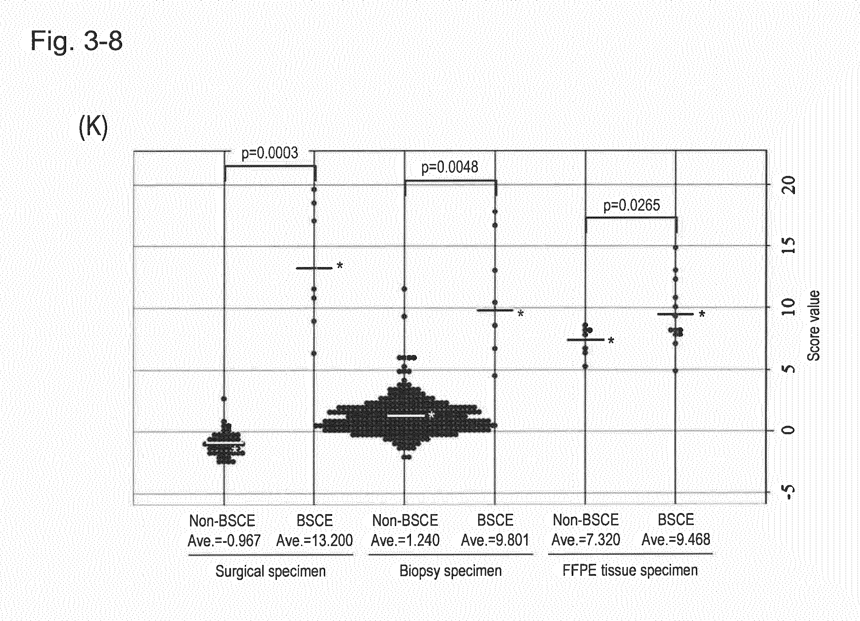

[Figure 3-1] Figure 3-1 (A) shows an ROC curve created according to the expression scores of 5 genes (the sum of logarithmic transformed relative expression ratios of 5 kinds of BSCE markers) from 57 surgical specimens obtained in Example 1 regarding 5 kinds of BSCE markers represented by NOs: R-1 to R-5 in Table 1. Figure 3-1 (B) shows a sensitivity-specificity curve from 57 surgical specimens obtained with the use of the 5 kinds of BSCE markers. The vertical axis represents "sensitivity" or "specificity" and the horizontal axis represents "the sum of logarithmic transformed relative expression ratios of the 5 kinds of BSCE markers (expression scores of 5 genes)."

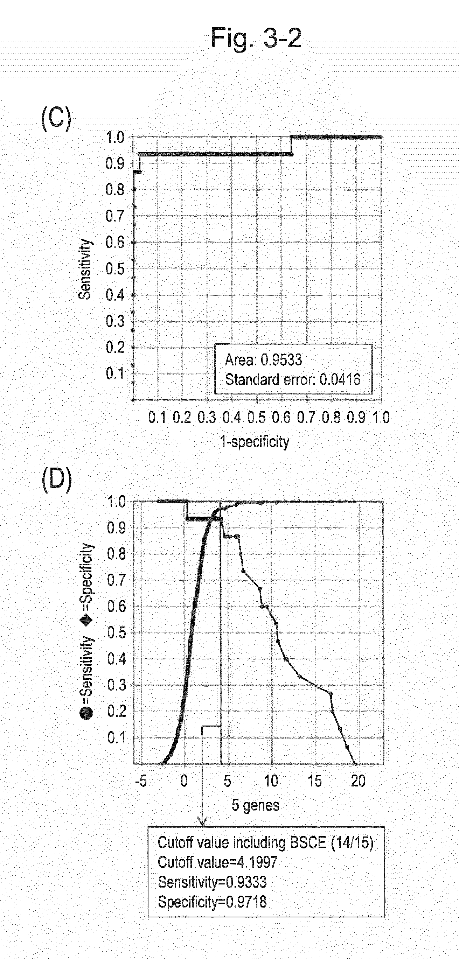

[Figure 3-2] Figure 3-2 (C) shows an ROC curve created from the expression scores of 5 genes for 57 surgical specimens and 312 biopsy specimens obtained in Example 1 regarding 5 kinds of BSCE markers represented by NOs: R-1 to R-5. Figure 3-2 (D) shows a sensitivity-specificity curve from 57 surgical specimens and 312 biopsy specimens obtained with the use of the 5 kinds of BSCE markers. The vertical axis represents "sensitivity" or "specificity" and the horizontal axis represents the "expression scores of 5 genes."
[Figure 3-3] Figure 3-3 (E) shows an ROC curve created from the expression scores of 5 genes for 57 surgical specimens and 20 FFPE tissue specimens obtained in Example 1 regarding 5 kinds of BSCE markers represented by NOs: R-1 to R-5. Figure 3-3 (F) shows a sensitivity-specificity curve from 57 surgical specimens and 20 FFPE tissue specimens obtained with the use of the 5 kinds of BSCE markers. The vertical axis represents "sensitivity" or "specificity" and the horizontal axis represents the "expression scores of 5 genes."
[Figure 3-4] Figure 3-4 (G) shows a heat map chart obtained by re-aligning 57 surgical specimens obtained in Example 1 in the ascending order of the expression scores of 5 genes (middle) and a graph plotting the values of the expression scores of 5 genes (bottom) regarding the expression scores of 5 genes for 5 kinds of BSCE markers represented by NOs: R-1 to R-5. The vertical line in the figure represents a cutoff value. In addition, of two bars in the top, the upper bar denoted by "Cancer type" indicates distinctions of cancer types (including normal tissues) and the lower bar denoted by "Basaloid" indicates distinction between BSCE and non-BSCE (colored portions correspond to BSCE).
[Figure 3-5] Figure 3-5 (H) shows a heat map chart obtained by re-aligning 57 surgical specimens and 312 biopsy specimens obtained in Example 1 in the ascending order of the expression scores of 5 genes (middle) and a graph plotting the values of the expression scores of 5 genes (bottom) regarding the expression scores of 5 genes for 5 kinds of BSCE markers represented by NOs: R-1 to R-5. The vertical line in the figure represents a cutoff value. In addition, of three bars in the top, the first bar denoted by "Cancer type" indicates distinctions of cancer types (including normal tissues), the second bar denoted by "Basaloid" indicates distinction between BSCE and non-BSCE (colored portions correspond to BSCE), and the third bar denoted by "Biopsy; Surgery" indicates distinction between surgical specimens and biopsy specimens.
[Figure 3-6] Figure 3-6 (I) shows a heat map chart obtained by re-aligning 57 surgical specimens and 20 FFPE tissue specimens obtained in Example 1 in the ascending order of the expression scores of 5 genes (middle) and a graph plotting the values of the expression scores of 5 genes (bottom) regarding the expression scores of 5 genes for 5 kinds of BSCE markers represented by NOs: R-1 to R-5. The vertical line in the figure represents a cutoff value. In addition, of four bars in the top, the top bar denoted by "Cancer type" indicates distinctions of cancer types (including normal tissues), the second bar denoted by "Diagnosis at Fukushima Medical University" indicates distinctions of cancer types as a result of pathological diagnosis of FFPE tissue specimens at Fukushima Medical University, the third bar denoted by "Basaloid" indicates distinction between BSCE and non-BSCE (colored portions correspond to BSCE), and the bottom bar denoted by "FFPE; Surgery" indicates distinction between surgical specimens and FFPE tissue specimens.
[Figure 3-7] Figure 3-7(J) is a group diagram for all analyzed specimens obtained using the expression scores of 5 genes for 5 kinds of BSCE markers represented by NOs: R-1 to R-5 when surgical specimens, biopsy specimens, and FFPE tissue specimens are all combined.
[Figure 3-8] Figure 3-8 (K) is a group scatter diagram created separately for each specimen type.

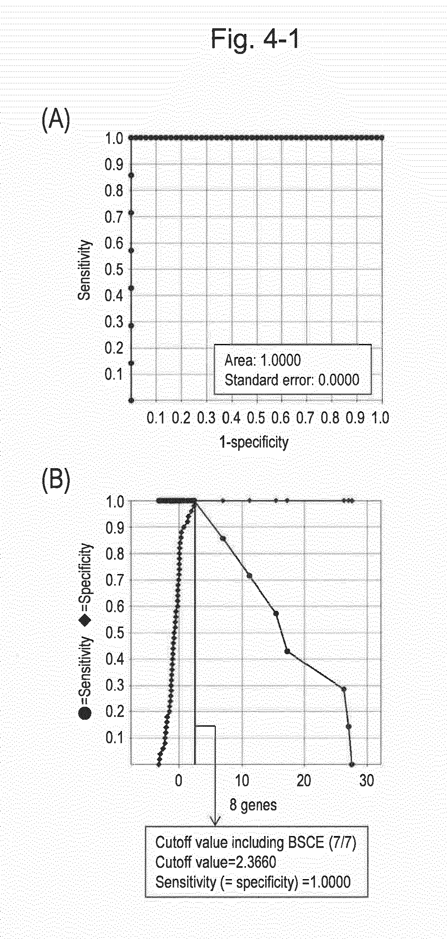

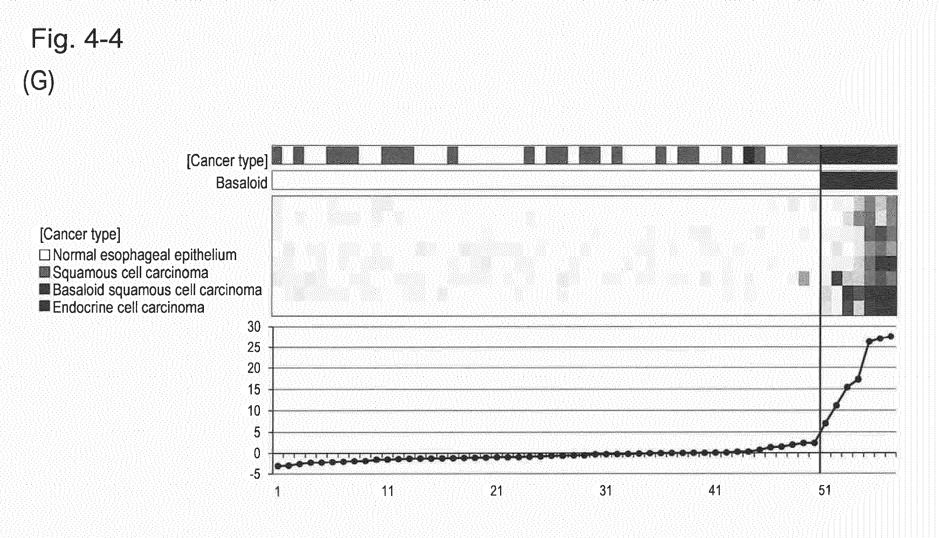

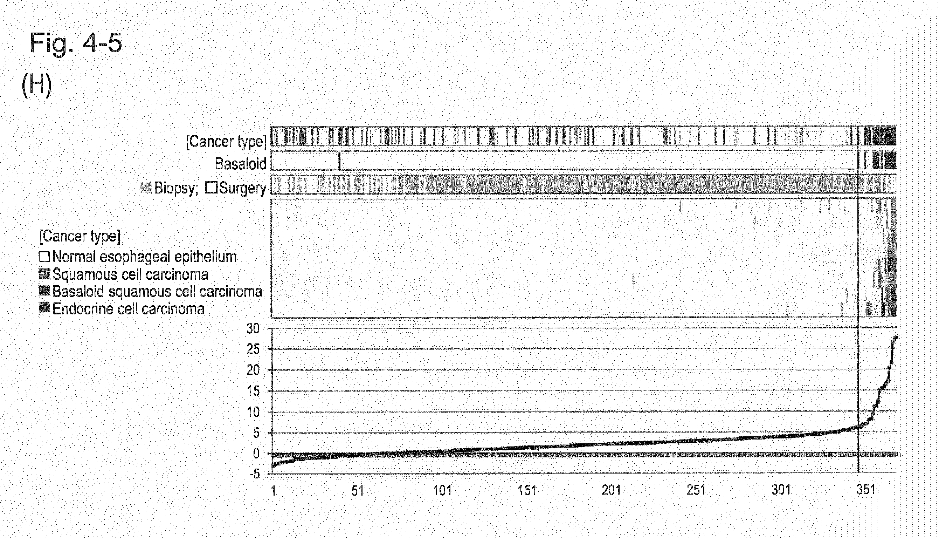

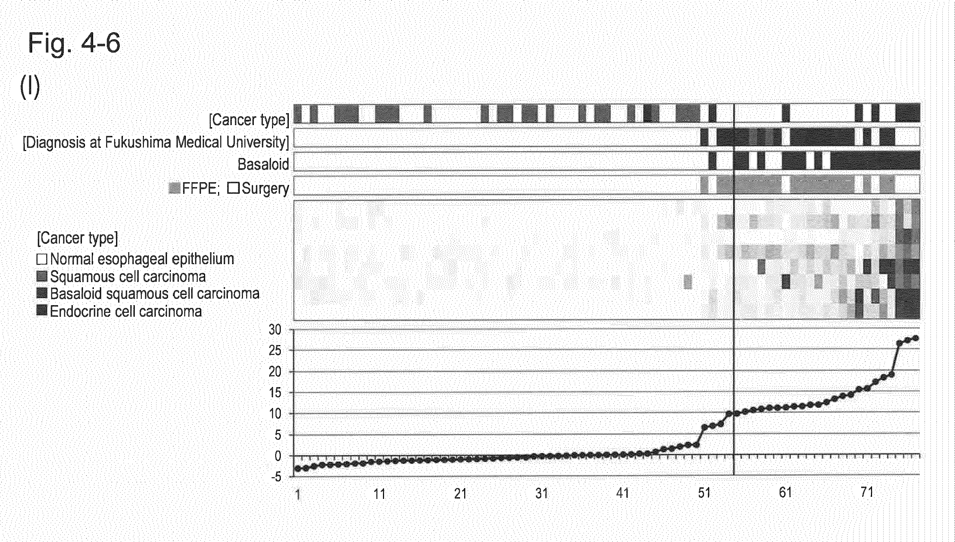

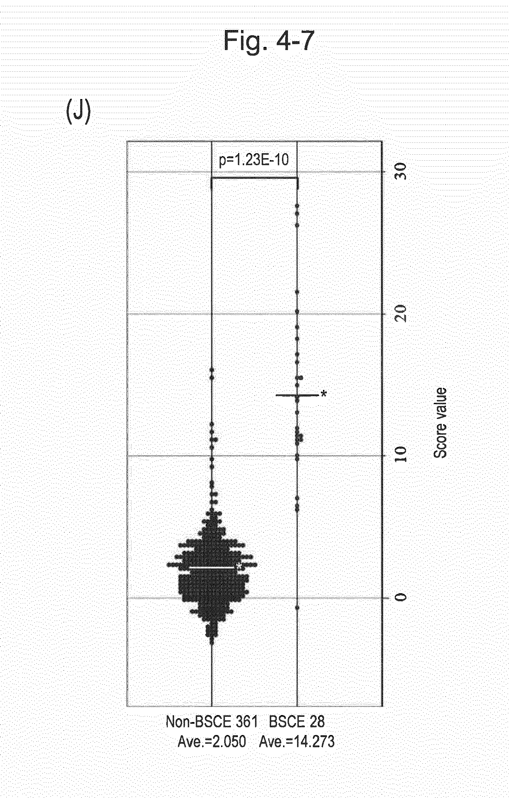

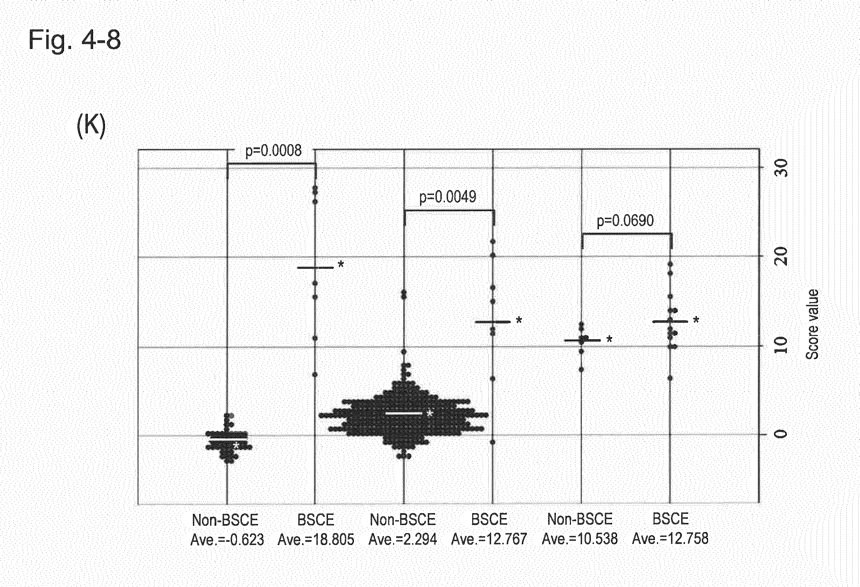

[Figure 4-1] Figure 4-1 (A) shows an ROC curve created from the expression scores of 8 genes (the sum of logarithmic transformed relative expression ratios of 8 kinds of BSCE markers) for 57 surgical specimens obtained in Example 1 regarding 8 kinds of BSCE markers represented by NOs: R-1 to R-8 in Table 1. Figure 4-1 (B) shows a sensitivity-specificity curve from 57 surgical specimens obtained with the use of the 8 kinds of BSCE markers. The vertical axis represents "sensitivity" or "specificity" and the horizontal axis represents "the sum of logarithmic transformed relative expression ratios of the 8 kinds of BSCE markers (expression scores of 8 genes)."

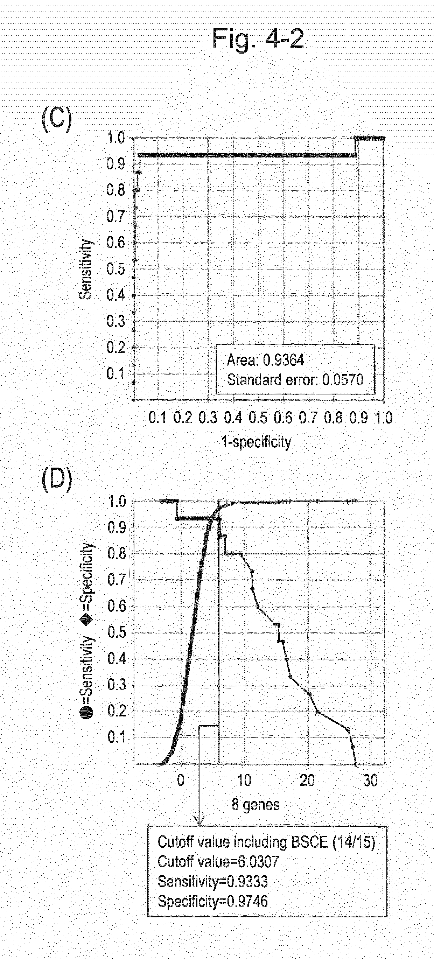

[Figure 4-2] Figure 4-2 (C) shows an ROC curve created from the expression scores of 8 genes for 57 surgical specimens and 312 biopsy specimens obtained in Example 1 regarding 8 kinds of BSCE markers represented by NOs: R-1 to R-8. Figure 4-2 (D) shows a sensitivity-specificity curve from 57 surgical specimens and 312 biopsy specimens obtained with the use of the 8 kinds of BSCE markers. The vertical axis represents "sensitivity" or "specificity" and the horizontal axis represents the "expression scores of 8 genes."

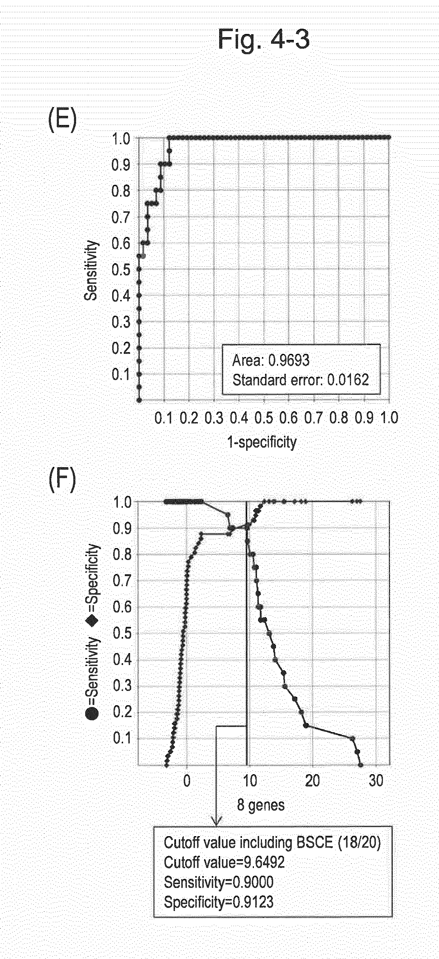

[Figure 4-3] Figure 4-3 (E) shows an ROC curve created from the expression scores of 8 genes for 57 surgical specimens and 20 FFPE tissue specimens obtained in Example 1 regarding 8 kinds of BSCE markers represented by NOs: R-1 to R-8. Figure 4-3 (F) shows a sensitivity-specificity curve from 57 surgical specimens and 20 FFPE tissue specimens obtained with the use of the 8 kinds of BSCE markers. The vertical axis represents "sensitivity" or "specificity" and the horizontal axis represents the "expression scores of 8 genes."
[Figure 4-4] Figure 4-4 (G) shows a heat map chart obtained by re-aligning 57 surgical specimens obtained in Example 1 in the ascending order of the expression scores of 8 genes (middle) and a graph plotting the values of the expression scores of 8 genes (bottom) regarding the expression scores of 8 genes for 8 kinds of BSCE markers represented by NOs: R-1 to R-8. The vertical line in the figure represents a cutoff value. In addition, of two bars in the top, the upper bar denoted by "Cancer type" indicates distinctions of cancer types (including normal tissues) and the lower bar denoted by "Basaloid" indicates distinction between BSCE and non-BSCE (colored portions correspond to BSCE).
[Figure 4-5] Figure 4-5 (H) shows a heat map chart obtained by re-aligning 57 surgical specimens and 312 biopsy specimens obtained in Example 1 in the ascending order of the expression scores of 8 genes (middle) and a graph plotting the values of the expression scores of 8 genes (bottom) regarding the expression scores of 8 genes for 8 kinds of BSCE markers represented by NOs: R-1 to R-8. The vertical line in the figure represents a cutoff value. In addition, of three bars in the top, the first bar denoted by "Cancer type" indicates distinctions of cancer types (including normal tissues), the second bar denoted by "Basaloid" indicates distinction between BSCE and non-BSCE (colored portions correspond to BSCE), and the third bar denoted by "Biopsy; Surgery" indicates distinction between surgical specimens and biopsy specimens.
[Figure 4-6] Figure 4-6 (I) shows a heat map chart obtained by re-aligning 57 surgical specimens and 20 FFPE tissue specimens obtained in Example 1 in the ascending order of the expression scores of 8 genes (middle) and a graph plotting the values of the expression scores of 8 genes (bottom) regarding the expression scores of 8 genes for 8 kinds of BSCE markers represented by NOs: R-1 to R-8. The vertical line in the figure represents a cutoff value. In addition, of four bars in the top, the top bar denoted by "Cancer type" indicates distinctions of cancer types (including normal tissues), the second bar denoted by "Diagnosis at Fukushima Medical University" indicates distinctions of cancer types as a result of pathological diagnosis of FFPE tissue specimens at Fukushima Medical University, the third bar denoted by "Basaloid" indicates distinction between BSCE and non-BSCE (colored portions correspond to BSCE), and the bottom bar denoted by "FFPE; Surgery" indicates distinction between surgical specimens and FFPE tissue specimens.
[Figure 4-7] Figure 4-7 (J) is a group scatter diagram for all analyzed specimens obtained using the expression scores of 8 genes for 8 kinds of BSCE markers represented by NOs: R-1 to R-8 when surgical specimens, biopsy specimens, and FFPE tissue specimens are all combined.
[Figure 4-8] Figure 4-8 (K) is a group scatter diagram created separately for each specimen type.

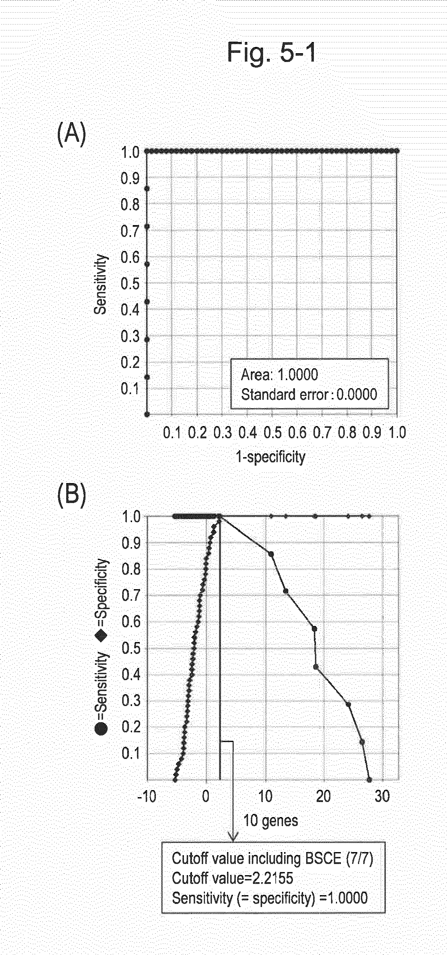

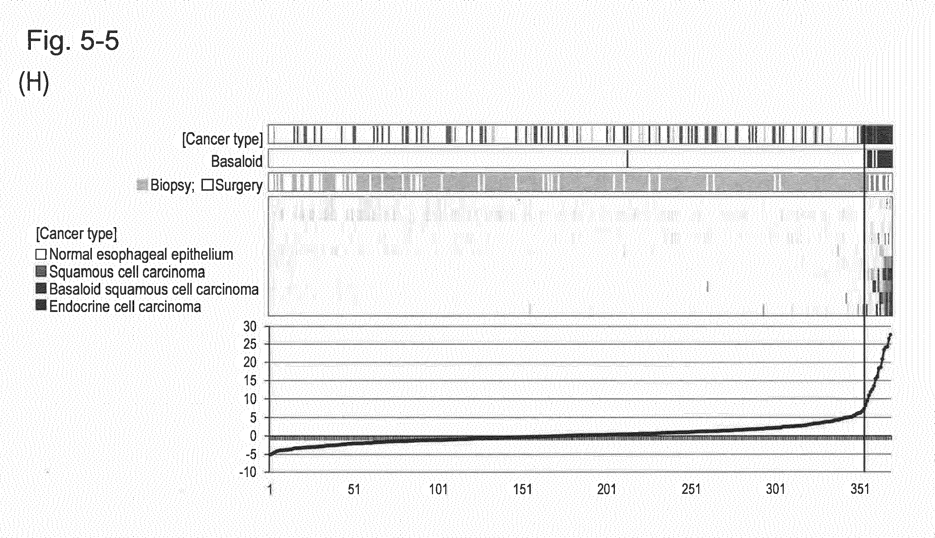

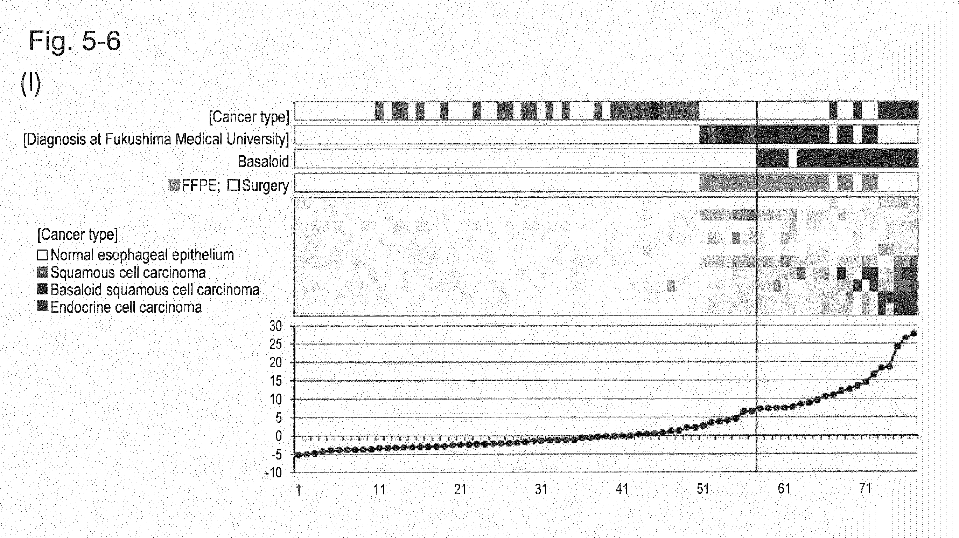

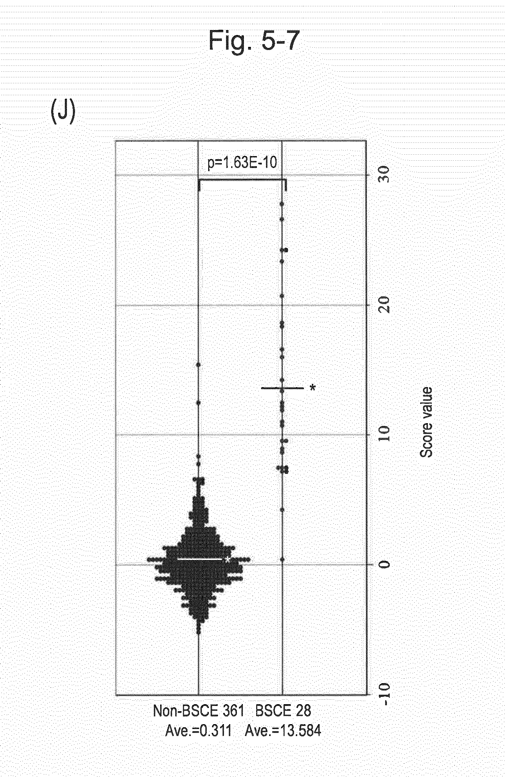

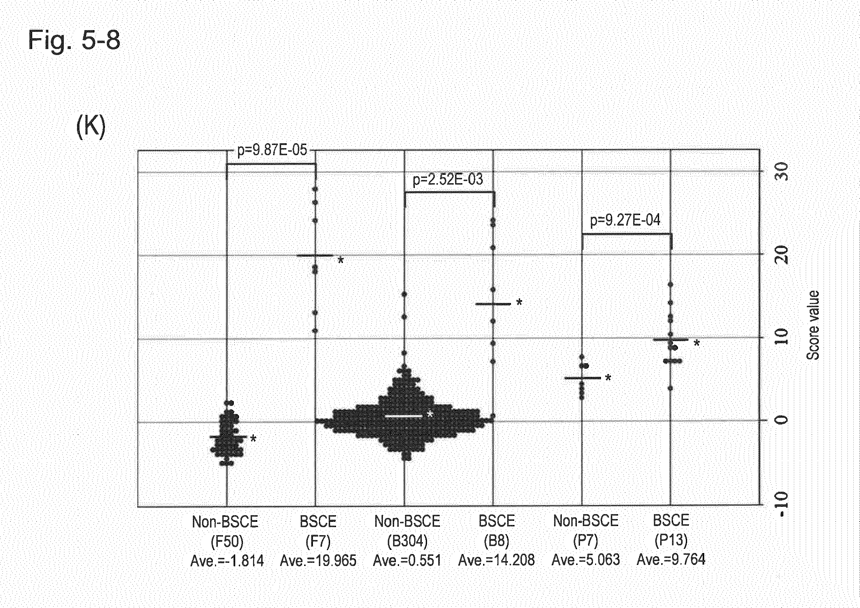

[Figure 5-1] Figure 5-1 (A) shows an ROC curve created from the expression scores of 10 genes (the sum of logarithmic transformed relative expression ratios of 10 kinds of BSCE markers represented by NOs: R-1 to R-5 and NOs: R-9 to R-13) for 57 surgical specimens obtained in Example 1 regarding 10 kinds of BSCE markers represented by NOs: R-1 to R-5 and NOs: R-9 to R-13 in Table 1. Figure 5-1 (B) shows a sensitivity-specificity curve from 57 surgical specimens obtained with the use of the 10 kinds of BSCE markers. The vertical axis represents "sensitivity" or "specificity" and the horizontal axis represents the sum of logarithmic transformed relative expression ratios of the 10 kinds of BSCE markers (expression scores of 10 genes).

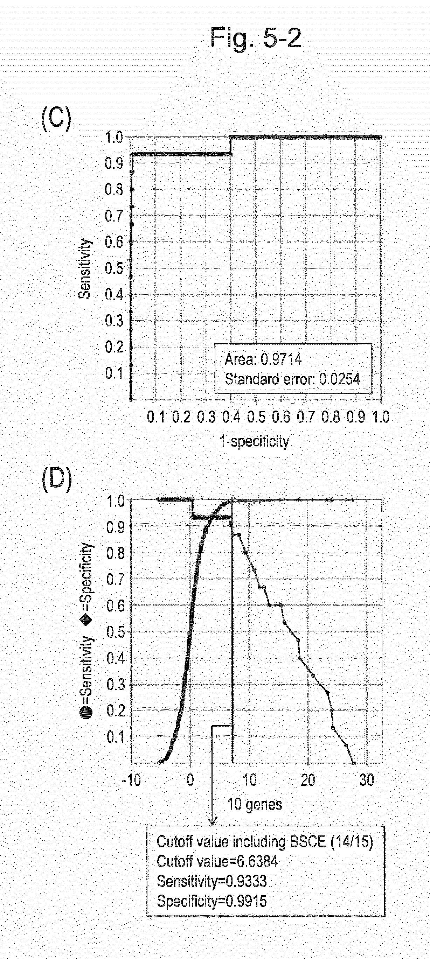

[Figure 5-2] Figure 5-2 (C) shows an ROC curve created from the expression scores of 10 genes for 57 surgical specimens and 312 biopsy specimens obtained in Example 1 regarding 10 kinds of BSCE markers represented by NOs: R-1 to R-5 and NOs: R-9 to R-13. Figure 5-2 (D) shows a sensitivity-specificity curve from 57 surgical specimens and 312 biopsy specimens obtained with the use of the 10 kinds of BSCE markers. The vertical axis represents "sensitivity" or "specificity" and the horizontal axis represents the "expression scores of 10 genes."

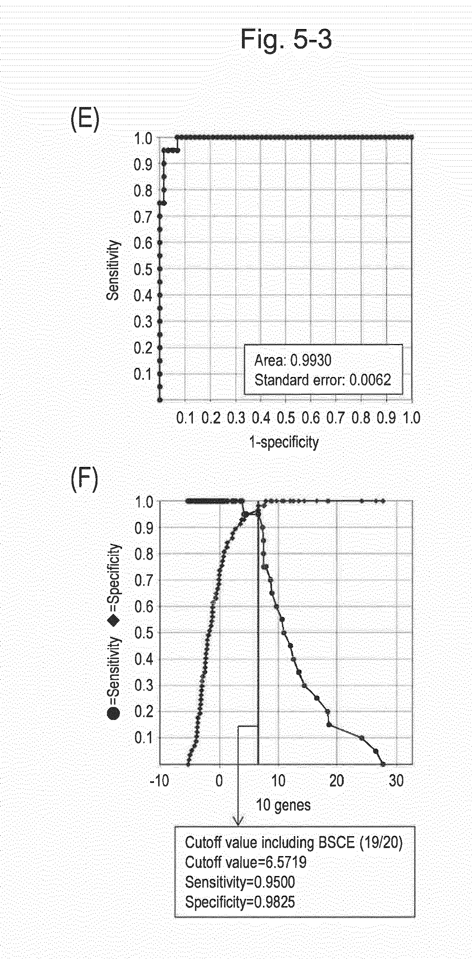

[Figure 5-3] Figure 5-3 (E) shows an ROC curve created from the expression scores of 10 genes for 57 surgical specimens and 20 FFPE tissue specimens obtained in Example 1 regarding 10 kinds of BSCE markers represented by NOs: R-1 to R-5 and NOs: R-9 to R-13.
Figure 5-3 (F) shows a sensitivity-specificity curve from 57 surgical specimens and 20 FFPE tissue specimens obtained with the use of the 10 kinds of BSCE markers. The vertical axis represents "sensitivity" or "specificity" and the horizontal axis represents the "expression scores of 10 genes."

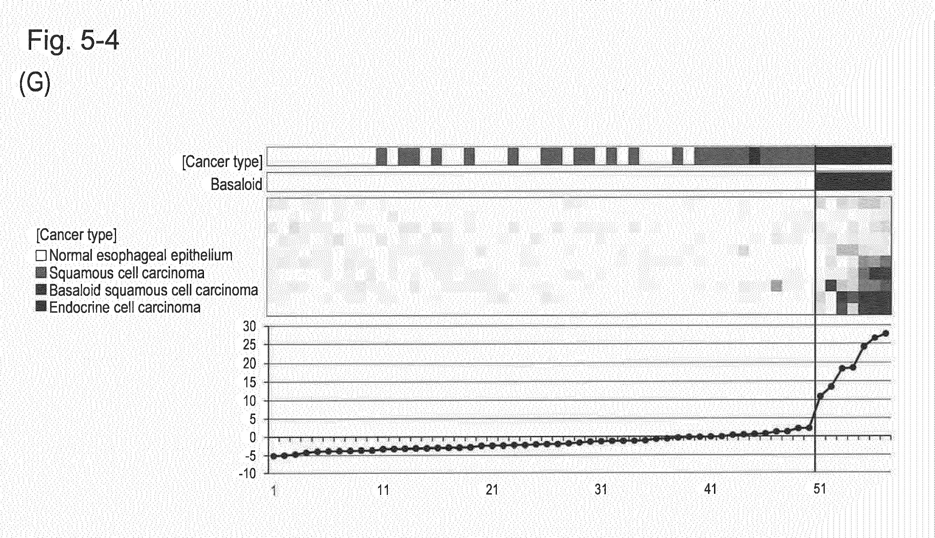

[Figure 5-4] Figure 5-4 (G) shows a heat map chart obtained by re-aligning 57 surgical specimens obtained in Example 1 in the ascending order of the expression scores of 10 genes (middle) and a graph plotting the values of the expression scores of 10 genes (bottom) regarding the expression scores of 10 genes for 10 kinds of BSCE markers represented by NOs: R-1 to R-5 and NOs: R-9 to R-13. The vertical line in the figure represents a cutoff value. In addition, of two bars in the top, the upper bar denoted by "Cancer type" indicates distinctions of cancer types (including normal tissues) and the lower bar denoted by "Basaloid" indicates distinction between BSCE and non-BSCE (colored portions correspond to BSCE).
[Figure 5-5] Figure 5-5 (H) shows a heat map chart obtained by re-aligning 57 surgical specimens and 312 biopsy specimens obtained in Example 1 in the ascending order of the expression scores of 10 genes (middle) and a graph plotting the values of the expression scores of 10 genes (bottom) regarding the expression scores of 10 genes for 10 kinds of BSCE markers represented by NOs: R-1 to R-5 and NOs: R-9 to R-13. The vertical line in the figure represents a cutoff value. In addition, of three bars in the top, the first bar denoted by "Cancer type" indicates distinctions of cancer types (including normal tissues), the second bar denoted by "Basaloid" indicates distinction between BSCE and non-BSCE (colored portions correspond to BSCE), and the third bar denoted by "Biopsy; Surgery" indicates distinction between surgical specimens and biopsy specimens.
[Figure 5-6] Figure 5-6 (I) shows a heat map chart obtained by re-aligning 57 surgical specimens and 20 FFPE tissue specimens obtained in Example 1 in the ascending order of the expression scores of 10 genes (middle) and a graph plotting the values of the expression scores of 10 genes (bottom) regarding the expression scores of 10 genes for 10 kinds of BSCE markers represented by NOs: R-1 to R-5 and NOs: R-9 to R-13. The vertical line in the figure represents a cutoff value. In addition, of four bars in the top, the top bar denoted by "Cancer type" indicates distinctions of cancer types (including normal tissues), the second bar denoted by "Diagnosis at Fukushima Medical University" indicates distinctions of cancer types as a result of pathological diagnosis of FFPE tissue specimens at Fukushima Medical University, the third bar denoted by "Basaloid" indicates distinction between BSCE and non-BSCE (colored portions correspond to BSCE), and the bottom bar denoted by "FFPE; Surgery" indicates distinction between surgical specimens and FFPE tissue specimens.
[Figure 5-7] Figure 5-7 (J) is a group scatter diagram for all analyzed specimens obtained using the expression scores of 10 genes for 10 kinds of BSCE markers represented by NOs: R-1 to R-5 and NOs: R-9 to R-13 when surgical specimens, biopsy specimens, and FFPE tissue specimens are all combined.
[Figure 5-8] Figure 5-8 (K) is a group scatter diagram created separately for each specimen type.

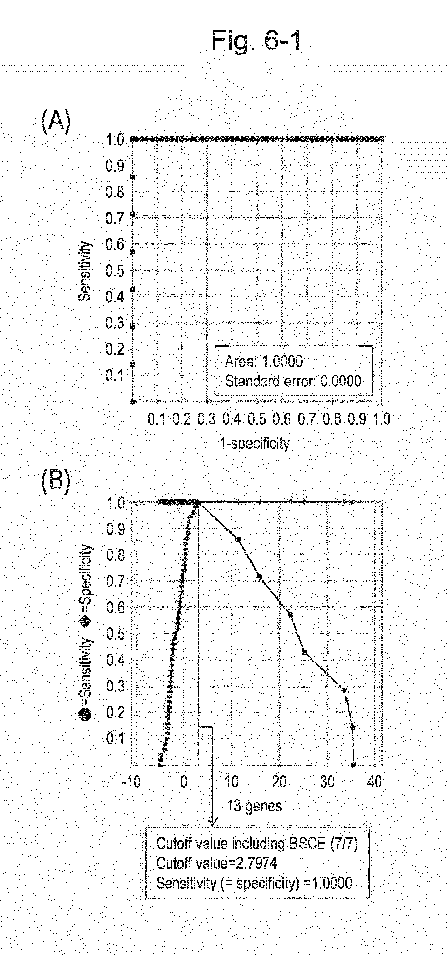

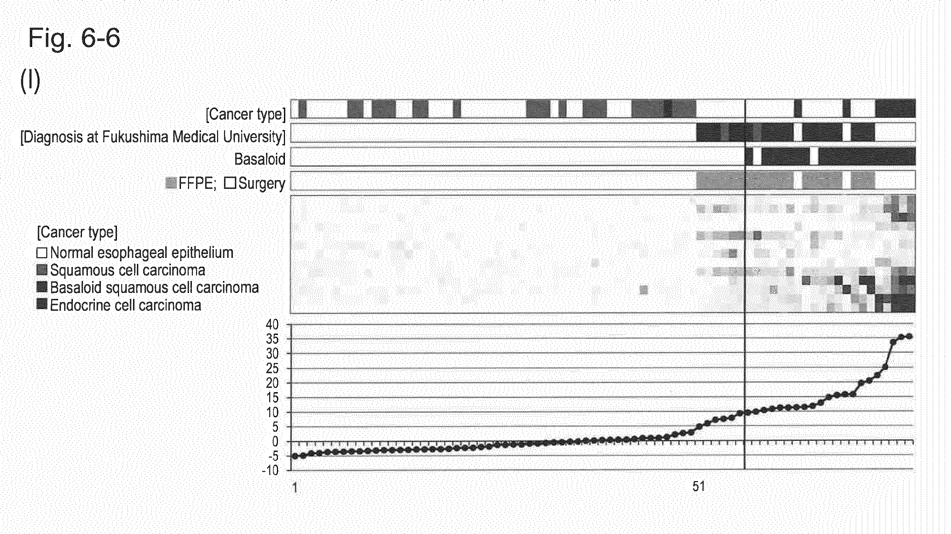

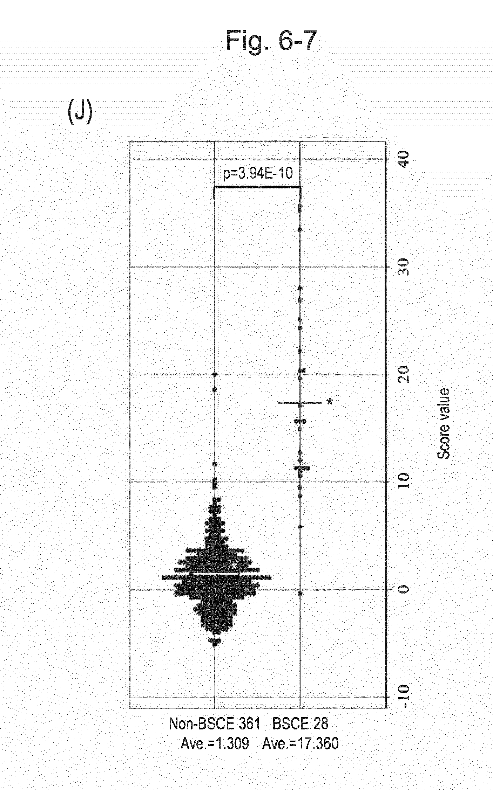

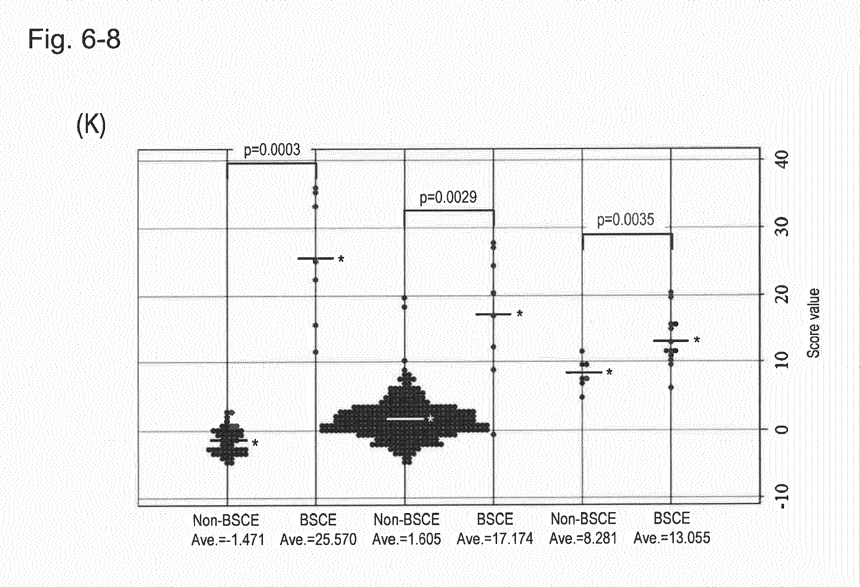

[Figure 6-1] Figure 6-1 (A) shows an ROC curve created from the expression scores of 13 genes (the sum of logarithmic transformed relative expression ratios of 13 kinds of BSCE markers) for 57 surgical specimens obtained in Example 1 regarding 13 kinds of BSCE markers represented by NOs: R-1 to R-13 in Table 1. Figure 6-1 (B) shows a sensitivity-specificity curve from 57 surgical specimens obtained with the use of the 13 kinds of BSCE markers. The vertical axis represents "sensitivity" or "specificity" and the horizontal axis represents "the sum of logarithmic transformed relative expression ratios of the 13 kinds of BSCE markers (expression scores of 13 genes)."

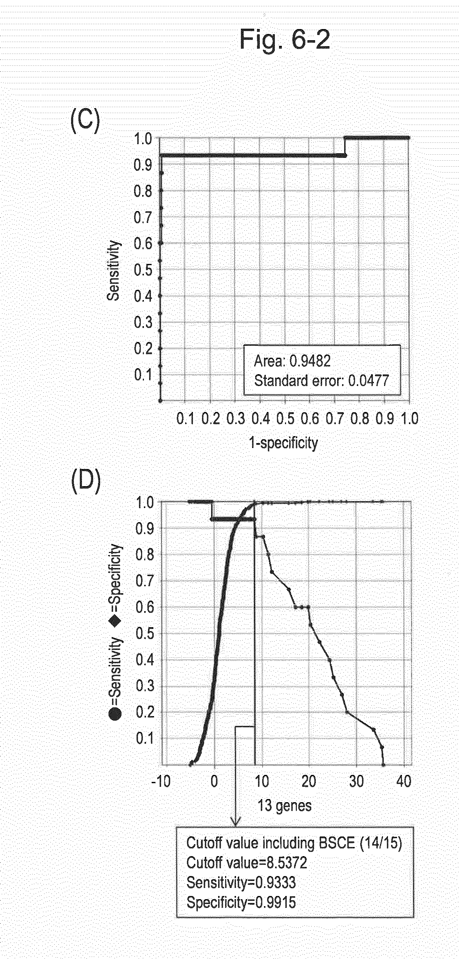

[Figure 6-2] Figure 6-2 (C) shows an ROC curve created from the expression scores of 13 genes for 57 surgical specimens and 312 biopsy specimens obtained in Example 1 regarding 13 kinds of BSCE markers represented by NOs: R-1 to R-13. Figure 6-2 (D) shows a sensitivity-specificity curve from 57 surgical specimens and 312 biopsy specimens obtained with the use of the 13 kinds of BSCE markers. The vertical axis represents "sensitivity" or "specificity" and the horizontal axis represents the "expression scores of 13 genes."

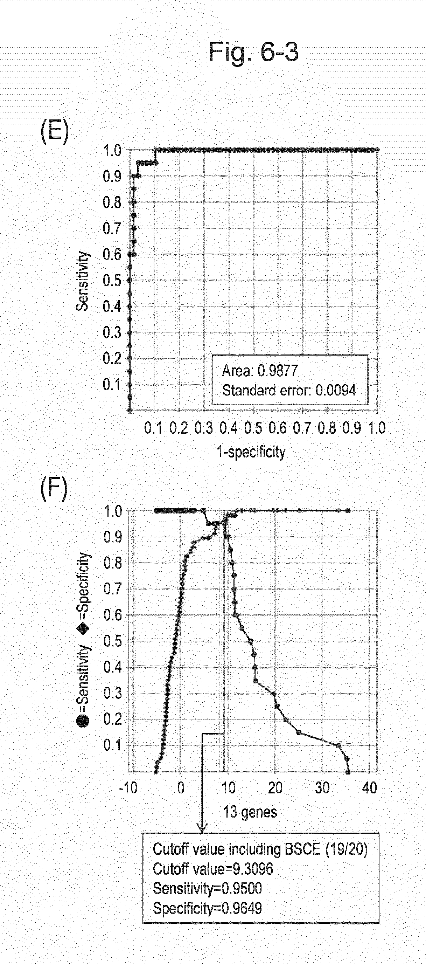

[Figure 6-3] Figure 6-3 (E) shows an ROC curve created from the expression scores of 13 genes for 57 surgical specimens and the expression scores of 13 genes for 20 FFPE tissue specimens obtained in Example 1 regarding 13 kinds of BSCE markers represented by NOs: R-1 to R-13. Figure 6-3 (F) shows a sensitivity-specificity curve from 57 surgical specimens and 20 FFPE tissue specimens obtained with the use of the 13 kinds of BSCE markers. The vertical axis represents "sensitivity" or "specificity" and the horizontal axis represents the "expression scores of 13 genes."

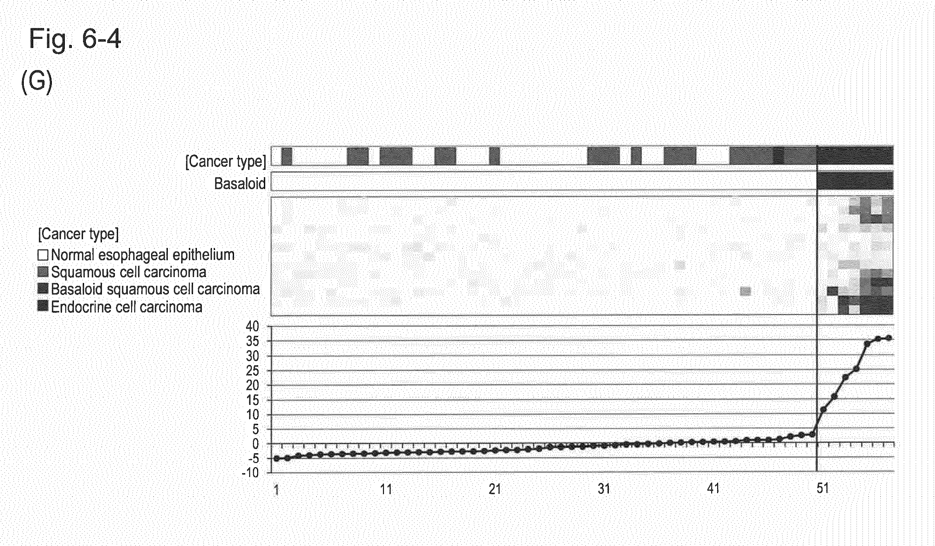

[Figure 6-4] Figure 6-4 (G) shows a heat map chart obtained by re-aligning 57 surgical specimens obtained in Example 1 in the ascending order of the expression scores of 13 genes (middle) and a graph plotting the values of the expression scores of 13 genes (bottom) regarding the expression scores of 13 genes for 13 kinds of BSCE markers represented by NOs: R-1 to R-13. The vertical line in the figure represents a cutoff value. In addition, of two bars in the top, the upper bar denoted by "Cancer type" indicates distinctions of cancer types (including normal tissues) and the lower bar denoted by "Basaloid" indicates distinction between BSCE and non-BSCE (colored portions correspond to BSCE).

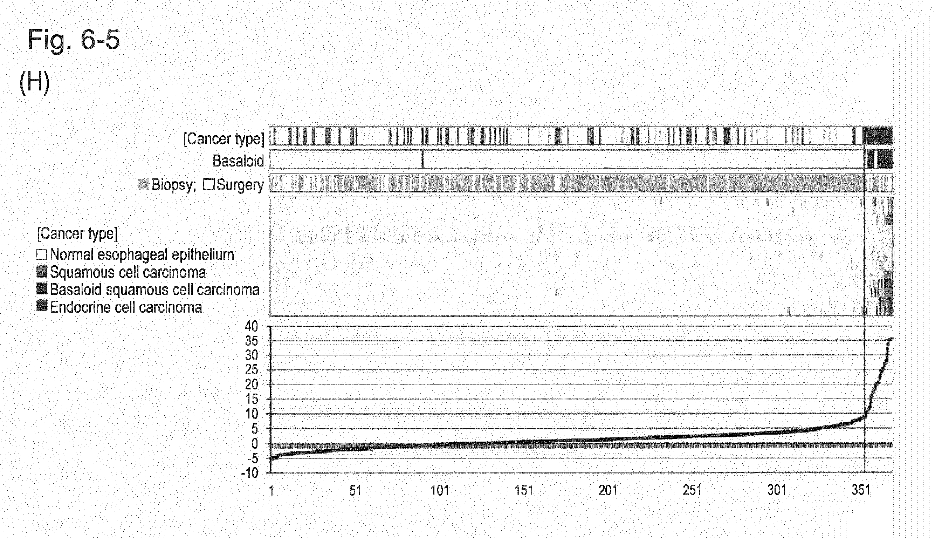

[Figure 6-5] Figure 6-5 (H) shows a heat map chart obtained by re-aligning 57 surgical specimens and 312 biopsy specimens obtained in Example 1 in the ascending order of the expression scores of 13 genes (middle) and a graph plotting the values of the expression scores of 13 genes (bottom) regarding the expression scores of 13 genes for 13 kinds of BSCE markers represented by NOs: R-1 to R-13. The vertical line in the figure represents a cutoff value. In addition, of three bars in the top, the first bar denoted by "Cancer type" indicates distinctions of cancer types (including normal tissues), the second bar denoted by "Basaloid" indicates distinction between BSCE and non-BSCE (colored portions correspond to BSCE), and the third bar denoted by "Biopsy; Surgery" indicates distinction between surgical specimens and biopsy specimens.
[Figure 6-6] Figure 6-6 (I) shows a heat map chart obtained by re-aligning 57 surgical specimens and 20 FFPE tissue specimens obtained in Example 1 in the ascending order of the expression scores of 13 genes (middle) and a graph plotting the values of the expression scores of 13 genes (bottom) regarding the expression scores of 13 genes for 13 kinds of BSCE markers represented by NOs: R-1 to R-13. The vertical line in the figure represents a cutoff value. In addition, of four bars in the top, the top bar denoted by "Cancer type" indicates distinctions of cancer types (including normal tissues), the second bar denoted by "Diagnosis at Fukushima Medical University" indicates distinctions of cancer types as a result of pathological diagnosis of FFPE tissue specimens at Fukushima Medical University, the third bar denoted by "Basaloid" indicates distinction between BSCE and non-BSCE (colored portions correspond to BSCE), and the bottom bar denoted by "FFPE; Surgery" indicates distinction between surgical specimens and FFPE tissue specimens.
[Figure 6-7] Figure 6-7 (J) is a group scatter diagram for all analyzed specimens obtained using the expression scores of 13 genes for 13 kinds of BSCE markers represented by NOs: R-1 to R-13 when surgical specimens, biopsy specimens, and FFPE tissue specimens are all combined.
[Figure 6-8] Figure 6-8 (K) is a group scatter diagram created separately for each specimen type.

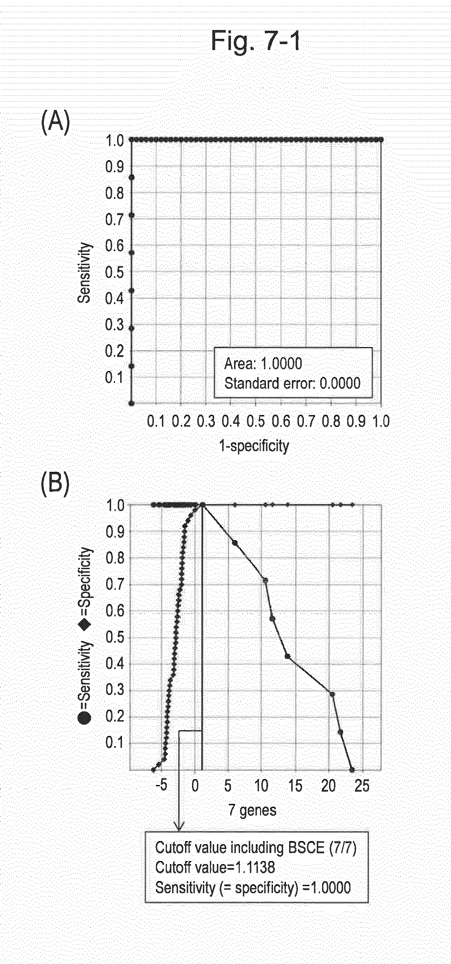

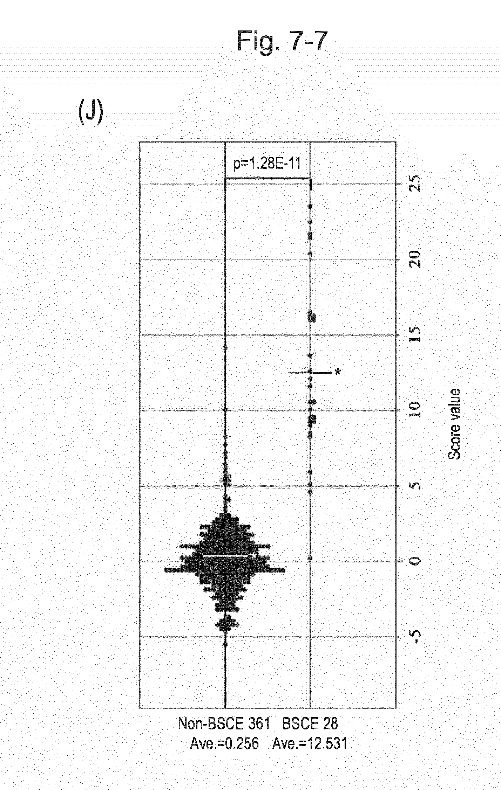

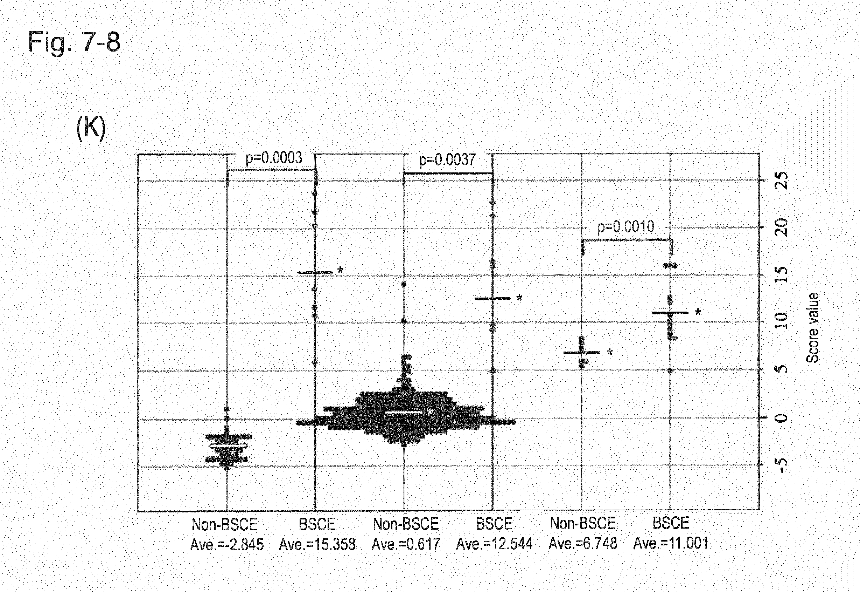

[Figure 7-1] Figure 7-1 (A) shows an ROC curve created from the expression scores of 7 genes (the sum of logarithmic transformed relative expression ratios of 7 kinds of BSCE markers) for 57 surgical specimens obtained in Example 1 regarding 7 kinds of BSCE markers represented by NOs: R-1 to R-5 and NOs: R-14 and R-15 in Table 1. Figure 7-1 (B) shows a sensitivity-specificity curve from 57 surgical specimens obtained with the use of the 7 kinds of BSCE markers. The vertical axis represents "sensitivity" or "specificity" and the horizontal axis represents "the sum of logarithmic transformed relative expression ratios of the 7 kinds of BSCE markers (expression scores of 7 genes)."

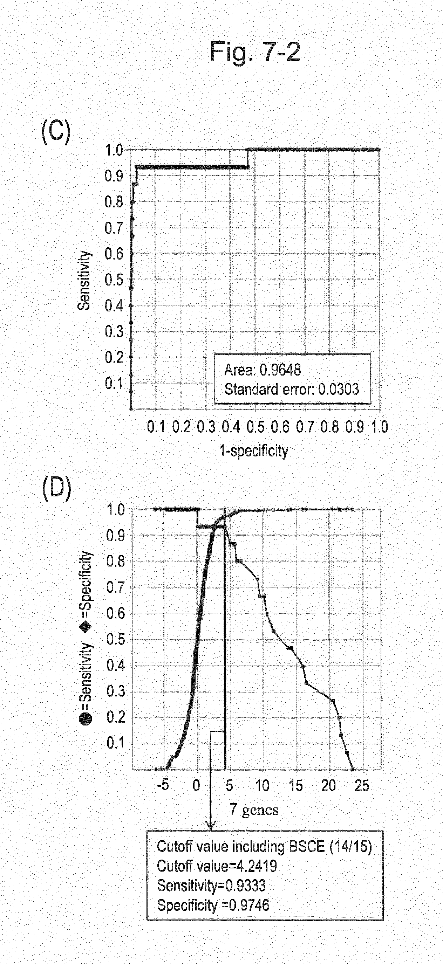

[Figure 7-2] Figure 7-2 (C) shows an ROC curve created from the expression scores of 7 genes for 57 surgical specimens and 312 biopsy specimens obtained in Example 1 regarding 7 kinds of BSCE markers represented by NOs: R-1 to R-5 and NOs: R-14 and R-15. Figure 7-2 (D) shows a sensitivity-specificiy curve from 57 surgical specimens and 312 biopsy specimens obtained with the use of the 7 kinds of BSCE markers. The vertical axis represents "sensitivity" or "specificity" and the horizontal axis represents the "expression scores of 7 genes."

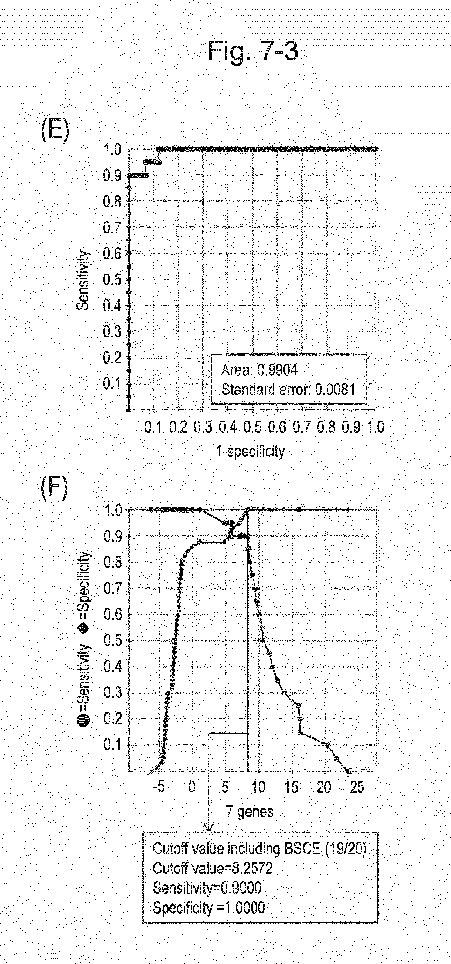

[Figure 7-3] Figure 7-3 (E) shows an ROC curve created from the expression scores of 7 genes for 57 surgical specimens and 20 FFPE tissue specimens obtained in Example 1 regarding 7 kinds of BSCE markers represented by NOs: R-1 to R-5 and NOs: R-14 and R-15. Figure 7-3 (F) shows a sensitivity-specificity curve from 57 surgical specimens and 20 FFPE tissue specimens obtained with the use of 7 kinds of BSCE markers. The vertical axis represents "sensitivity" or "specificity" and the horizontal axis represents the "expression scores of 7 genes."

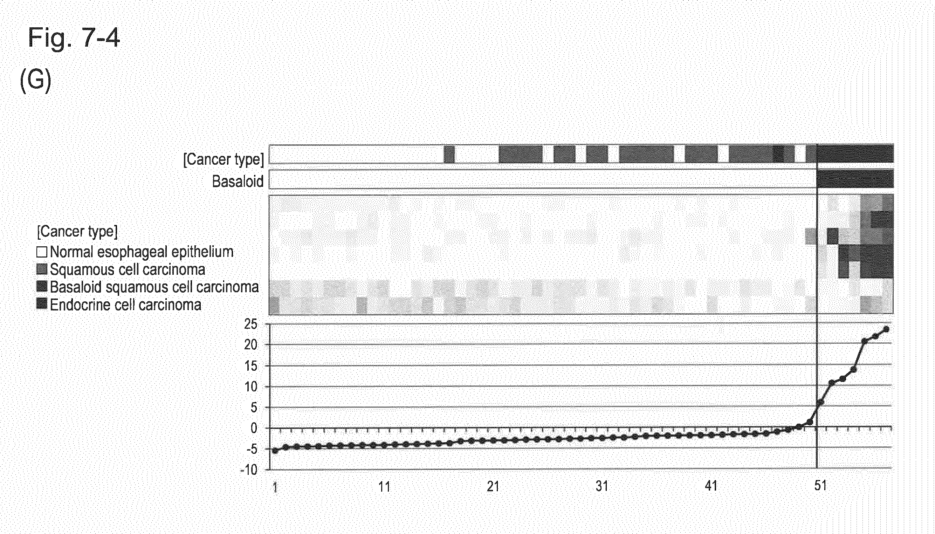

[Figure 7-4] Figure 7-4 (G) shows a heat map chart obtained by re-aligning 57 surgical specimens obtained in Example 1 in the ascending order of the expression scores of 7 genes (middle) and a graph plotting the values of the expression scores of 7 genes (bottom) regarding the expression scores of 7 genes for 7 kinds of BSCE markers represented by NOs: R-1 to R-5 and NOs: R-14 and R-15. The vertical line in the figure represents a cutoff value. In addition, of two bars in the top, the upper bar denoted by "Cancer type" indicates distinctions of cancer types (including normal tissues) and the lower bar denoted by "Basaloid" indicates distinction between BSCE and non-BSCE (colored portions correspond to BSCE).

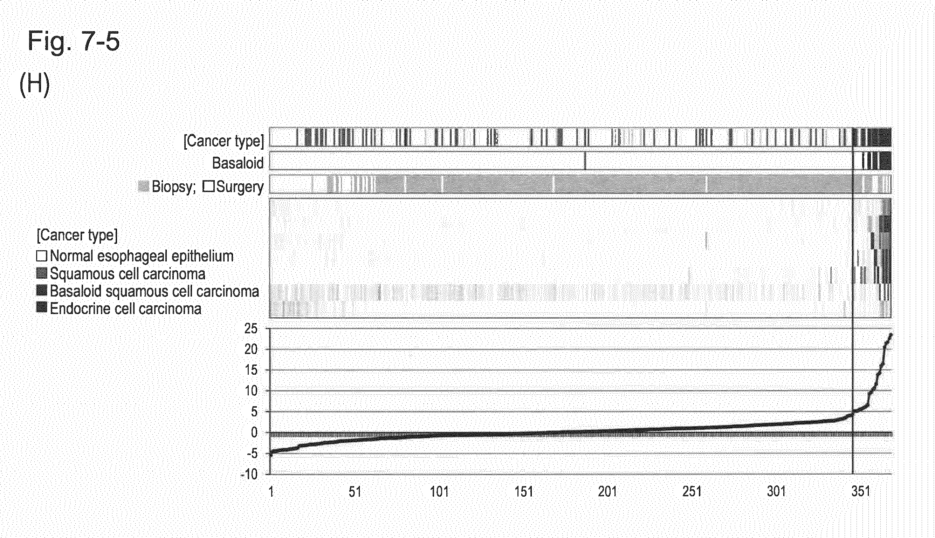

[Figure 7-5] Figure 7-5 (H) shows a heat map chart obtained by re-aligning 57 surgical specimens and 312 biopsy specimens obtained in Example 1 in the ascending order of the expression scores of 7 genes (middle) and a graph plotting the values of the expression scores of 7 genes (bottom) regarding the expression scores of 7 genes for 7 kinds of BSCE markers represented by NOs: R-1 to R-5 and NOs: R-14 and R-15. The vertical line in the figure represents a cutoff value. In addition, of three bars in the top, the first bar denoted by "Cancer type" indicates distinctions of cancer types (including normal tissues), the second bar denoted by "Basaloid" indicates distinction between BSCE and non-BSCE (colored portions correspond to BSCE), and the third bar denoted by "Biopsy; Surgery" indicates distinction between surgical specimens and biopsy specimens.

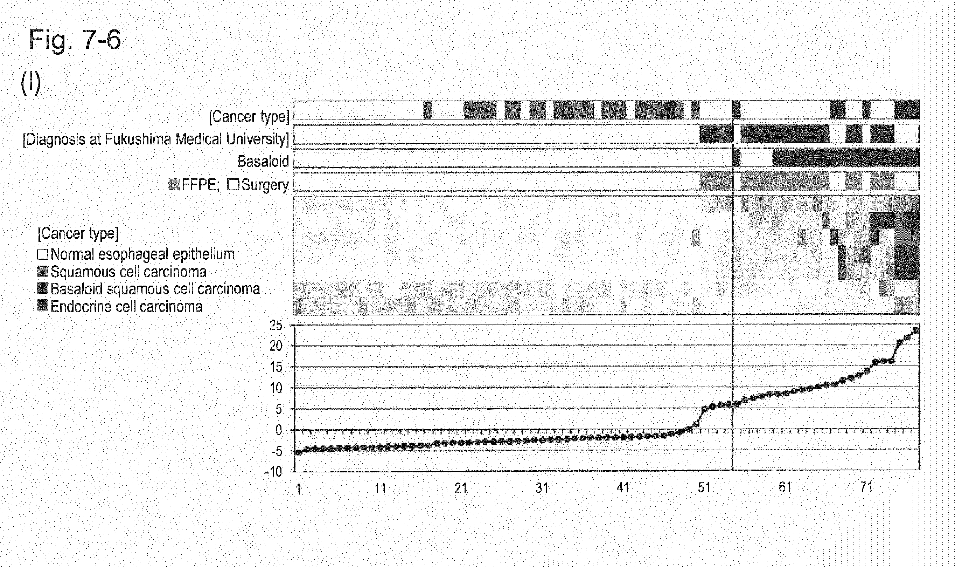

[Figure 7-6] Figure 7-6 (I) shows a heat map chart obtained re-aligning 57 surgical specimens and 20 FFPE tissue specimens obtained in Example 1 in the ascending order of the expression scores of 7 genes (middle) and a graph plotting the values of the expression scores of 7 genes (bottom) regarding the expression scores of 7 genes for 7 kinds of BSCE markers represented by NOs: R-1 to R-5 and NOs: R-14 and R-15. The vertical line in the figure represents a cutoff value. In addition, of four bars in the top, the top bar denoted by "Cancer type" indicates distinctions of cancer types (including normal tissues), the second bar denoted by "Diagnosis at Fukushima Medical University" indicates distinctions of cancer types as a result of pathological diagnosis of FFPE tissue specimens at Fukushima Medical University, the third bar denoted by "Basaloid" indicates distinction between BSCE and non-BSCE (colored portions correspond to BSCE), and the bottom bar denoted by "FFPE; Surgery" indicates distinction between surgical specimens and FFPE tissue specimens.
[Figure 7-7] Figure 7-7 (J) is a group scatter diagram for all analyzed specimens obtained using the expression scores of 7 genes for 7 kinds of BSCE markers represented by NOs: R-1 to R-5 and NOs: R-14 and R-15 when surgical specimens, biopsy specimens, and FFPE tissue specimens are all combined.
[Figure 7-8] Figure 7-8 (K) is a group scatter diagram created separately for each specimen type.

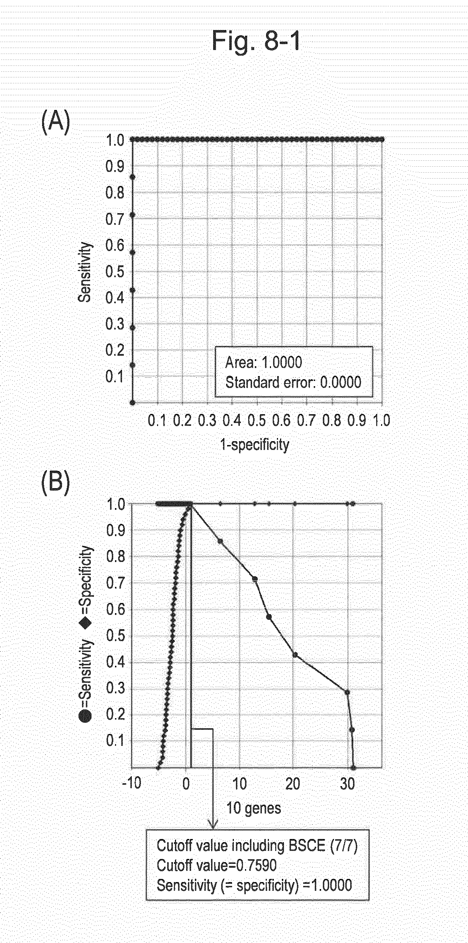

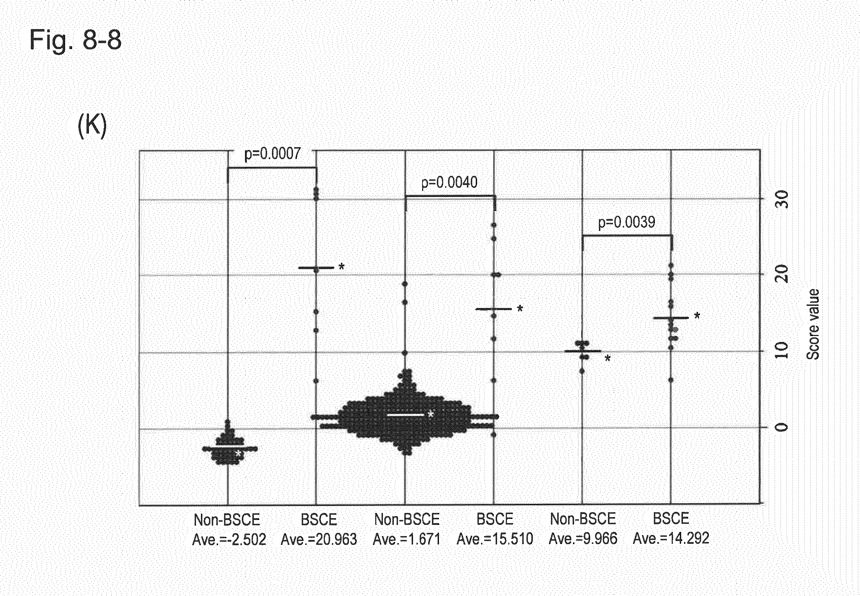

[Figure 8-1] Figure 8-1 (A) shows an ROC curve created from the expression scores of 10' genes (the sum of logarithmic transformed relative expression ratios of 10 kinds of BSCE markers represented by NOs: R-1 to R-8 and NOs: R-14 and R-15) for 57 surgical specimens obtained in Example 1 regarding 10 kinds of BSCE markers represented by NOs: R-1 to R-8 and NOs: R-14 and R-15 in Table 1. Figure 8-1 (B) shows a sensitivity-specificity curve from 57 surgical specimens obtained with the use of the 10 kinds of BSCE markers. The vertical axis represents "sensitivity" or "specificity" and the horizontal axis represents "the sum of logarithmic transformed relative expression ratios of the 10 kinds of BSCE markers (expression scores of 10' genes)."

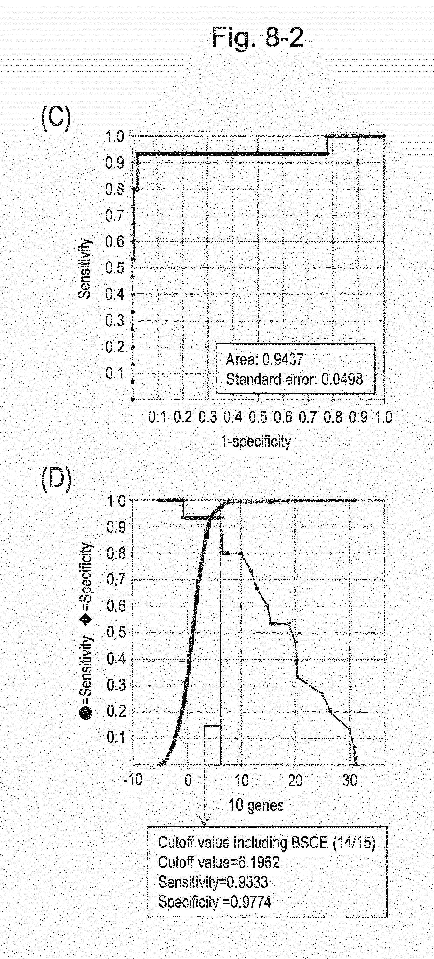

[Figure 8-2] Figure 8-2 (C) shows an ROC curve created from the expression scores of 10' genes for 57 surgical specimens and 312 biopsy specimens obtained in Example 1 regarding 10 kinds of BSCE markers represented by NOs: R-1 to R-8 and NOs: R-14 and R-15. Figure 8-2 (D) shows a sensitivity-specificity curve from 57 surgical specimens and 312 biopsy specimens obtained with the use of the 10 kinds of BSCE markers. The vertical axis represents "sensitivity" or "specificity" and the horizontal axis represents the "expression scores of 10' genes."

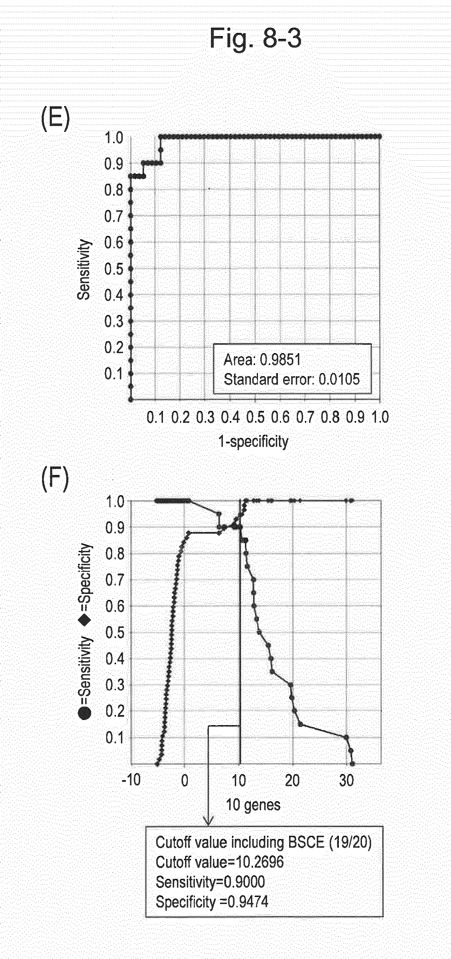

[Figure 8-3] Figure 8-3 (E) shows an ROC curve created from the expression scores of 10' genes for 57 surgical specimens and 20 FFPE tissue specimens obtained in Example 1 regarding 10 kinds of BSCE markers represented by NOs: R-1 to R-8 and NOs: R-14 and R-15. Figure 8-3 (F) shows a sensitivity-specificity curve from 57 surgical specimens and 20 FFPE tissue specimens obtained with the use of 10 kinds of BSCE markers. The vertical axis represents "sensitivity" or "specificity" and the horizontal axis represents the "expression scores of 10' genes."

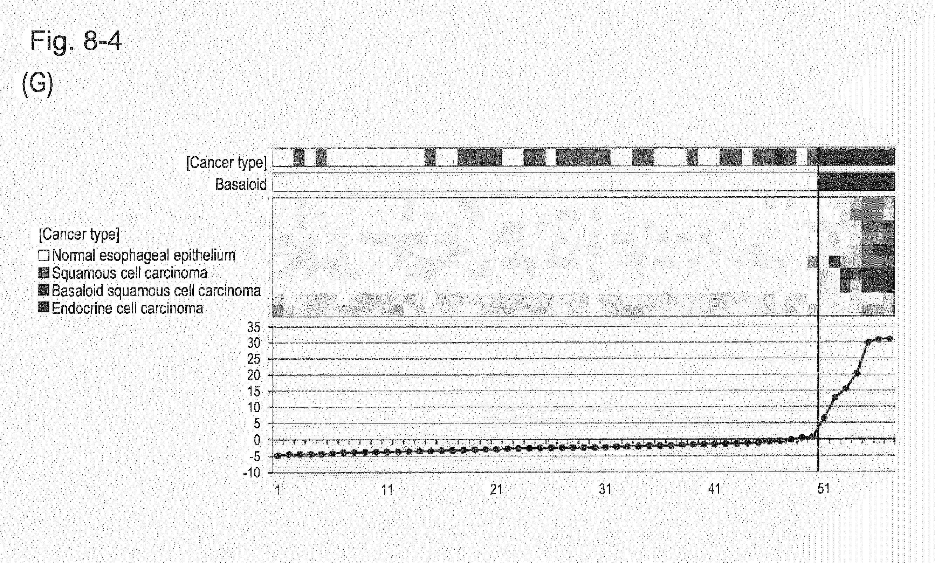

[Figure 8-4] Figure 8-4 (G) shows a heat map chart obtained by re-aligning 57 surgical specimens obtained in Example 1 in the ascending order of the expression scores of 10' genes (middle) and a graph plotting the values of the expression scores of 10' genes (bottom) regarding the expression scores of 10' genes for 10 kinds of BSCE markers represented by NOs: R-1 to R-8 and NOs: R-14 and R-15. The vertical line in the figure represents a cutoff value. In addition, of two bars in the top, the upper bar denoted by "Cancer type" indicates distinctions of cancer types (including normal tissues) and the lower bar denoted by "Basaloid" indicates distinction between BSCE and non-BSCE (colored portions correspond to BSCE).

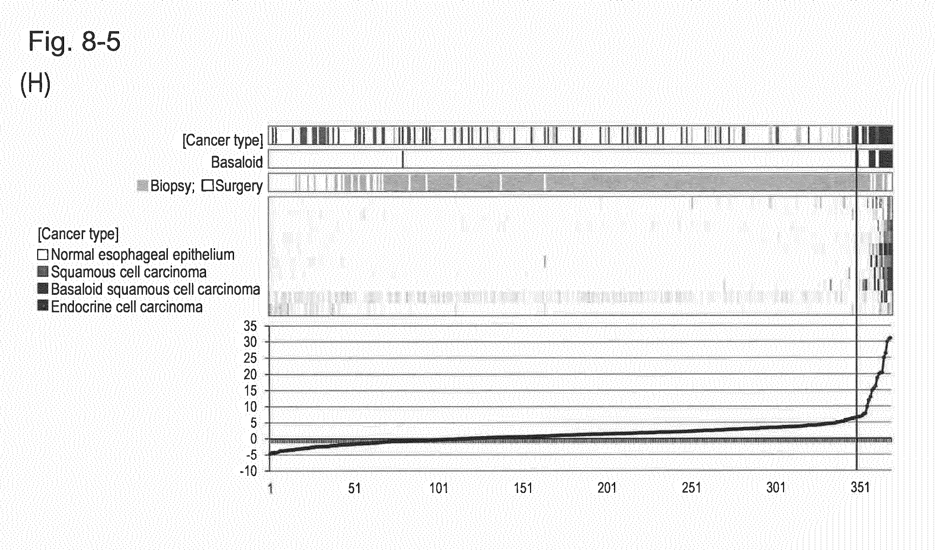

[Figure 8-5] Figure 8-5 (H) shows a heat map chart obtained by re-aligning 57 surgical specimens and 312 biopsy specimens obtained in Example 1 in the ascending order of the expression scores of 10' genes (middle) and a graph plotting the values of the expression scores of 10' genes (bottom) regarding the expression scores of 10' genes for 10 kinds of BSCE markers represented by NOs: R-1 to R-8 and NOs: R-14 and R-15. The vertical line in the figure represents a cutoff value. In addition, of three bars in the top, the first bar denoted by "Cancer type" indicates distinctions of cancer types (including normal tissues), the second bar denoted by "Basaloid" indicates distinction between BSCE and non-BSCE (colored portions correspond to BSCE), and the third bar denoted by "Biopsy; Surgery" indicates distinction between surgical specimens and biopsy specimens.

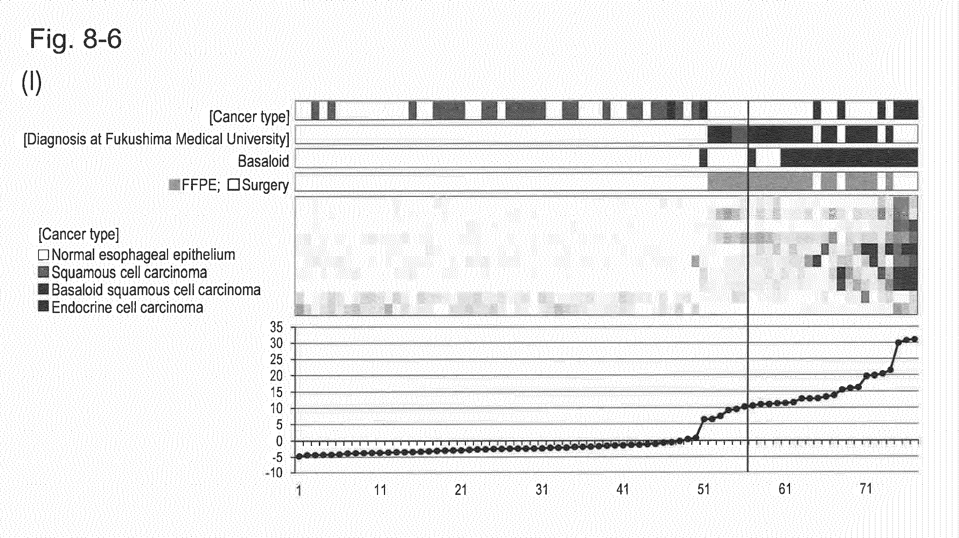

[Figure 8-6] Figure 8-6 (I) shows a heat map chart obtained by re-aligning 57 surgical specimens and 20 FFPE tissue specimens obtained in Example 1 in the ascending order of the expression scores of 10' genes (middle) and a graph plotting the values of the expression scores of 10' genes (bottom) regarding the expression scores of 10' genes for 10 kinds of BSCE markers represented by NOs: R-1 to R-8 and NOs: R-14 and R-15. The vertical line in the figure represents a cutoff value. In addition, of four bars in the top, the top bar denoted by "Cancer type" indicates distinctions of cancer types (including normal tissues), the second bar denoted by "Diagnosis at Fukushima Medical University" indicates distinctions of cancer types as a result of pathological diagnosis of FFPE tissue specimens at Fukushima Medical University, the third bar denoted by "Basaloid" indicates distinction between BSCE and non-BSCE (colored portions correspond to BSCE), and the bottom bar denoted by "FFPE; Surgery" indicates distinction between surgical specimens and FFPE tissue specimens.

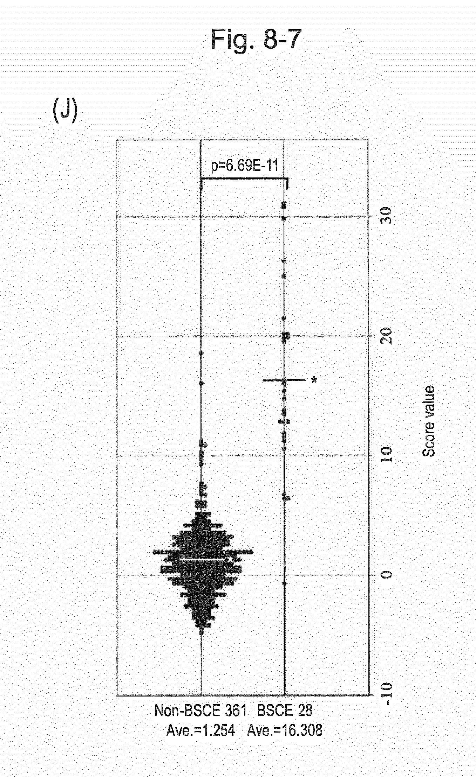

[Figure 8-7] Figure 8-7 (J) is a group scatter diagram for all analyzed specimens obtained using the expression scores of 10' genes for 10 kinds of BSCE markers represented by NOs: R-1 to R-8 and NOs: R-14 and R-15 when surgical specimens, biopsy specimens, and FFPE tissue specimens are all combined.
[Figure 8-8] Figure 8-8 (K) is a group scatter diagram created separately for each specimen type.

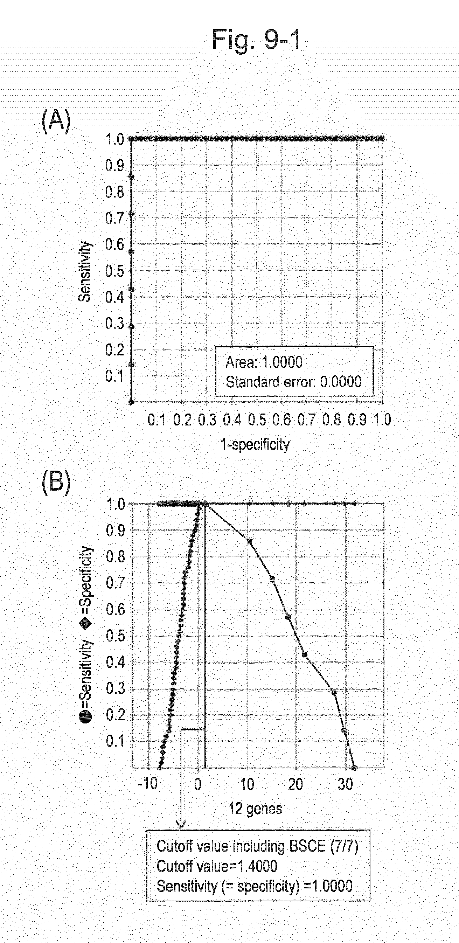

[Figure 9-1] Figure 9-1 (A) shows an ROC curve created from the expression scores of 12 genes (the sum of logarithmic transformed relative expression ratios of 12 kinds of BSCE markers) for 57 surgical specimens obtained in Example 1 regarding 12 kinds of BSCE markers represented by NOs: R-1 to R-5 and NOs: R-9 to R-15 in Table 1. Figure 9-1 (B) shows a sensitivity-specificity curve from 57 surgical specimens obtained with the use of the 12 kinds of BSCE markers. The vertical axis represents "sensitivity" or "specificity" and the horizontal axis represents "the sum of logarithmic transformed relative expression ratios of the 12 kinds of BSCE markers (expression scores of 12 genes)."

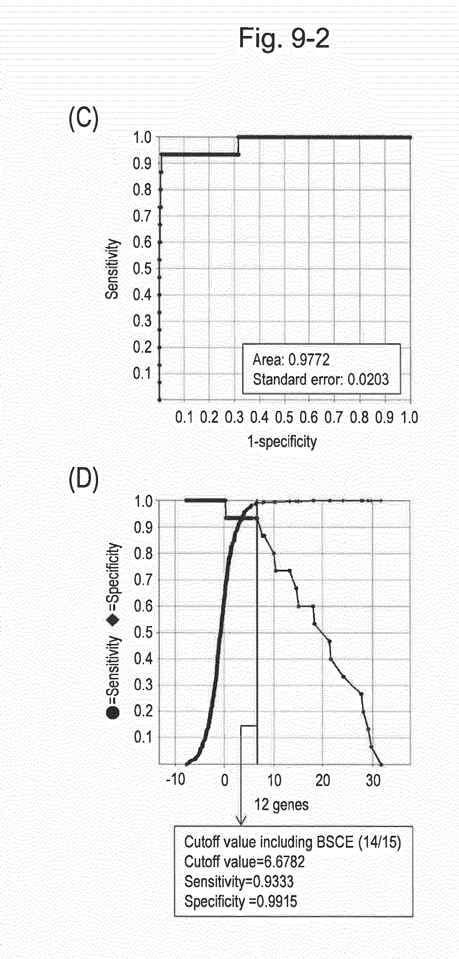

[Figure 9-2] Figure 9-2 (C) shows an ROC curve created from the expression scores of 12 genes for 57 surgical specimens and 312 biopsy specimens obtained in Example 1 regarding 12 kinds of BSCE markers represented by NOs: R-1 to R-5 and NOs: R-9 to R-15. Figure 9-2 (D) shows a sensitivity-specificity curve from 57 surgical specimens and 312 biopsy specimens obtained with the use of the 12 kinds of BSCE markers. The vertical axis represents "sensitivity" or "specificity" and the horizontal axis represents the "expression scores of 12 genes."

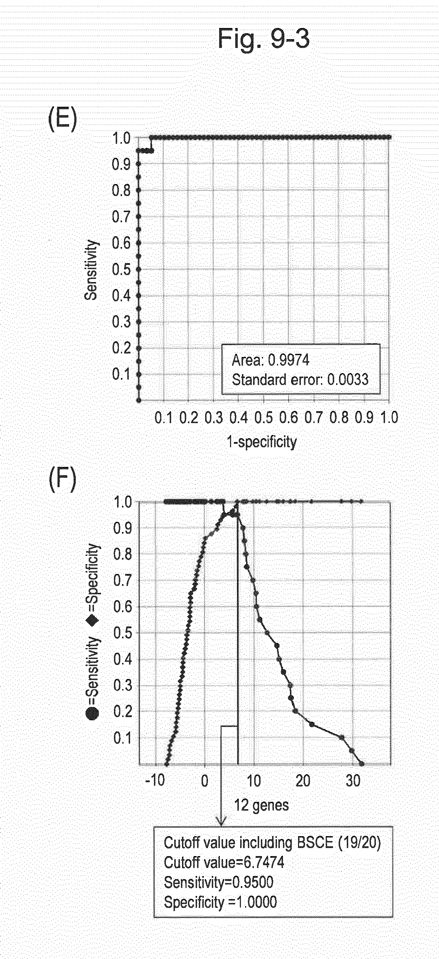

[Figure 9-3] Figure 9-3 (E) shows an ROC curve created from the expression scores of 12 genes for 57 surgical specimens and 20 FFPE tissue specimens obtained in Example 1 regarding 12 kinds of BSCE markers represented by NOs: R-1 to R-5 and NOs: R-9 to R-15. Figure 9-3 (F) shows a sensitivity-specificity curve from 57 surgical specimens and 20 FFPE tissue specimens obtained with the use of the 12 kinds of BSCE markers. The vertical axis represents "sensitivity" or "specificity" and the horizontal axis represents the "expression scores of 12 genes."

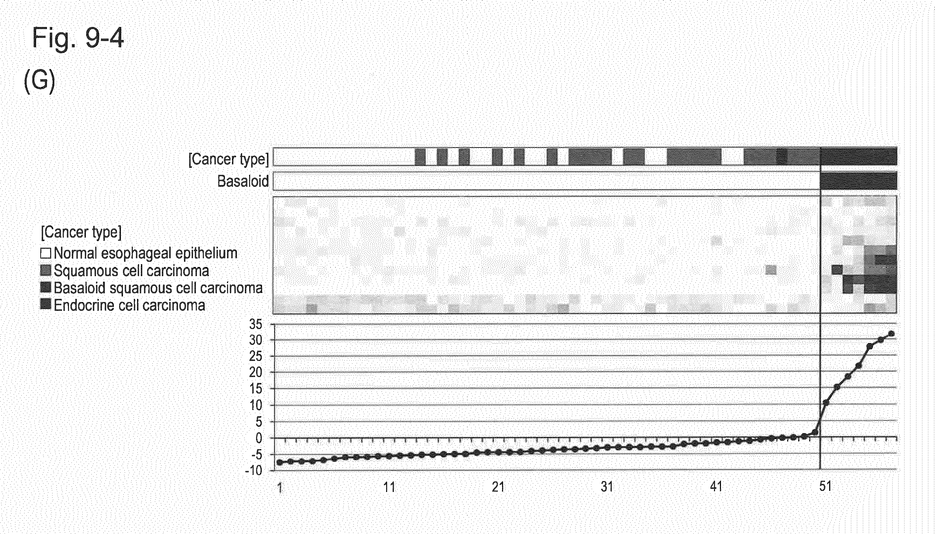

[Figure 9-4] Figure 9-4 (G) shows a heat map chart obtained by re-aligning 57 surgical specimens obtained in Example 1 in the ascending order of the expression scores of 12 genes (middle) and a graph plotting the values of the expression scores (bottom) regarding the expression scores of 12 genes for 12 kinds of BSCE markers represented by NOs: R-1 to R-5 and NOs: R-9 to R-15. The vertical line in the figure represents a cutoff value. In addition, of two bars in the top, the upper bar denoted by "Cancer type" indicates distinctions of cancer types (including normal tissues) and the lower bar denoted by "Basaloid" indicates distinction between BSCE and non-BSCE (colored portions correspond to BSCE).

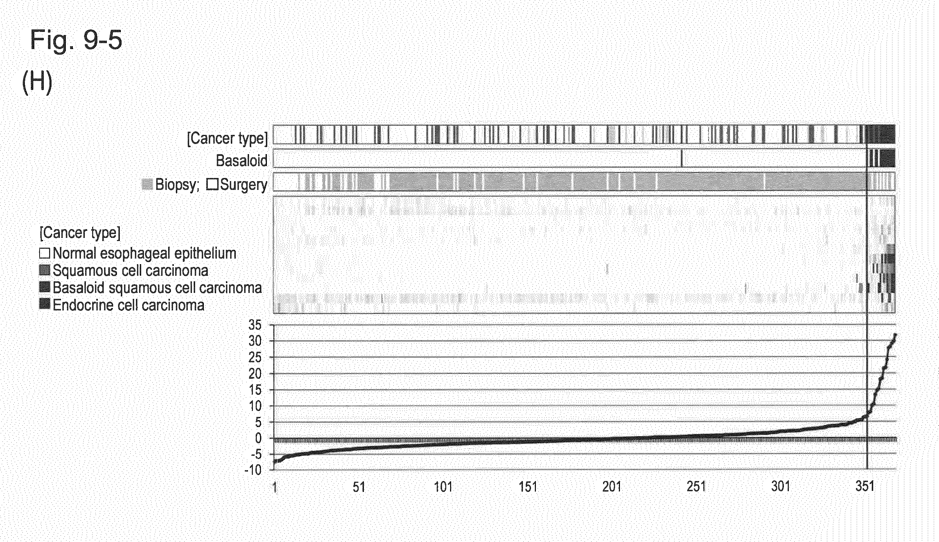

[Figure 9-5] Figure 9-5 (H) shows a heat map chart obtained by re-aligning 57 surgical specimens and 312 biopsy specimens obtained in Example 1 in the ascending order of the expression scores of 12 genes (middle) and a graph plotting the values of the expression scores (bottom) regarding the expression scores of 12 genes for 12 kinds of BSCE markers represented by NOs: R-1 to R-5 and NOs: R-9 to R-15. The vertical line in the figure represents a cutoff value. In addition, of three bars in the top, the first bar denoted by "Cancer type" indicates distinctions of cancer types (including normal tissues), the second bar denoted by "Basaloid" indicates distinction between BSCE and non-BSCE (colored portions correspond to BSCE), and the third bar denoted by "Biopsy; Surgery" indicates distinction between surgical specimens and biopsy specimens.

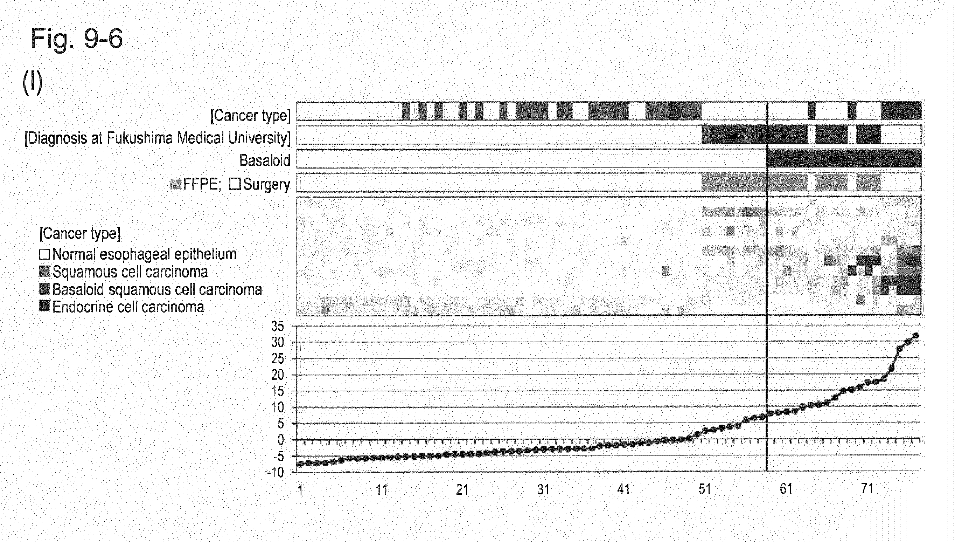

[Figure 9-6] Figure 9-6 (I) shows a heat map chart obtained by re-aligning 57 surgical specimens and 20 FFPE tissue specimens obtained in Example 1 in the ascending order of the expression scores of 12 genes (middle) and a graph plotting the values of the expression scores of 12 genes (bottom) regarding the expression scores of 12 genes for 12 kinds of BSCE markers represented by NOs: R-1 to R-5 and NOs: R-9 to R-15. The vertical line in the figure represents a cutoff value. In addition, of four bars in the top, the top bar denoted by "Cancer type" indicates distinctions of cancer types (including normal tissues), the second bar denoted by "Diagnosis at Fukushima Medical University" indicates distinctions of cancer types as a result of pathological diagnosis of FFPE tissue specimens at Fukushima Medical University, the third bar denoted by "Basaloid" indicates distinction between BSCE and non-BSCE (colored portions correspond to BSCE), and the bottom bar denoted by "FFPE; Surgery" indicates distinction between surgical specimens and FFPE tissue specimens.

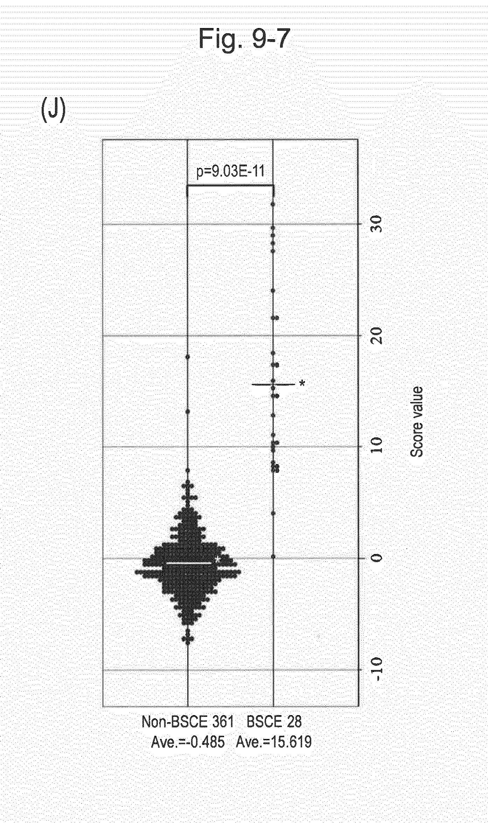

[Figure 9-7] Figure 9-7 (J) is a group scatter diagram for all analyzed specimens obtained using the expression scores of 12 genes for 12 kinds of BSCE markers represented by NOs: R-1 to R-5 and NOs: R-9 to R-15 when surgical specimens, biopsy specimens, and FFPE tissue specimens are all combined.

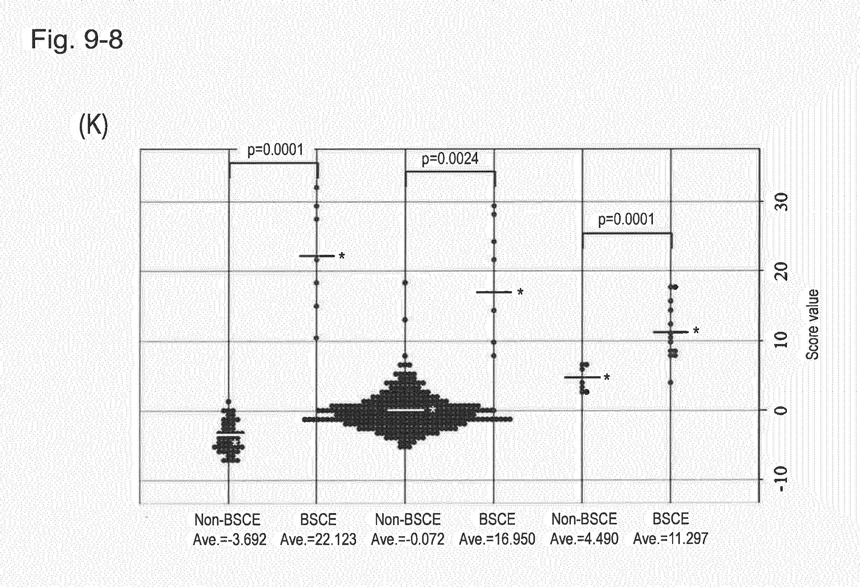

[Figure 9-8] Figure 9-8 (K) is a group scatter diagram created separately for each specimen type.

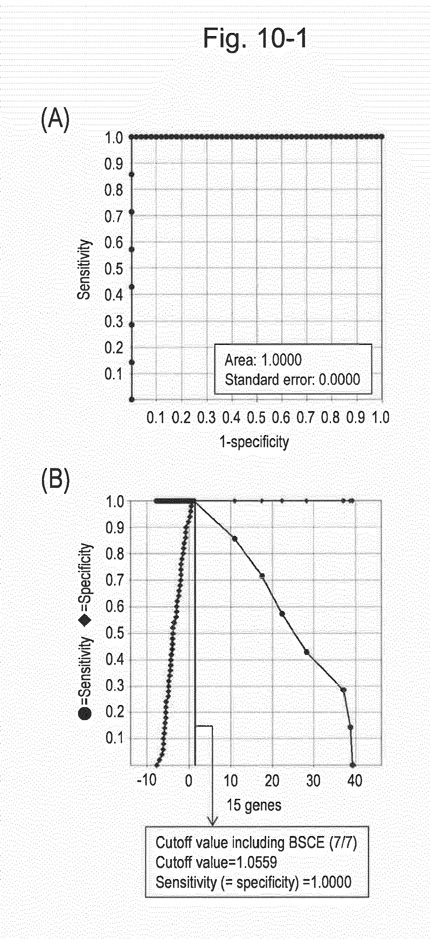

[Figure 10-1] Figure 10-1 (A) shows an ROC curve created from the expression scores of 15 genes (the sum of logarithmic transformed relative expression ratios of 15 kinds of BSCE markers) for 57 surgical specimens obtained in Example 1 regarding 15 kinds of BSCE markers represented by NOs: R-1 to R-15 in Table 1. Figure 10-1 (B) shows a sensitivity-specificity curve from 57 surgical specimens obtained with the use of the 15 kinds of BSCE markers. The vertical axis represents "sensitivity" or "specificity" and the horizontal axis represents the sum of logarithmic transformed relative expression ratios of the 15 kinds of BSCE markers (expression scores of 15 genes).

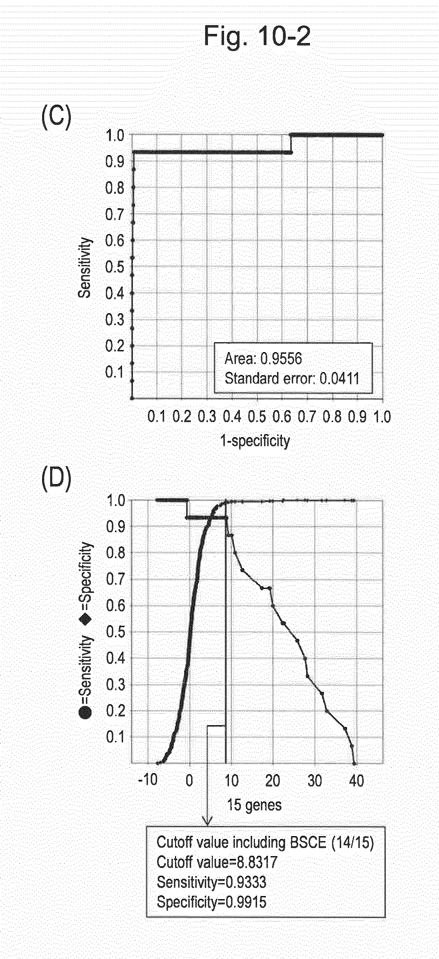

[Figure 10-2] Figure 10-2 (C) shows an ROC curve created from the expression scores of 15 genes for 57 surgical specimens and 312 biopsy specimens obtained in Example 1 regarding 15 kinds of BSCE markers represented by NOs: R-1 to R-15. Figure 10-2 (D) shows a sensitivity-specificity curve from 57 surgical specimens and 312 biopsy specimens obtained with the use of the 15 kinds of BSCE markers. The vertical axis represents "sensitivity" or "specificity" and the horizontal axis represents the "expression scores of 15 genes."

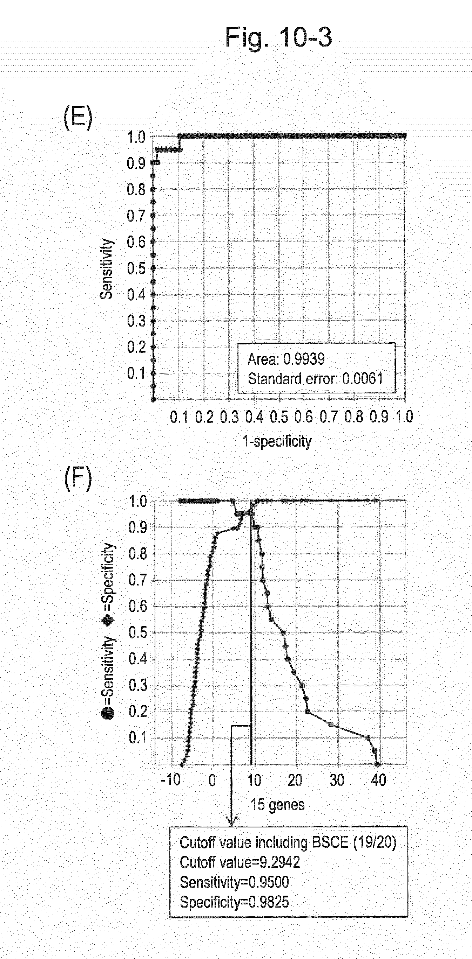

[Figure 10-3] Figure 10-3 (E) shows an ROC curve created from the expression scores of 15 genes for 57 surgical specimens and 20 FFPE tissue specimens obtained in Example 1 regarding 15 kinds of BSCE markers represented by NOs: R-1 to R-15. Figure 10-3 (F) shows a sensitivity-specificity curve from 57 surgical specimens and 20 FFPE tissue specimens obtained with the use of the 15 kinds of BSCE markers. The vertical axis represents "sensitivity" or "specificity" and the horizontal axis represents the "expression scores of 15 genes."

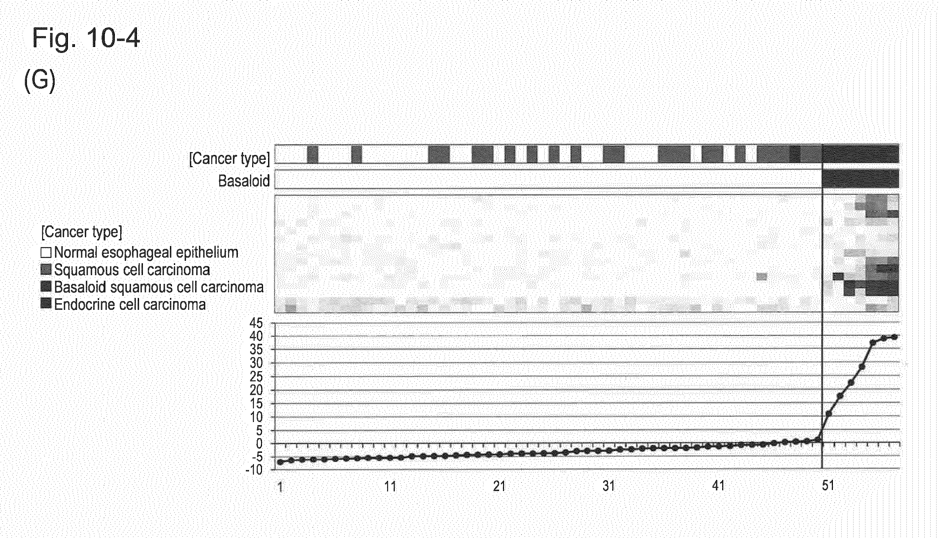

[Figure 10-4] Figure 10-4 (G) shows a heat map chart obtained by re-aligning 57 surgical specimens obtained in Example 1 in the ascending order of the expression scores of 15 genes (middle) and a graph plotting the values of the expression scores (bottom) regarding the expression scores of 15 genes for 15 kinds of BSCE markers represented by NOs: R-1 to R-15. The vertical line in the figure represents a cutoff value. In addition, of two bars in the top, the upper bar denoted by "Cancer type" indicates distinctions of cancer types (including normal tissues) and the lower bar denoted by "Basaloid" indicates distinction between BSCE and non-BSCE (colored portions correspond to BSCE).

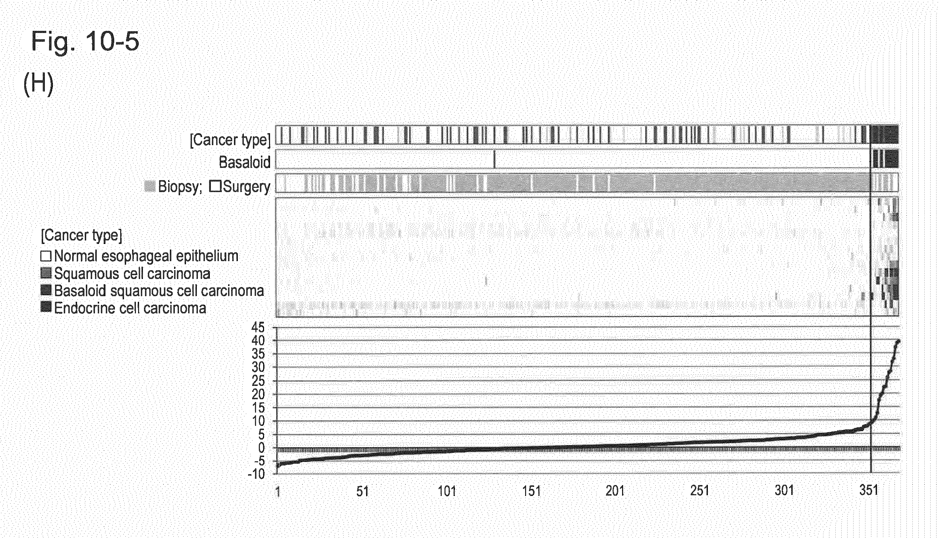

[Figure 10-5] Figure 10-5 (H) shows a heat map chart obtained by re-aligning 57 surgical specimens and 312 biopsy specimens obtained in Example 1 in the ascending order of the expression scores of 15 genes (middle) and a graph plotting the values of the expression scores of 15 genes (bottom) regarding the expression scores of 15 genes for 15 kinds of BSCE markers represented by NOs: R-1 to R-15. The vertical line in the figure represents a cutoff value. In addition, of three bars in the top, the first bar denoted by "Cancer type" indicates distinctions of cancer types (including normal tissues), the second bar denoted by "Basaloid" indicates distinction between BSCE and non-BSCE (colored portions correspond to BSCE), and the bottom bar denoted by "Biopsy; Surgery" indicates distinction between surgical specimens and biopsy specimens.

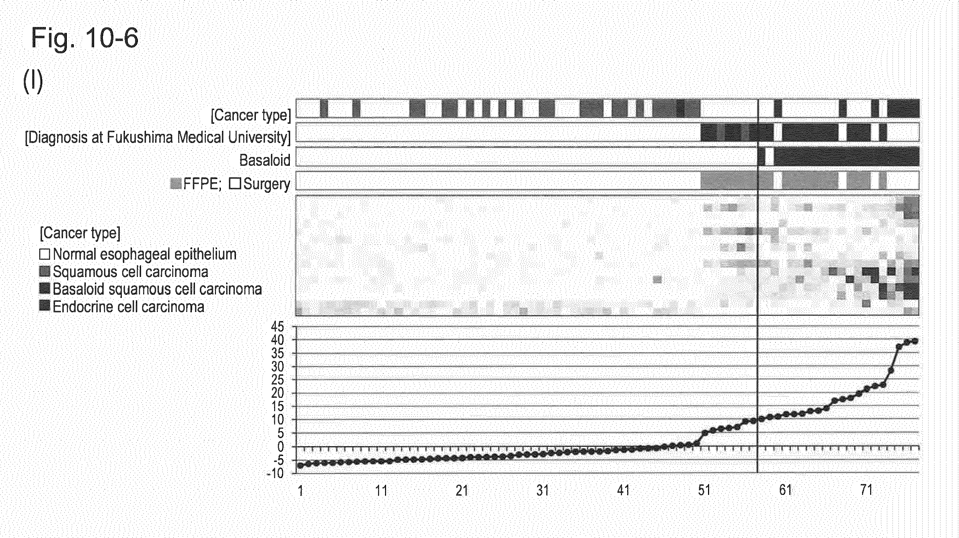

[Figure 10-6] Figure 10-6 (I) shows a heat map chart obtained by re-aligning 57 surgical specimens and 20 FFPE tissue specimens obtained in Example 1 in the ascending order of the expression scores of 15 genes (middle) and a graph plotting the values of the expression scores of 15 genes (bottom) regarding the expression scores of 15 genes for 15 kinds of BSCE markers represented by NOs:. R-1 to R-15. The vertical line in the figure represents a cutoff value. In addition, of four bars in the top, the top bar denoted by "Cancer type" indicates distinctions of cancer types (including normal tissues), the second bar denoted by "Diagnosis at Fukushima Medical University" indicates distinctions of cancer types as a result of pathological diagnosis of FFPE tissue specimens at Fukushima Medical University, the third bar denoted by "Basaloid" indicates distinction between BSCE and non-BSCE (colored portions correspond to BSCE), and the bottom bar denoted by "FFPE; Surgery" indicates distinction between surgical specimens and FFPE tissue specimens.

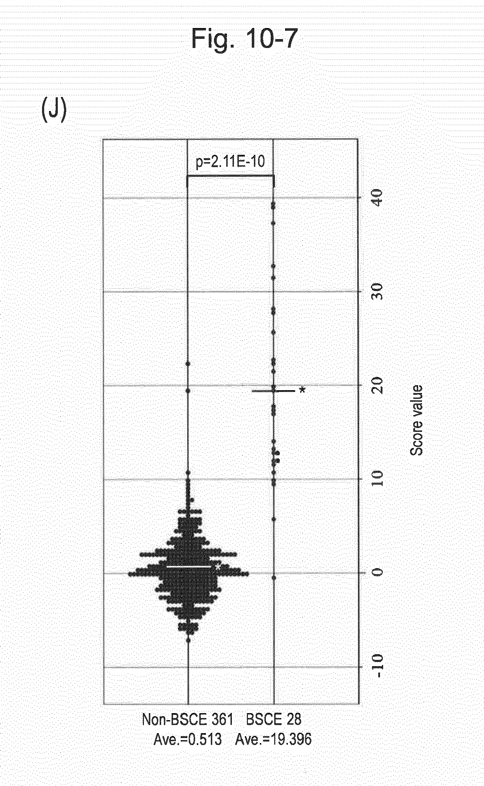

[Figure 10-7] Figure 10-7 (J) is a group scatter diagram for all analyzed specimens obtained using the expression scores of 15 genes for 15 kinds of BSCE markers represented by NOs: R-1 to R-15 when surgical specimens, biopsy specimens, and FFPE tissue specimens areall combined.

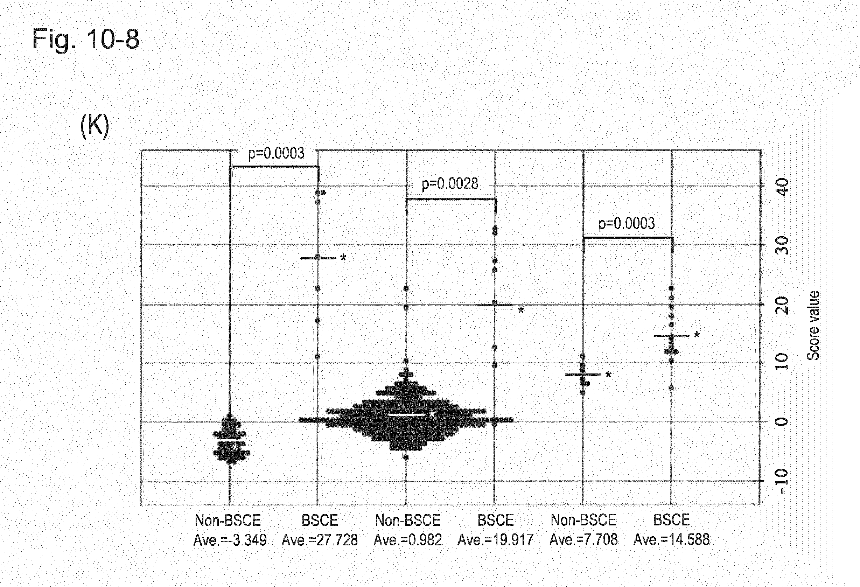

[Figure 10-8] Figure 10-8 (K) is a group scatter diagram created separately for each specimen type.

### Description of Embodiments

### 1. Marker for basaloid squamous cell carcinoma of the esophagus

### 1-1. Outline

Described herein is a marker for basaloid squamous cell carcinoma of the esophagus (BSCE marker). The BSCE marker described herein consists of a group of at least 5 genes. It is possible to differentiate the presence or absence of BSCE or whether the existing esophageal cancer is BSCE or another esophageal cancer by measuring the expression levels of the genes in samples of a test subject and a healthy subject or a group of healthy subjects, thereby providing a correct differential diagnosis.

### 1-2. Definition

The "esophagus" is a tubular organ having a length of 20 to 25 cm which extends from the lower end of the cricoid cartilage to the stomach and is formed with three regions corresponding to the cervical esophagus, the thoracic esophagus, and the abdominal esophagus.

"Esophageal cancer" is a malignant tumor arising from the mucosa of the esophagus. In addition to major esophageal cancers such as squamous cell carcinoma (esophageal squamous cell carcinoma) and adenocarcinoma (esophageal adenocarcinoma), special histopathologic type esophageal cancer such as basaloid squamous cell carcinoma of the esophagus (BSCE) is known.

"Esophageal squamous cell carcinoma" is an esophageal cancer arising from squamous epithelium that is the original esophageal mucosa. Esophageal squamous cell carcinoma forms solid nest, and differentiates into stratified squamous epithelium. Esophageal squamous cell carcinoma tends to keratinize or differentiate into layers. In many cases, intercellular bridges are observed. Esophageal squamous cell carcinoma has a high incidence in the cervical esophagus or thoracic esophagus, accounting for about 90% of esophageal cancers in Japan.

"Esophageal adenocarcinoma" is an esophageal cancer that occurs in glandular cells and it often occurs on the gastric side of the abdominal esophagus. Glandular cells form esophageal glands that act on mucus secretion in the esophagus inner wall. In Western countries, the incidence of Barrett's esophageal adenocarcinoma that occurs with the background of Barrett's esophagus is high, accounting for more than half of esophageal cancers.

"Basaloid squamous cell carcinoma of the esophagus (BSCE)" is a relatively rare esophageal cancer which is classified into a special histopathologic type esophageal cancer as described above. The cancer cells have characteristics similar to basal cells that occur in the esophagus. They are characterized by small cells that grow in solid nests-like or cords-like manner and sometimes form an irregular adenoid, microcystic structure. In addition, deposition of hyaline-like (basal membrane-like) materials is observed inside and outside of the nests. Ductal differentiation of the cancer may be partially observed. Esophageal squamous cell carcinoma is often found in the epithelium and the invasive portion may have esophageal squamous cell carcinoma.

The term "marker for basaloid squamous cell carcinoma of the esophagus (BSCE marker)" used herein refers to a biomarker capable of differentiating the presence or absence of BSCE or differentiating BSCE from other major esophageal cancers (i.e., esophageal squamous cell carcinoma and esophageal adenocarcinoma).

The term "differentiating" used herein means to determine whether or not a test subject with a past history of an esophageal cancer has or had BSCE or whether an esophageal cancer patient has any of BSCE and other major esophageal cancers other than BSCE.

### 1-3. Constitution

Described herein, BSCE markers are composed of a group of genes for differentiating BSCE. The "group of genes for differentiating BSCE" described herein consists of at least 5 genes (the COL9A2 gene, the FGF3 gene, the NPTX2 gene, the COL9A3 gene, and the COL9A1 gene) comprising nucleotide sequences shown in SEQ ID NOs: 141 to 145, respectively. In other words, these 5 genes constitute a group of essential genes for differentiating BSCE for the BSCE markers described herein. The 5 genes correspond to NOs: R-1 to R-5, respectively, in Table 1, and are genes encoding proteins consisting of the amino acid sequences shown in SEQ ID NOs: 71 to 75. Specifically, genes consisting of the nucleotide sequences shown in SEQ ID NOs: 1 to 5 can be exemplified. Each gene that constitutes BSCE markers is herein referred to as a "gene for differentiating BSCE."

**[Table 1]**

| No. | gene name | symbol | ID | SEQ ID NO: | | |
|---|---|---|---|---|---|---|
| | | | | A | B | C |
| R-1 | collagen, type IX, alpha 2 | COL9A2 | NM_001852.3 | 1 | 71 | 141 |
| R-2 | fibroblast growth factor 3 | FGF3 | NM_005247.2 | 2 | 72 | 142 |
| R-3 | neuronal pentraxin II | NPTX2 | NM_002523.2 | 3 | 73 | 143 |
| R-4 | collagen, type IX, alpha 3 | COL9A3 | NM_001853.3 | 4 | 74 | 144 |
| R-5 | collagen, type IX, alpha 1, transcript variant 1 | COL9A1 | NM_001851.4 | 5 | 75 | 145 |
| R-6 | protein phosphatase 1, regulatory (inhibitor) subunit 1B | PPP1R1B | NM_032192.3 | 6 | 76 | 146 |
| R-7 | tweety family member 1, transcript variant 1 | TTYH1 | NM_020659.3 | 7 | 77 | 147 |
| R-8 | ankyrin repeat and BTB (POZ) domain containing 2 | ABTB2 | NM_145804.2 | 8 | 78 | 148 |
| R-9 | tubulin tyrosine ligase-like family member 4 | TTLL4 | NM_014640.4 | 9 | 79 | 149 |
| R-10 | protein tyrosine phosphatase, non-receptor type 5 (striatum-enriched), transcript variant 2 | PTPN5 | NM_032781.3 | 10 | 80 | 150 |
| R-11 | ets variant 6 | ETV6 | NM_001987.4 | 11 | 81 | 151 |
| R-12 | interleukin 17 receptor D | IL17RD | NM_017563.3 | 12 | 82 | 152 |
| R-13 | coiled-coil domain containing 8 | CCDC8 | NM_032040_4 | 13 | 83 | 153 |
| R-14 | low density lipoprotein receptor-related protein 6 | LRP6 | NM_002336.2 | 14 | 84 | 154 |
| R-15 | ATPase, H+ transporting, lysosomal 56/58kDa, V1 subunit B1 | ATP6V1B1 | NM_001692.3 | 15 | 85 | 155 |
| R-16 | ras homolog family member T1. transcript variant 1 | RHOT1 | NM_001033568.2 | 16 | 86 | 156 |
| R-17 | serine/threon ne kinase 36, transcript variant 1 | STK36 | NM_015690.4 | 17 | 87 | 157 |
| R-18 | oxytocin/neurophysin I prepropeptide | OXT | NM_000915.3 | 18 | 88 | 158 |
| R-19 | multiple EGF-like-domains 6 | MEGF6 | NM_001409.3 | 19 | 89 | 159 |
| R-20 | WAP four-disulfide core domain 1 | WFDC1 | NM_021197.3 | 20 | 90 | 160 |
| R-21 | sema domain, transmembrane domain, and cytoplasmic domain, (semaphorin) 6A | SEMA6A | NM_020796.4 | 21 | 91 | 161 |
| R-22 | Rho guanine nucleotide exchange factor 19 | ARHGEF19 | NM_153213.3 | 22 | 92 | 162 |
| R-23 | phospholipase A2, group VI (cytosolic, calcium-independent), transcript variant X18 | PLA2G6 | XM_005261771.3 | 23 | 93 | 163 |
| R-24 | phospholipase C, beta 4, transcript variant 1. | PLCB4 | NM_000933.3 | 24 | 94 | 164 |
| R-25 | fibroblast growth factor receptor 1, transcript variant 1 | FGFR1 | NM_023110.2 | 25 | 95 | 165 |
| R-26 | SRY (sex determining region Y)-box 9 | SOX9 | NM_000346.3 | 26 | 96 | 166 |
| R-27 | ninein-like | NINL | NM_025176.4 | 27 | 97 | 167 |
| R-28 | transmembrane protein 63A | TMEM63A | NM_014698.2 | 28 | 98 | 168 |
| R-29 | SH3 domain containing ring finger 1 | SH3RF1 | NM_020870.3 | 29 | 99 | 169 |
| R-30 | early B-cell factor 4 | EBF4 | NM_001110514.1 | 30 | 100 | 170 |
| R-31 | fibroblast growth factor 19 | FGF19 | NM_005117.2 | 31 | 101 | 171 |
| R-32 | cilia and flagella associated protein 65 | CFAP65 | NM_194302.3 | 32 | 102 | 172 |
| R-33 | chemokine (C motif) ligand 1 | XCL1 | NM_02995.2 | 33 | 103 | 173 |
| R-34 | atrophin 1, transcript variant 2 | ATN 1 | NM_001940.3 | 34 | 104 | 174 |
| R-35 | SRY (sex determining region Y)-box 10 | SOX10 | NM_006941.3 | 35 | 105 | 175 |
| R-36 | transcription factor AP-2 gamma (activating emancer binding protein 2 gamma) | TFAP2C | NM_03222.3 | 36 | 106 | 176 |
| R-37 | transmembrane 4 L six family member 5 | TM4SF5 | NM_003963.2 | 37 | 107 | 177 |
| R-38 | immunoglobulin mu binding protein 2 | IGHMBP2 | NM_002180.2 | 38 | 108 | 178 |
| R-39 | glutathione peroxidase 7 | GPX7 | NM_015696.4 | 39 | 109 | 179 |
| R-40 | sema domain, immunoglobulin domain (Ig), transmembrane domain (TM) and short cytoplasmic domain, (semaphorin) 4C | SEMA4C | NM_017789.4 | 40 | 110 | 180 |
| R-41 | deltex 3, E3 ubiquitin ligase, transcript variant 1 | DTX3 | NM_178502.3 | 41 | 111 | 181 |
| R-42 | inositol polyphosphate-5-phosphatase F, transcript variant 1 | INPP5F | NM_014937.3 | 42 | 112 | 182 |
| R-43 | dedicator of cytokinesis 1, transcript variant 2 | DOCK1 | NM_001380.4 | 43 | 113 | 183 |
| R-44 | RAB, membe of RAS oncogene family-like 2B transcript variant 2 | RABL2B | NM_007081.2 | 44 | 114 | 184 |
| R-45 | nuclear receptor corepressor 2, transcript variant 1 | NCOR2 | NM_006312.5 | 45 | 115 | 185 |
| R-46 | FK506 binding protein 9, 63 kDa, transcript variant 1 | FKBP9 | NM 007270.4 | 46 | 116 | 186 |
| R-47 | inhibitor of growth family, member 4, transcript variant 1 | ING4 | NM_016162.3 | 47 | 117 | 187 |
| R-48 | myeloid/lymphoid or mixed-lineage leukemia; translocated to, 6 | MLLT6 | NM_005937.3 | 48 | 118 | 188 |
| R-49 | intraflagellar transport 172 | IFT172 | NM_015662.2 | 49 | 119 | 189 |
| R-50 | acyl-CoA synthetase short-chain family member 1, transcript variant 1 | ACSS1 | NM_032501.3 | 50 | 120 | 190 |
| R-51 | tripartite motif containing 17 (TRIM17), transcript variant 1 | TRIM17 | NM_016102.3 | 51 | 121 | 191 |
| R-52 | signal peptide, CUB domain, EGF-like 3, transcript variant 1 | SCUBE3 | NM_152753.3 | 52 | 122 | 192 |
| R-53 | EMI domain containing 1, transcript variant 1 | EMID1 | NM_133455.3 | 53 | 123 | 193 |
| R-54 | ral guanine nucleotide dissociation stimulator-like 3, transcript variant 1 | RGL3 | NM_001161616.2 | 54 | 124 | 194 |
| R-55 | sorting nexin 22, transcript variant 1 | SNX22 | NM_024798.2 | 55 | 125 | 195 |
| R-56 | T-box 3, transcript variant 1 | TBX3 | NM_005996.3 | 56 | 126 | 196 |
| R-51 | insulin-like growth factor 1 receptor, transcript variant 1 | IGF1R | NM_000875.4 | 57 | 127 | 197 |
| R-58 | paired immunoglobin-like type 2 receptor beta | PILRB | NM_178238.3 | 58 | 128 | 198 |
| R-59 | KIAA1324-like, transcript variant 1 | KlAA1324L | NM_152748.3 | 59 | 129 | 199 |
| R-60 | frizzled class receptor 1 | FZD1 | NM_003505.1 | 60 | 130 | 200 |
| R-61 | dpy-19-like 2 (C. elegans) | DPY19L2 | NM_173812.4 | 61 | 131 | 201 |
| R-62 | fibrillin 3 | FBN3 | NM_032447.3 | 62 | 132 | 202 |
| R-63 | protein-L-isoaspartate (D-aspartate) O-methyltransferase domain containing 2, transcript variant 1 | PCMTD2 | NM_018257.2 | 63 | 133 | 203 |
| R-64 | TBC1 domain family, member 32, transcript variant 1 | TBC1D32 | NM_152730.5 | 64 | 134 | 204 |
| R-65 | homeobox A9 | HOXA9 | NM_152739.3 | 65 | 135 | 205 |
| R-66 | thioredoxin-related transmembrane protein 4 (TMX4) | TMX4 | NM_021156.3 | 66 | 136 | 206 |
| R-61 | nerve growth factor receptor | NGFR | NM_002507.3 | 67 | 137 | 207 |
| R-68 | UDP-Gal:betaGlcNAc beta 1,4-galactosyltransferase, polypeptide 5 | B4GALT5 | NM_004776.3 | 68 | 138 | 208 |
| R-69 | transmembrane protein 98, transcript variant 1 | TMEM98 | NM_015544.2 | 69 | 139 | 209 |
| R-70 | calpain 6 | CAPN6 | NM_014289.3 | 70 | 140 | 210 |

In Table 1, "gene name" means the name of a gene for differentiating BSCE, "symbol" means the abbreviation for a gene for differentiating BSCE, and "ID" means the accession number of a gene for differentiating BSCE. In addition, "SEQ ID NO" is composed of A denoting the full-length nucleotide sequence of a gene for differentiating BSCE, B denoting the full-length amino acid sequence of a protein encoded by the gene, and C denoting the nucleotide sequence used as a probe.

Described herein, BSCE markers may include selected genes for differentiating BSCE in addition to the group of essential genes for differentiating BSCE. The terms "selected genes for differentiating BSCE" as used herein refer to genes other than essential genes for differentiating BSCE which belong to the group of genes for differentiating BSCE described in Table 1. In a case in which there are a plurality of selected genes for differentiating BSCE, they are often herein referred to as a "group of selected genes for differentiating BSCE."

Described herein, BSCE markers may further include, as selected genes for differentiating BSCE, one or more of 3 genes (the PPP1R1B gene, the TTYH1 gene, and the ABTB2 gene) comprising the nucleotide sequences shown in SEQ ID NOs: 146 to 148, respectively, in addition to the group of essential genes for differentiating BSCE. Preferably, the genes comprising the nucleotide sequences shown in SEQ ID NOs: 146 to 148 correspond to NOs: R-6 to R-8, respectively, in Table 1, and are genes encoding proteins consisting of the amino acid sequences shown in SEQ ID NOs: 76 to 78. For example, genes consisting of the nucleotide sequences shown in SEQ ID NOs: 6 to 8 can be exemplified.

Described herein, BSCE markers may further include, as selected genes for differentiating BSCE, one or more of 5 genes (the TTLL4 gene, the PTPN5 gene, the ETV6 gene, the IL17RD gene, and the CCDC8 gene) comprising the nucleotide sequences shown in SEQ ID NOs: 149 to 153, respectively, in addition to the group of essential genes for differentiating BSCE. Preferably, the genes comprising the nucleotide sequences shown in SEQ ID NOs: 149 to 153 correspond to NOs: R-9 to R-13, respectively, in Table 1, and are genes encoding proteins consisting of the amino acid sequences shown in SEQ ID NOs: 79 to 83. For example, genes consisting of the nucleotide sequences shown in SEQ ID NOs: 9 to 13 can be exemplified. In this case, the BSCE marker may further include at least one of the above-mentioned genes comprising the nucleotide sequences shown in SEQ ID NOs: 146 to 148, which are selected genes for differentiating BSCE, preferably genes encoding proteins consisting of the amino acid sequences shown in SEQ ID NOs: 76 to 78, and more preferably genes consisting of the nucleotide sequences shown in SEQ ID NOs: 6 to 8.

Described herein, BSCE markers may further include, as selected genes for differentiating BSCE, one or more of genes comprising the nucleotide sequences shown in SEQ ID NOs: 154 to 210, respectively, in addition to the group of essential genes for differentiating BSCE. Preferably, the genes comprising the nucleotide sequences shown in SEQ ID NOs: 154 to 210 correspond to NOs: R-14 to R-70, respectively, in Table 1, and are genes encoding proteins consisting of the amino acid sequences shown in SEQ ID NOs: 84 to 140. For example, genes consisting of the nucleotide sequences shown in SEQ ID NOs: 14 to 70 can be exemplified. In this case, the BSCE marker may further include at least one of the above-mentioned genes comprising the nucleotide sequences shown in SEQ ID NOs: 146 to 153, which are selected genes for differentiating BSCE, preferably genes encoding proteins consisting of the amino acid sequences shown in SEQ ID NOs: 76 to 83, and more preferably genes consisting of the nucleotide sequences shown in SEQ ID NOs: 6 to 13.

Therefore, the BSCE markers may be composed of a group of 5 to 70 genes for differentiating BSCE. In general, the BSCE markers have higher degrees of differentiation accuracy according to the method for differentiating the contraction of BSCE as the number of kinds of genes included therein increases.

In description, the BSCE markers described herein are genes consisting of nucleotide sequences of DNAs (cDNAs). However, mRNAs consisting of RNA sequences which are generated as transcription products upon expression of the genes, proteins consisting of amino acid sequences generated as translation products of the genes, and partial fragments thereof also reflect the expression of genes as the markers, and therefore, they can indirectly serve as BSCE markers.

In addition, genes for differentiating BSCE that constitute BSCE markers described herein also include nucleotides having 70% or more, 75% or more, 80% or more, 85% or more, 90% or more, 95% or more, 96% or more, 97% or more, 98% or more, or 99% or more base identity with the nucleotide sequences of the above-described genes, nucleotides with a deletion, substitution, or addition of one or more nucleotides in the nucleotide sequences of the above-described genes, and nucleotides wherein the nucleic acids hybridize to nucleotide fragments consisting of nucleotide sequences complementary to partial nucleotide sequences of the genes under stringent conditions and the enzymatic activity is retained. Specific examples of such nucleotides include nucleotides comprising degenerate codons encoding the same amino acid sequence, mutated genes such as various mutants (variants) of individual genes and point-mutated genes, and ortholog genes of organisms of other species such as chimpanzee. The term "base identity" used herein refers to a percentage (%) of the number of identical bases in nucleotide sequences of nucleotides to be compared with respect to the total number of bases of genes described in Table 1 when two nucleotide sequences are aligned, if necessary with a gap such that the degree of matching between both nucleotide sequences is maximized. The terms "a plurality of nucleotides" mean 2 to 30 nucleotides, 2 to 14 nucleotides, 2 to 10 nucleotides, 2 to 8 nucleotides, 2 to 6 nucleotides, 2 to 5 nucleotides, 2 to 4 nucleotides, or 2 to 3 nucleotides. In addition, the term "stringent conditions" refers to conditions that do not cause a non-specific hybrid to be formed. In general, lower salt concentrations at higher temperatures tend to result in highly stringent conditions. Low stringent conditions correspond to, for example, conditions for washing after hybridization at 1×SSC, 0.1% SDS, and about 37°C and more strictly at 0.5×SSC, 0.1% SDS, and about 42°C to 50°C. More highly stringent conditions correspond to conditions for washing after hybridization at, for example, 50°C to 70°C, 55°C to 68°C, or 65°C to 68°C with 0.1×SSC and 0.1% SDS. In general, highly stringent conditions are preferable. The combinations of SSC, SDS, and the temperature described above are merely examples. Persons skilled in the art also can determine stringency of hybridization by combining the probe concentration, probe base length, hybridization time, and other conditions with SSC, SDS, and the temperature, as appropriate.

### 2. Method for differentiating contraction of basaloid squamous cell carcinoma of the esophagus

### 2-1. Outline

The first aspect of the present invention relates to a method for assisting differentiating contraction of basaloid squamous cell carcinoma of the esophagus (BSCE) in a test subject. The method for differentiating contraction of BSCE of the present invention is a method comprising obtaining expression profiles of the BSCE markers which are at least the 5 genes comprising nucleotide sequences shown in SEQ ID NOs: 141 to 145, which are contained in samples collected from a test subject and a healthy subject or a group of healthy subjects, and assisting differentiating whether an esophageal cancer in the test subject is basaloid squamous cell carcinoma of the esophagus or another esophageal cancer based on the expression profiles. The terms "expression profile of the BSCE marker" mean information on the expression level of each of genes for differentiating BSCE included in BSCE markers. The expression profiles described herein especially correspond to gene expression patterns established based on information about the expression levels of a plurality of genes for differentiating BSCE. In general, obtaining more gene expression profiles enables differentiation with higher degrees of accuracy.

### 2-2. Method

The method for differentiating contraction of BSCE of the present invention comprises a measurement step, a calculation step, and a determination step as essential steps. Hereinafter, each step is specifically explained.

### 2-2-1. Measurement step

The term "measurement step" is a step of measuring expression levels of the BSCE markers which are at least the 5 genes comprising nucleotide sequences shown in SEQ ID NOs: 141 to 145 per unit amount of samples collected from a test subject and a healthy subject or a group of healthy subjects, thereby obtaining measurement values thereof.

The term "test subject" used herein refers to a human individual who provides a sample and is subjected to a test. The test subject may be either an individual having a past history of an esophageal cancer or an individual suspected to be affected with an esophageal cancer. The term "individual having a past history of an esophageal cancer" described herein may include a patient currently affected with an esophageal cancer and a person having a past history of an esophageal cancer who was affected with an esophageal cancer in the past. Since histopathological distinction of BSCE from esophageal squamous cell carcinoma is difficult, a patient who has been histopathologically diagnosed as suspected esophageal squamous cell carcinoma or a person having a past history of esophageal squamous cell carcinoma is a suitable test subject.

The term "a healthy subject" used herein refers to a human in a healthy state. Note that healthy human cells are herein included in healthy subjects in a broad sense. It is therefore determined that a human in a healthy state not only at the level of individuals but also at the level of cells, which means, for example, a normal portion of a tissue collected from an esophageal cancer patient, can be referred to as a healthy subject.

The term "healthy state" used herein refers to a state in which a person is not affected with at least an esophageal cancer. In other words, the healthy subject described herein is a human individual (including human cells) who is not affected with esophageal cancer, preferably a healthy human individual (including human cells) without any disease or abnormality. A healthy subject used in this aspect is not particularly limited in terms of physical conditions such as gender, age, height, and weight and the number of individuals. However, it is preferable that the person is of the same gender of a test subject and has physical conditions such as age, height, and weight identical or similar to the test subject. A group consisting of a plurality of healthy subjects is herein referred to as a "group of healthy subjects."

The term "sample" used herein refers to an object that is collected from the test subject or the healthy subject or the group of healthy subjects and subjected to the method for differentiating contraction of BSCE in this aspect, which corresponds to, for example, a tissue, cells, body fluid, or peritoneal washing fluid. The term "tissue" and "cells" described herein may be derived from any sites of a test subject or a healthy subject. However, they are preferably specimens collected by biopsy or excised by surgery and more specifically an esophageal tissue or esophageal cells. Esophageal cancer cells collected by biopsy or an esophageal tissue or esophageal cells of a suspected esophageal cancer are particularly preferable. Such tissue or cells may be formalin-fixed and then paraffin-embedded (FFPE: Formalin-Fixed Paraffin Embedded). The term "body fluid" used herein refers to a liquid biological sample collected from a test subject or a healthy subject. Examples of body fluid include blood (including serum, plasma and interstitial fluid), spinal fluid (cerebrospinal fluid), urine, lymph, digestive fluid, ascitic fluid, pleural effusion, fluid surrounding nerve roots, and extracts of tissues or cells. Blood is preferable.

A tissue or cells can be collected as samples by biopsy or surgical excision. In addition, body fluid can be collected as a sample by a method known in the art. For example, blood or lymph can be collected as a sample in accordance with a known blood collection method. The amount of a sample required for the method for differentiating contraction of BSCE in this aspect is not particularly limited. It is desirable to collect a tissue or cells in an amount of at least 10 µg, preferably at least 0.1 mg. However, a tissue or cells may be obtained as a biopsy material. In addition, the sufficient amount of body fluid such as blood or lymph can be at least 0.1 mL, preferably at least 1 mL, and more preferably at least 10 mL. If necessary, a sample can be prepared and treated such that the BSCE markers which are at least the 5 genes comprising nucleotide sequences shown in SEQ ID NOs: 141 to 145 can be measured. For example, if a sample is a tissue or cells, homogenization, cell lysis treatment, removal of contaminants by centrifugation or filtration, the addition of protease inhibitors, etc. are exemplified. Details of these treatment procedures are specifically described in Green & Sambrook, Molecular Cloning, 2012, Fourth Ed., Cold Spring Harbor Laboratory Press for reference.

The term "unit amount" used herein refers to any determined sample amount. For example, it corresponds to a volume (expressed in µL or mL) or a weight (expressed in µg, mg, or g). Although the unit amount is not particularly specified, the same unit amount is employed for a series of measurements according to the method for differentiating contraction of BSCE. Therefore, the unit amount of a sample from a test subject and the unit amount of a sample from a healthy subject are always the same when measured in this step. The unit amount may be changed when the method for differentiating contraction of BSCE is conducted in a new setting. It is, however, convenient to use the same unit amount in each method for differentiating contraction of BSCE, thereby making it possible to reuse measurement values of the previous measurement as measurement value for a healthy subject or a group of healthy subjects as long as the other conditions are the same without the need to conduct measurement every time the method for differentiating contraction of BSCE is carried out.

The term "gene expression level" used herein refers to the expression level of a gene for differentiating BSCE (the amount of a transcription product), expression intensity, or expression frequency. The gene expression level described herein is not limited to the expression level of a wild-type gene for differentiating BSCE and thus may include the expression level of a mutant gene such as a point-mutated gene. In addition, transcription products showing expression of genes for differentiating BSCE may include atypical transcription products (variants) such as splice variants and fragments thereof. This is because it is possible to construct a gene expression profile according to the method of the present invention even with information based on mutant genes, transcription products, or fragments thereof. The gene expression levels can be obtained as measurement values by measuring, e.g., transcription products of a group of genes for differentiating BSCE included in BSCE markers, i.e., mRNA amounts or the amounts or activity levels of proteins as translation products of mRNA.

The term "measurement value" used herein refers to a value obtained by method for measuring a gene expression level. The measurement value may be an absolute value of the mRNA amount or protein amount in a sample expressed in a unit such as nanogram (ng) or microgram (µg), or a relative value expressed as absorbance relative to a control value, fluorescence intensity of a labeled molecule, or the like. Further, in the case of the measurement value of protein activity, it may be specific activity.

Hereinafter, a method for measuring a transcription product or a translation product of each gene for differentiating BSCE will be specifically described.

### (1) Measurement of transcription products

Measurement of transcription products of genes for differentiating BSCE may be measurement of mRNA amounts or measurement of amounts of cDNAs obtained through reverse transcription of mRNA. In general, for measurement of a transcription product of a gene, a method for measuring the gene expression level as an absolute value or relative value using a nucleotide comprising all or a part of the nucleotide sequence of the above-described gene as a primer or a probe is employed.

In this aspect, primers or probes are usually composed of naturally occurring nucleic acids such as DNA and RNA. DNA is particularly preferable because of high stability and the ease of synthesis at an inexpensive cost. It is also possible to combine naturally occurring nucleic acids and chemically modified nucleic acids or pseudo nucleic acids as needed. Examples of chemically modified nucleic acids or pseudo nucleic acids include peptide nucleic acid (PNA), Locked Nucleic Acid (LNA; registered trademark), methyl phosphonate DNA, a phosphorothioate DNA, and 2'-O-methyl RNA. Further, the primers and probes may be labeled or modified with fluorescent substances and/or quencher substances, or labeling substances such as radioactive isotopes (e.g., ³²P, ³³P, ³⁵S), or modifiers such as biotin or (strept) avidin and magnetic beads. Labeling substances are not limited, and commercially available products can be used. For example, it is possible to use fluorescent substances such as FITC, Texas, Cy3, Cy5, Cy7, Cyanine3, Cyanine5, Cyanine7, FAM, HEX, VIC, fluorescamine and derivatives thereof, and rhodamine and derivatives thereof. As quencher substances, AMRA, DABCYL, BHQ-1, BHQ-2, BHQ-3, etc. can be used. A position for labeling a primer or a probe with a labeling substance can be determined, as appropriate, depending on the characteristics or intended use of a modifier. In general, the 5 'or 3' end is often modified. In addition, a single primer or probe molecule may be labeled with one or more labeling substances. A nucleotide can be labeled with such substance by a known method.

A nucleotide used as a primer or probe may be any nucleotide consisting of a sense strand or an antisense strand of each of genes included in the BSCE markers which are at least the 5 genes comprising nucleotide sequences shown in SEQ ID NOs: 141 to 145.

The base length of a primer or a probe is not particularly limited. In a case in which a probe is used in a hybridization method described later, the base length thereof is from at least a 10-base length to the full-length of a gene for differentiating BSCE, preferably from a 15-base length to the full-length of a gene for differentiating BSCE, more preferably from a 30-base length to the full-length of a gene for differentiating BSCE, and further preferably from a 50-base length to the full-length of a gene for differentiating BSCE. In a case in which a probe is used for a microarray, the base length thereof is a 10- to 200-base length, preferably a 20- to 150-base length, and more preferably a 30- to 100-base length. In general, a longer probe results in higher hybridization efficiency and higher sensitivity. On the other hand, a shorter probe results in lower sensitivity, but conversely, it also results in higher specificity. Specific examples of a probe include nucleotides of 80 bases consisting of nucleotide sequences shown in SEQ ID NOs: 141 to 210 in C in Table 1. Meanwhile, for primers, each of a forward primer and a reverse primer may have a length of 10 to 50 bases, preferably 15 to 30 bases.

Preparation of the above-described primers or probes is known to persons skilled in the art. For example, they can be prepared according to the method described in Green & Sambrook, Molecular Cloning (2012) mentioned above. It is also possible to provide a contracted manufacturer for nucleic acid synthesis with sequence information for commissioned manufacturing.

Measurement of transcription products of genes for differentiating BSCE may be conducted by a known nucleic acid quantification method and it is not particularly limited. For example, the hybridization method or the nucleic acid amplification method is exemplified.

The term "hybridization method" refers to a method for detecting or quantifying a target nucleic acid or a fragment thereof using, as a probe, a nucleic acid fragment having a nucleotide sequence complementary to all or a part of the nucleotide sequence of a target nucleic acid to be detected and utilizing base pairing between the nucleic acid and the probe. In this aspect, target nucleic acids are mRNAs or cDNAs of each gene for differentiating BSCE included in BSCE markers or fragments thereof. In general, the hybridization method is preferably carried out under stringent conditions to eliminate non-target nucleic acids that is nonspecifically hybridized. The above-described highly stringent conditions at a low salt concentration and a high temperature are more preferable. There are several known hybridization methods involving different detection means, which are preferably, for example, a Northern blot method (Northern hybridization method), a microarray method, a surface plasmon resonance method, and a quartz crystal microbalance method.

The term "Northern blot method" is the most common method for analyzing gene expression, in which total RNA or mRNA prepared from a sample is separated by electrophoresis through agarose gel or polyacrylamide gel, etc. before blotting on a filter, and then, a target nucleic acid is detected using a probe having a nucleotide sequence specific to a target RNA. It is also possible to quantify a target nucleic acid by labeling the probe with a suitable marker such as a fluorescent dye or a radioactive isotope, and by, for example, a measurement device such as a chemiluminescence imaging system (e.g., Light Capture; ATTO Corporation), a scintillation counter, or an imaging analyzer (e.g., Fujifilm Corporation: BAS series). The Northern blot method is a well-known prominent technique in the art. For example, Green, M.R. and Sambrook, J. (2012) mentioned above can be referred to.

The term "microarray method" is a method for detecting and quantifying a nucleic acid hybridized to an array spot by fluorescence or the like, in which a sample containing a target nucleic acid is allowed to react on a microarray or microchip in which a nucleic acid fragment complementary to all or a part of the nucleotide sequence of the target nucleic acid as a probe is disposed as a small spot at a high density on a substrate and immobilized. The target nucleic acid may be RNA such as mRNA or DNA such as cDNA. Detection and quantification can be achieved by detecting and measuring fluorescence or the like based on the hybridization of the target nucleic acid or the like using a microplate reader or a scanner. Based on the measured fluorescence intensity, it is possible to detect the amount of mRNA or the cDNA or the abundance ratio thereof relative to reference mRNA. The microarray method is also a technique well-known in the art. For example, DNA Microarray Method (DNA microarray and the latest PCR method (2000); editorial supervisors: Masaaki Muramatsu and Hiroyuki Nawa; Shujunsha Co., Ltd) may be referred to.

The term "surface plasmon resonance (SPR) method" is a method for detecting and quantifying an adsorbate on the surface of a metal thin film at very high sensitivity by utilizing the surface plasmon resonance phenomenon, in which the reflected light intensity is significantly attenuated in a certain incidence angle (resonance angle) when the incident angle of laser light applied to the metal thin film is changed. In the present invention, for example, a probe having a sequence complementary to the nucleotide sequence of a target nucleic acid is immobilized to the surface of a metal thin film, other portions of the surface of the metal thin film are treated by blocking, and a sample collected from a test subject or a healthy subject or a group of healthy subjects is distributed on the surface of the metal thin film, thereby allowing base pairing to form between the target nucleic acid and the probe so as to detect or quantify the target nucleic acid based on the difference in measurement values before and after the distribution of the sample. Detection and quantification by the surface plasmon resonance method can be carried out using, for example, an SPR sensor marketed by Biacore, Inc. This technology is well known in the art. For example, Kazuhiro Nagata and Hiroshi Handa, Real-time Analysis Experimental Method for Interaction of Biological Materials, Springer-Verlag Tokyo, Tokyo, 2000 can be referred to.

The term "quartz crystal microbalance (QCM) method" is a mass measurement method for quantitatively capturing a very small amount of an adsorbate based on the amount of change in resonance frequency by utilizing the phenomenon that when a substance is adsorbed to the surface of an electrode attached to a crystal oscillator, the resonance frequency of the crystal oscillator decreases in accordance with the mass of the substance. In the case of detection and quantification according to the this method, it is also possible to detect and quantify a target nucleic acid based on, for example, base pairing between a probe having a sequence complementary to the nucleotide sequence of the target nucleic acid immobilized on the electrode surface and the target nucleic acid in a sample collected from a test subject or a healthy subject or a group of healthy subjects by utilizing the commercially available QCM sensor as in the SPR method. This technique is well known in the art. For example, Christopher J. et al., 2005, Self-Assembled Monolayers of a Form of Nanotechnology, Chemical Review, 105:1103-1169 and Toyosaka Moriizumi, Takamichi Nakamoto, (1997) Sensor Engineering, Shokodo Co., Ltd. can be referred to.

The term "nucleic acid amplification method" is a method for amplifying a specific region of a target nucleic acid by a nucleic acid polymerase using forward/reverse primers. For example, the PCR method (including the RT-PCR method), the NASBA method, the ICAN method, and the LAMP (registered trademark) (including the RT-LAMP method) are exemplified. The PCR method is preferable. As a method for measuring a transcription product of a gene using the nucleic acid amplification method, a quantitative nucleic acid amplification method such as the real-time RT-PCR method is used. As the real-time RT-PCR method, an intercalator method using SYBR (registered trademark) Green or the like, a Taqman (registered trademark) probe method, a digital PCR method, and a cycling probe method are known, and any of such methods can be used. Any of these methods is a known method and described in an appropriate protocol in the art. Therefore, such protocol can be referred to.

A method for quantifying transcription product of a gene by real-time RT-PCR method will be briefly described below by way of an example. The real-time RT-PCR method is a method for quantifying a nucleic acid by PCR using a temperature cycler system provided with a function to detect fluorescence intensity derived from an amplification product in a reaction system in which a PCR amplification product is specifically fluorescence-labeled using, as a template, cDNA prepared from mRNA in a sample by reverse transcription reaction. The amount of the amplification product of the target nucleic acid during reaction is monitored in real time, and the results are regression-analyzed by a computer. The method for labeling an amplification product may be a method using fluorescence-labeled probes (e.g., the TaqMan (registered trademark) PCR method) and an intercalator method using a reagent which specifically binds to double-stranded DNA. The TaqMan (registered trademark) PCR method uses a probe modified with a quencher substance at the 5'end and a fluorescent dye at the 3'end, Usually, the quencher substance at the 5'end suppresses the fluorescent dye at the 3' end. However, as a result of PCR, the probe is degraded due to 5'->3' exonuclease activity of the Taq polymerase, which releases the suppression by the quencher substance. This results in emission of fluorescence. The fluorescence amount reflects the amount of the amplification product. Since the cycle number (CT) when the amplification product reaches the detection limit is inversely related to the initial template amount, the initial template amount is quantified by measuring CT in the real-time measurement method. An absolute value of the initial template amount of an unknown sample can be calculated with a calibration curve created by measuring CT for a template of known several different amounts. As a reverse transcriptase used in RT-PCR, for example, M-MLV RTase, ExScriptRTase (Takara Bio Inc.), Super Script II RT (Thermo Fisher Scientific Inc.), or the like can be used.

In general, reaction conditions for real-time PCR would vary depending on the base length of a nucleic acid fragment to be amplified, the amount of a nucleic acid to be used as a template, the base lengths and Tm values of primers to be used, the optimal reaction temperature and optimal pH of a nucleic acid polymerase to be used, etc. Therefore, the reaction conditions can be determined as appropriate based on the known PCR method in consideration of these factors. As an example, usually, it is possible to perform an elongation reaction by repeating about 15 to 40 cycles including as one cycle a denaturation reaction at 94°C to 95°C for 5 seconds to 5 minutes, an annealing reaction at 50°C to 70°C for 10 seconds to 1 minute, and an elongation reaction at 68°C to 72°C for 30 seconds to 3 minutes. In a case in which a commercially available kit marketed by a manufacturer is used, real-time PCR may be performed in accordance with the protocol provided with the kit in principle.

The nucleic acid polymerase used in real-time PCR is a DNA polymerase, and in particular, a heat-resistant DNA polymerase. Such a nucleic acid polymerase is commercially available in various kinds, and it is also possible to utilize these commercially available products. For example, Taq DNA polymerase provided with the Applied Biosystems TaqMan MicroRNA Assays Kit (Thermo Fisher Scientific, Inc.) is exemplified. Such a commercially available kit is very useful because a buffer or the like optimized for activity of the provided DNA polymerase is included therewith.

### (2) Measurement of translation products

Measurement of translation products of genes for differentiating BSCE may be measurement of the amounts of proteins (peptides) encoded by genes for differentiating BSCE or measurement of activity levels of the proteins.

### (2-1) Measurement of the protein amount

In general, for measurement of the amount of a protein, a method in which a binding agent that recognizes the amino acid sequence of the protein, and in particular, a highly specific and characteristic amino acid sequence, and binds the amino acid sequence is employed. Examples of such binding agent include an antibody or an antibody fragment thereof and a nucleic acid aptamer.

### (Antibody)

An antibody or a fragment thereof for use in this step recognizes, as an epitope, a part of a protein encoded by a gene for differentiating BSCE (BSCR marker protein) and specifically binds thereto by an antigen-antibody reaction, thereby making it possible to detect the BSCE marker protein. The term "part" used herein means a region consisting of 5 to 15, preferably 5 to 10, and more preferably 6 to 10 contiguous amino acids.

The antibody used in this step may be any of a polyclonal antibody, a monoclonal antibody, and a recombinant antibody. To allow more specific detection, a monoclonal antibody or a recombinant antibody is preferred. Globulin type of the antibody is not particularly limited and it may be any of IgG, IgM, IgA, IgE, IgD, and IgY. IgG and IgM are preferred. In addition, the species of organism as the origin of the antibody in this aspect is not particularly limited. It can be any animal including a mammal or a bird. Examples thereof include mice, rats, guinea pigs, rabbits, goats, donkeys, sheep, camels, horses, chickens, and humans.

The term "recombinant antibody" used herein refers to, for example, a chimeric antibody, a humanized antibody, or a synthetic antibody.

The term "chimeric antibody" refers to an antibody obtained by replacing the constant regions (C regions) of the light and heavy chains of a certain antibody with the C regions of the light and heavy chains of another antibody. For example, in a mouse anti-human monoclonal antibody, an antibody which substituted the C regions of the light and heavy chains thereof by the C regions of a suitable human antibody corresponds to a chimeric antibody. In other words, in this case, a variable region (V region) including CDR is derived from the mouse antibody and the C region is derived from the human antibody.

The term "humanized antibody" is also referred to as a "reconstruction (reshaped) human antibody" which is a mosaic antibody obtained by substituting CDR in an antibody from a non-human mammal for a target antigen by CDR of a human antibody. For example, an antibody obtained by preparing a recombinant antibody gene by substituting DNA sequences encoding each CDR region (CDR1 to CDR3) of a mouse anti-human FBXO32 antibody by DNA sequences encoding each corresponding CDR region from an appropriate human antibody and allowing the gene to be expressed corresponds to a humanized antibody.

The term "synthetic antibody" refers to an antibody synthesized by a chemical method or a recombinant DNA method. For example, a monomeric polypeptide molecule obtained by artificially linking at least one VL and at least one VH of a certain antibody via a linker peptide or the like having a suitable length and a suitable sequence or a multimeric polypeptide thereof corresponds to a synthetic antibody. Specific examples of such polypeptide include single-chain Fv (scFv: single chain Fragment of variable region) (see Pierce Catalog and Handbook, 1994-1995, Pierce Chemical Co., Rockford, IL), a diabody, a triabody, and a tetrabody. Usually, VL and VH are located on separate polypeptide chains (the light chain and the heavy chain) in an immunoglobulin molecule. Single-chain Fv is a synthetic antibody fragment having a structure in which V regions on these two polypeptide chains are linked via a flexible linker having a sufficient length such that the regions are included in a single polypeptide chain. Both V regions in a single-chain Fv can form one functional antigen binding site by self-assembling with each other. Single-chain Fv can be obtained by integrating a recombinant DNA encoding it into a phage genome using a known technique and allowing the DNA to be expressed. A diabody is a molecule having a structure based on the dimer structure of single-chain Fv (Holliger et al., 1993, Proc. Natl. Acad. Sci. USA 90:6444-6448). A triabody and a tetrabody have a trimeric structure and a tetrameric structure, respectively, based on the single-chain Fv structure as in a diabody. They are trivalent and tetravalent antibody fragments, respectively, and they may be multi-specific antibodies.

It is preferable that an antibody used in this step has high affinity with a BSCE marker protein having a dissociation constant of 10⁻⁸ M or less, preferably 10⁻⁹ M or less, and more preferably 10⁻¹⁰ M or less. The dissociation constant can be determined using a technique known in the art. For example, it may be determined using speed evaluation kit software by the BIAcore system (GE Healthcare Inc.).

A polyclonal antibody of this aspect used in this step can be obtained by a method known in the art for immunizing a suitable animal with a BSCE marker protein. Further, a monoclonal antibody can also be obtained by a known method which is a commonly used technique in the art. For example, after immunization of a mouse or the like with a BSCE marker protein or a peptide fragment thereof, antibody-producing cells are collected from the immunized animal. The antibody-producing cells are fused to a myeloma cell line, thereby generating hybridoma cells. Accordingly, a hybridoma, which produces a monoclonal antibody that binds to a BSCE marker protein or the like used as a target antigen, may be identified.

Examples of an "antibody fragment" include Fab, F(ab')₂, and Fv, which are peptide fragments having antigen-binding activity of any antibody described above.

An antibody or an antibody fragment thereof for use in this step may be modified. The term "modification" used herein includes labeling necessary for antibody detection or functional modification necessary for antigen-specific binding activation. Labeling includes, for example, labeling with the above-described fluorescent materials, fluorescent proteins (e.g., PE, APC, and GFP), enzymes (e.g., horseradish peroxidase, alkaline phosphatase, and glucose oxidase), or biotin or (strept)avidin. In addition, an example of modification is glycosylation of an antibody, which is performed for adjusting the affinity to a BSCE marker protein as a target antigen. A specific example is modification for causing loss of glycosylation at a glycosylation site by introducing a substitution into an amino acid residue constituting the glycosylation site in the framework (FR) region of an antibody so as to remove the glycosylation site.

### (Nucleic acid aptamer)

The term "nucleic acid aptamer" refers to an aptamer composed of nucleic acids, which is a ligand molecule capable of specifically inhibiting or suppressing the function of a target substance, such as physiological activity of the target substance, by strongly and specifically binding to the target substance, wherein the binding is provided by its conformation formed based on a secondary structure and also a tertiary structure of single-stranded nucleic acid molecules via a hydrogen bonding or the like. Nucleotides constituting a nucleic acid aptamer are usually RNAs and/or DNAs which are natural nucleic acids. However, they may include non-natural nucleotides which are capable of transcription or replication. Preferably, an RNA aptamer consisting of RNAs only or a DNA aptamer consisting of DNAs only is employed.

A nucleic acid aptamer for use in this step can be prepared using a BSCE marker protein as a target molecule by a method known in the art. For example, an in vitro screening method based on the systematic evolution of ligands by exponential enrichment (SELEX) method is exemplified. For example, in the case of separating an RNA aptamer, the SELEX method is a method comprising repeating a series of cycles of "selecting an RNA molecule bound to a BSCE marker protein as a target molecule from an RNA pool composed of many RNA molecules having a random sequence region and primer binding regions at both ends of the random sequence region, amplifying the recovered RNA molecule by an RT-PCR reaction, conducting transcription using the obtained cDNA molecule as a template to obtain amplification products of the selected RNA molecule, and preparing an RNA pool of the amplification products for the next round" for several rounds to several tens of rounds, thereby selecting RNA molecules having stronger ability to bind to a target molecule. Meanwhile, in the case of separating DNA aptamer, the SELEX method is a method comprising repeating a series of cycles of "selecting a DNA molecule bound to a BSCE marker protein as a target molecule from a DNA pool composed of many DNA molecules having a random sequence region and primer binding regions at both ends of the random sequence region, amplifying the selected DNA molecule by a PCR reaction, and preparing a DNA pool of the amplification products for the next round" for several rounds to several tens of rounds, thereby selecting a DNA molecule having stronger ability to bind to a target molecule. The nucleotide sequence lengths of the random sequence region and the primer binding region are not particularly limited. In general, preferably, the random sequence region is in the range of 20 to 80 bases, and the primer binding region is in the range of 15 to 40 bases. To increase the specificity to a target molecule, a molecule similar to the target molecule may be mixed with an RNA pool or an RNA pool in advance, resulting in a pool consisting of RNA molecules or DNA molecules that do not bind to the target molecule, and this resulted pool may be used. The nucleic acid molecules eventually obtained by the above method are used as nucleic acid aptamers of this aspect. The SELEX method is a known method, and therefore, it can be specifically carried out in accordance with, for example, Pan et al. (Proc. Natl. Acad. Sci. 1995, U.S.A.92: 11509-11513).

A nucleic acid aptamer for use in this step can also be labeled with a labeling substance such as a fluorescent substance. Regarding type of the labeling substance, any of the above-described labeling substances can be used as long as it does not inhibit the ability to bind to a target molecule. In addition, as in a case in which a reagent for differentiating basaloid squamous cell carcinoma of the esophagus is the above-described probe, a nucleic acid aptamer can also be provided in a state of being immobilized to a solid support. Material of the solid support to immobilize a nucleic acid aptamer is not limited. For example, it is possible to use the same material as the substrate described above.

### (2-2) Measurement of protein activity

For measurement of activity of a protein, a measurement method corresponding to activity of the protein is employed. For example, in a case in which a BSCE marker protein binds to another substance so as to function, as in a relationship between a peptide and a protein, such as a ligand and a receptor, or a transcription factor and DNA, a low molecular weight compound and a protein, or a protein and a nucleic acid, binding activity of a BSCE marker protein maybe measured. A specific example of measuring binding activity is a measurement method using the surface plasmon resonance method or the quartz crystal microbalance method described above. For example, in a case in which a BSCE marker protein is a transcription factor, activity of the BSCE marker protein can be measured by immobilizing target DNA sequence on the metal thin film surface and distributing a sample collected from a subject, a healthy subject, or a group of healthy subjects that may include a BSCE marker protein on the metal thin film surface, thereby detecting the amount of the BSCE marker protein bound to the target DNA sequence. Further, in a case in which a BSCE marker protein functions as an enzyme, catalytic activity thereof can be measured by adding a substrate.

### 2-2-2. Calculation step

The term "calculation step" refers to a step of calculating particular values based on measurement values obtained in the measurement step. This step includes two independent steps, which are a combined value calculation step and an average measurement value calculation step, and either or both of the steps can be selected.

### (a) Combined value calculation step

In the combined value calculation step, the expression ratio of each of genes for differentiating BSCE included in BSCE markers based on the measurement values obtained in the measurement step is calculated, thereby obtaining the combined value thereof. The term "gene expression ratio" used herein refers to a ratio calculated based on the measurement values for a test subject and a healthy subject or a group of healthy subjects obtained in the measurement step for each of genes included in BSCE markers. The expression ratio is calculated as a ratio relative to a sample common to all specimens (hereinafter referred to as "common reference"). The common reference for calculation of the expression ratio may be anything as long as it is common to all specimens. For example, it may be a particular cell line or a mixture of a plurality of cell lines. Alternatively, it may be a healthy subject. In such case, it is preferable to adopt a group of a plurality of healthy subjects, although only one healthy subject may be adopted.

In calculation of the expression ratio, specifically, when, for example, A1 represents a measurement value of a test subject and B1 represents a measurement value of a common reference for the COL9A2 gene, which is included in the group of essential genes for differentiating BSCE for BSCE markers, the expression ratio of the COL9A2 gene is expressed as A1/B1. Similarly, when, for example, A2 represents a measurement value of a test subject and B2 represents a measurement value of a common reference for the FGF3 gene, the expression ratio of the FGF3 gene is expressed as A2/B2. In addition, when A3 represents a measurement value of a test subject and B3 represents a measurement value of a common reference for the NTPX2 gene, the expression ratio of the NTPX2 gene is expressed as A3/B3, when A4 represents a measurement value of a test subject and B4 represents a measurement value of a common reference for the COL9A3 gene, the expression ratio of the COL9A3 gene is expressed as A4/B4, and when A5 represents a measurement value of a test subject and B5 represents a measurement value of a common reference for the COL9A 1 gene, the expression ratio of the COL9A1 gene is expressed as A5/B5. In this case, the combined value of the expression ratios obtained in this step is expressed as A1/B1+A2/B2+A3/B3+A4/B4+A5/B5. When calculating the expression ratio between a subject and the common reference, it is possible to calculate an average measurement value for samples contained in the common reference in advance and calculate the expression ratio based on the average measurement value and the measurement value of a test subject. For example, in a case in which each specimen contained in the common reference consists of Ba, Bb, Bc, and Bd, if the measurement values of the COL9A2 gene are represented by Ba1, Bb1, Bc1, and Bd1, the gene expression ratio of the COL9A2 gene is expressed as 4A1/(Ba1 + Bb1 + Bc1 + Bd1). In a case in which BSCE markers include a group of selected genes for differentiating BSCE in addition to a group of essential genes for differentiating BSCE, the expression ratios of the selected genes for differentiating BSCE are also calculated and combined. The expression ratio of each gene for differentiating BSCE can also be expressed as B/A.

### (b) Average measurement value calculation step

In the average measurement value calculation step, an average measurement value for the group of genes for differentiating BSCE included in BSCE markers is calculated based on the measurement values obtained in the measurement step for each of the group of test subjects and the group of healthy subjects. In addition, an average measurement value for the group of genes for differentiating BSCE included in BSCE markers is calculated for each of healthy subjects constituting the group of healthy subjects. In a case in which, for example, BSCE markers include 7 kinds of genes for differentiating BSCE including a group of essential genes for differentiating BSCE, the average measurement value can be calculated by dividing the sum of the measurement values of the respective genes for differentiating BSCE by 7 that is the number of the kinds of genes. In the average measurement value calculation step, in principle, an average measurement value is calculated. However, if necessary, the sum of the respective measurement values of the test subject and the group of healthy subjects, which means the value obtained before dividing the sum by the number of kinds of genes for differentiating BSCE included in BSCE markers, can be calculated.

### 2-2-3. Determination step

The term "determination step" refers to a step of determining contraction of BSCE in a test subject based on the values obtained in the calculation step. In this step, determination is carried out by different methods based on the values obtained in two steps in the calculation step, which means the combined value calculation step and the average measurement value calculation step.

### (a) Determination based on the values obtained in the combined value calculation step

When the combined value calculation step is conducted in the calculation step, if the resulting combined value exceeds a predetermined cutoff value derived from an ROC curve, it is determined that the subject is highly likely to have BSCE.

A "receiver operating characteristic (ROC) curve" is created by plotting with a vertical axis representing the true position fraction (TPF), i.e., sensitivity and a horizontal axis representing the false position fraction (FPF), i.e., (1-specificity) while changing the cutoff point as a parameter, wherein the cutoff point represents the threshold value for judging the result of the test as positive. Specificity means a rate at which a negative subject is accurately determined to be negative.

It is possible to decide based on the created ROC curve which cutoff point is adopted as a cutoff value in consideration of the severity of disease, the significance of the test, and other various conditions. In general, when a cutoff point is determined to be a low point of a false positive rate, the number of healthy subjects to be positive decreases, whereas many patients having the disease are excluded, resulting in a decreased sensitivity. Conversely, when sensitivity is increased, the false positive rate for healthy subjects increases. In general, to increase both sensitivity and specificity (toward 1), the cutoff value may be set to a value giving a point closest to the point (0, 1) on the ROC curve. In a situation in which a test is performed as a screening test prior to a highly invasive test such as biopsy, the cutoff value may be set such that the sensitivity becomes high in order to reduce false-negative results.

### (b) Determination based on the values obtained in the average measurement value calculation step

When the average measurement value calculation step is performed in the calculation step, in a case in which the average measurement value of the test subject is statistically significantly larger than the obtained the average measurement value of the group of healthy subjects or the group of non-BSCE esophageal cancer patients, it is determined that the test subject is highly likely to have basaloid squamous cell carcinoma of the esophagus.

The term "statistically significant" used herein mean that when the difference between a test subject and a group of healthy subjects or a group of non-BSCE esophageal cancer patients is statistically processed, there is a significant difference therebetween. For example, a case in which the risk rate (significance level) of the resulting value is small, specifically, a case in which the risk rate is less than 5% (p<0.05), less than 1% (p<0.01), or less than 0.1% (p<0.001) is exemplified. The "p (value)" specified herein means a probability that a hypothesis is accidentally judged as correct in a statistical test, assuming a distribution of a statistic. Therefore, a smaller "p" means that a hypothesis is more likely to be true. A statistical test to be used is not particularly limited, and thus a known test by which the presence or absence of statistical significance can be determined may be used as appropriate. For example, the Student's t-test, covariate analysis of variance, etc. maybe employed.

As specified herein, in a case in which there is a statistically significant difference in a comparison between the average measurement value of a test subject and that of a group of healthy subjects or a non-BSCE esophageal cancer patients, it is determined that the test subject is affected with BSCE. For example, when the average measurement value of a test subject is 1.5 times or more, 2.0 times or more, 3.0 times or more, 4 times or more, 5 times or more, or 6 times or more than that of a group of healthy subjects or a non-BSCE esophageal cancer patients, it can be usually considered that there is a statistically significant difference.

### 2-3. Advantageous effects

According to the method for differentiating contraction of BSCE in this aspect, by examining a specimen excised by biopsy or surgery, a test subject who has provided the specimen can be provided with an accurate differential diagnosis indicating whether the test subject is affected with BSCE or another esophageal cancer. It becomes possible to provide a definitive diagnosis by the method for differentiating contraction of BSCE in this aspect with high accuracy of diagnosis, which is advantageous in that it is possible to recognize that the pathological conditions are different from those of usual kinds of esophageal squamous cell carcinoma and consider a decision on the recurrence risk or a treatment method to cope with BSCE.

In addition, reliable diagnosis of BSCE and the progress in accumulation of cases based on the diagnosis make it possible to understand further pathological conditions of BSCE as a rare disease and derive the optimal therapy. Further, it can contribute to the improvement of prognosis of BSCE that is usually accompanied by poor prognosis.

Conventionally, BSCE has been judged based on pathologic diagnosis while there has been a difference between physicians who judge diagnostic results or between hospitals. However, according to the method for differentiating contraction of BSCE in this aspect, it is possible to provide objective data to assist BSCE diagnosis.

### 3. Reagent for detecting basaloid squamous cell carcinoma of the esophagus

### 3-1. Outline

The second aspect of the present invention relates to a reagent for detecting basaloid squamous cell carcinoma of the esophagus (BSCE). The BSCE detection reagent in this aspect enables differentiating an esophageal cancer of a test subject as basaloid squamous cell carcinoma of the esophagus or another esophageal cancer by, for example, applying the reagent to a sample derived from the test subject affected with the esophageal cancer.

### 3-1-1. Constitution

The BSCE detection reagent in this aspect consists of a set of probes or primers for detecting transcription products (i.e., mRNAs or cDNAs) of genes for differentiating BSCE included in BSCE markers. A specific constitution thereof is described in the measurement step of the first aspect. For example, in a case in which transcription products of a group of 5 essential genes for differentiating BSCE included in BSCE markers are detected, the BSCE detection reagent contains a group of probes consisting of the nucleotide sequences shown in SEQ ID NOs: 141 to 145 (including complementary sequences). In this case, the BSCE detection reagent may further contain one or more probes consisting of the nucleotide sequences shown in SEQ ID NOs: 146 to 148. More preferably, the BSCE detection reagent may contain one or more probes consisting of the nucleotide sequences shown in SEQ ID NOs: 149 to 153 and still more preferably one or more probes consisting of the nucleotide sequences shown in 154 to 210.

In a case in which the BSCE detection reagent in this aspect is formed with the above-described probes, a reagent for differentiating BSCE may be provided in a state in which each probe is immobilized on a substrate to form a DNA microarray or a DNA microchip. Although material of the substrate for immobilizing each probe is not limited, a glass plate, a quartz plate, a silicon wafer, or the like is usually used. The size of the substrate is, for example, 3.5 mm×5.5 mm, 18 mm×l8 mm, or 22 mm×75 mm, which can be set variously depending on the number of spots and spot sizes for each probe. For a probe, 0.1µg to 0.5µg of nucleotides is usually used per spot. Examples of a method for immobilizing nucleotides include: a method in which nucleotides are electrostatically bound to a solid-phase support surface-treated with a polycation such as polylysine, poly-L- lysine, polyethyleneimine, or polyalkylamine with the use of charges of nucleotides; and a method in which nucleotides, into which a functional group such as an amino group, an aldehyde group, an SH group, or biotin has been introduced, are covalently bound to the surface of a solid phase, onto which a functional group such as an amino group, an aldehyde group, or an epoxy group has been introduced.

In addition, in a case in which the BSCE detection reagent described herein is an antibody or a fragment thereof which recognizes a BSCE marker protein in a specific period of time, the reagent for differentiating BSCE can be provided in a state of being immobilized on a solid-phase support. Examples of the solid-phase support that can be used include insoluble supports in the form of beads, microplates, test tubes, sticks, or test pieces made of materials such as polystyrene, polycarbonate, polyvinyl toluene, polypropylene, polyethylene, polyvinyl chloride, nylon, polymethacrylate, latex, gelatin, agarose, cellulose, sepharose, glass, metals, ceramics, or magnetic substances. Immobilization may be carried out by binding the solid-phase support with an antibody or a fragment thereof by a known method such as a physical adsorption method, a chemical binding method, or a combination method thereof.

### 3-2. Advantageous effects

It is possible to objectively and accurately determine whether or not a test subject having a past history of an esophageal cancer is or was affected with BSCE, whether such test subject is affected with BSCE or another major esophageal cancer other than BSCE, or whether or not a test subject suspected of being affected with esophageal cancer is affected with BSCE by applying the BSCE detection reagent in this aspect to a test subject having a past history of an esophageal cancer or a test subject suspected of being affected with esophageal cancer. In particular, the use of BSCE detection reagent in this aspect enables readily differentiating BSCE from esophageal squamous cell carcinoma, which has been difficult based on conventional pathological diagnosis.

### 4. Kit for detecting basaloid squamous cell carcinoma of the esophagus

### 4-1. Outline

The third aspect of the present invention relates to a kit for detecting basaloid squamous cell carcinoma of the esophagus (BSCE). The BSCE detection kit of the present invention includes, as an essential component, the BSCE detection reagent in the second aspect.

### 4-2. Constitution

The constitution of the BSCE detection reagent in the second aspect, which is included in the BSCE detection kit of the present invention contains a group of at least the probes consisting of nucleotide sequences shown in SEQ ID NOs: 141 to 145. Each of these can detect transcription products or translation products of a group of at least the 5 genes comprising nucleotide sequences shown in SEQ ID NOs: 141 to 145 for differentiating BSCE included in BSCE markers. It is preferable that each can detect one or more of 3 selected genes for differentiating BSCE comprising the nucleotide sequences shown in SEQ ID NOs: 146 to 148, more preferably one or more of 5 selected genes for differentiating BSCE comprising nucleotide sequences shown in SEQ ID NOs: 149 to 153, and further preferably one or more of 57 selected genes for differentiating BSCE comprising nucleotide sequences shown in SEQ ID NOs: 154 to 210. It is also possible that combinations of a set of probes or primers, antibodies, nucleic acid aptamers, etc. are included.

The BSCE detection kit of the present invention may include other reagents necessary for detecting a BSCE marker such as a buffer, a secondary antibody, instructions for detection and differentiation of results, in addition to the BSCE detection reagent in the second aspect. Examples

Hereinafter, the present invention will be described more specifically with reference to Examples. However, the scope of the present invention is not intended to be limited by these Examples.

### <Example 1>

### (Object)

Genes for differentiating BSCE to be included in BSCE markers are identified.

### (Method and Results)

### 1. Biological samples

### 1-1. Subjects

Subjects were 98 patients selected among esophageal cancer patients from whom the consent on this study was obtained at the Department of Regenerative Surgery, Fukushima Medical University during a period between January 2008 and July 2015, from whom it was possible to collect biological samples by surgery or endoscopic biopsy.

### 1-2. Method for collecting biological samples

(1) Regarding a surgical specimen, a specimen having a size of about 7×7 mm was collected as a sample from a lesion and from a normal site at least 5 cm apart from the lesion immediately after cancer removal, and then, placed in a freezing tube and frozen in liquid nitrogen. The remaining cancer specimen was formalin-fixed paraffin-embedded (FFPE) to prepare sections. The sections were subjected to pathological diagnosis. The number of surgical specimens was 57 samples including 7 samples diagnosed as BSCE.
(2) Regarding a biopsy specimen, a specimen having a size of about 3×3 mm was collected as a sample from a lesion and from a normal site using biopsy forceps (OLYMPUS FB-230K) during upper gastrointestinal endoscopy, and then, placed in a freezing tube and frozen in liquid nitrogen. In addition, a specimen was again collected from a lesion site, which is as close to the site for collecting specimen to be frozen as possible, and formalin-fixed. Thereafter, FFPE sections were prepared and subjected to pathological diagnosis described later.

### 1-3. Obtainment of FFPE tissue

As a sample for verification, 20 specimens of FFPE tissue marketed as "basaloid squamous cell carcinoma of the esophagus" from USbiomax, Inc. were purchased.

### 1-4. Pathological diagnosis

Pathological sections were prepared from the FFPE sections prepared in "1-2. Method for collecting biological samples" (FFPE tissue) or the FFPE tissue purchased in "1-3. Obtainment of FFPE tissue." The pathological sections were stained with hematoxylin and eosin (HE) and two pathologists independently performed a histopathological diagnosis. Pathological diagnosis was given in accordance with pathological standards presented by Wain et al., and six patterns of the pathological conditions presented by Imamhasan et al. (Imamhasan A., et al., 2012, Human Pathology 43:2012-2023) were used for reference. The six patterns are the following A to F.
(A) Solid nest with comedo-type necrosis
(B) Cribriform pattern and pseudoacini formation
(C) Ductal differentiation
(D) Small nests with a microcystic and/or trabecular pattern
(E) Hyaline-like material deposition
(F) Coexistence of invasive SCC component

Each case was diagnosed in a comprehensive manner in consideration of the incidence rates of observations of A to F above as well. In this Example, a specimen partially containing components of basaloid squamous cell carcinoma of the esophagus was determined as basaloid squamous cell carcinoma of the esophagus even when the specimen is mainly composed of components of squamous cell carcinoma.

Pathological images were taken using an HS all-in-one fluorescence microscope BZ-9000 (KEYENCE, Osaka, Japan). Image processing was carried out using Illustrator (Adobe System Inc., CA, USA).

### 1-5. Classification of specimens

Maker genes for differentiating BSCE from other esophageal cancers were extracted from the surgical specimens described above. Specifically, the extraction was conducted as follows.

Of 98 patients, there were 18 patients from whom only surgical specimens were collected, 68 patients from whom only biopsy specimens were collected, and 12 patients from whom both surgical specimens and biopsy specimens were collected. As a result of pathological and histopathological examination, only 7 out of 98 patients were diagnosed as BSCE. Gene expression analysis was conducted by collecting a plurality of specimens (surgical specimens and biopsy specimens) from each individual. There were 369 specimens in total from which sufficient amounts of RNAs were obtained and for which comprehensive gene expression analysis could be conducted. The 369 specimens consisted of 57 surgical specimens and 312 biopsy specimens. The surgical specimens consisted of 26 specimens of normal esophageal epithelium, 23 specimens of esophageal squamous cell carcinoma, 7 specimens of basaloid squamous cell carcinoma of the esophagus (BSCE), and 1 specimen of endocrine cell carcinoma of the esophagus. The biopsy specimens consisted of 229 specimens of normal esophageal epithelium, 51 specimens of squamous cell carcinoma, 8 specimens of BSCE, 1 specimen of endocrine cell carcinoma, 21 specimens of adenocarcinoma, and 2 specimens of intraepithelial tumor.

Table 2 describes clinicopathological characteristics of BSCE patients who underwent surgery. The patients were 5 male (M) cases and 1 female (F) case, whose average age was 62.6 years old (55 to 68 years old). There were 3 cases of patients (3/6, 50%) who had been diagnosed as BSCE by preoperative biopsy. The average tumor diameter was 28.6 mm (12 mm to 45 mm), and the site where the tumor was located was middle thoracic esophagus in all cases. According to the TNM Stage classification in the 7th edition of UICC, there were 4 cases of stage I, 1 case of stage II, and 1 case of stage III. One of the cases of stage I died from the primary disease in 58 months after surgery. The one case of stage III died from the primary disease in 19 months after surgery. The one case of stage II has been surviving with postoperative recurrence. The three cases of stage I have been surviving without recurrence (observation period of 7 to 60 months).

**[Table 2]**

| No. | Age | sex | Biopsy diagnosis | Pathological diagnosis | Postoperative treatment | Prognosis | Size of Tumor (mm) | Tumor type | Depth of invasion (pT) | Lymph node metastasis(pN) | Lymphatic invasion(ly) | Venous invasion(v) | UICC Stage |
|---|---|---|---|---|---|---|---|---|---|---|---|---|---|
| 1 | 68 | M | ASC | BSC | CRT | 58M-Death (Lung meta) | 42 | type2 | pT1 | pN0 | 0 | 1 | IA |
| 2 | 55 | M | SCC | BSC | None | 60M-Alive | 30 | type5 | pT1 | pN0 | 0 | 2 | IA |
| 3 | 56 | M | SCC | BSC, SCC | CRT | 19M-Death (Lung meta) | 25 | type3 | pT4 | pN3 | 2 | 2 | IIIC |
| 4 | 68 | F | SCC(1st) | BSC | None | 37M-Alive | 18 | type2 | pT1 | pN0 | 0 | 1 | IA |
| | | | BSC(2nd) | | | | | | | | | | |
| 5 | 64 | M | BSC | BSC, SCC | CRT | 29M-Alive (LN meta) | 12 | type0-IIa | pT1 | pN1 | 0 | 0 | IIB |
| 6 | 65 | M | BSC | BSC, SCC | None | 7M-Alive | 45 | type0-IIc | pT1 | pN0 | 0 | 0 | IA |

| | | | | | | | | | | | | | |
|---|---|---|---|---|---|---|---|---|---|---|---|---|---|
| ASC:Adeno squamous carcinoma SCC:Squamous cell carcinoma BSC:Basaloid squamous cell carcinoma CT:Chemotherapy CRT:Chemoradiotherapy | | | | | | | | | | | | | |

### 2. Preparation of DNA microarray

A DNA microarray was prepared using synthetic DNA for microarrays. A DNA microarray for poly(A)+ RNA (mRNA) (hereinafter referred to as "System 1 ") was prepared by forming an array of 31797 kinds of synthetic DNA (80 mers) corresponding to human-derived transcription products on a microscope slide. In addition, a DNA microarray for total RNA (hereinafter referred to as "System 2") was prepared by forming an array of 14400 kinds of synthetic DNA (80 mers) corresponding to human-derived transcription products on a microscope slide. As a method for preparing a DNA microarray, the preparation method described in Schena M., et al., 1995, Science, 270:467-470 was referred to. Specifically, a DNA microarray was prepared by printing a human gene fragment library (manufactured by Micro Diagnostic Laboratories) on a microscope slide (manufactured by Matsunami Glass Ind., Ltd., HA-coated microscope slide) using a ultratrace dispenser (manufactured by Micro Diagnostic Laboratories) in accordance with the protocol recommended by the manufacturer. This DNA microarray was allowed to stand for 1 hour at 80°C in a gas phase incubator and further irradiated with 120-mJ ultraviolet light using a UV cross linker (Hoefer Inc., UVC500). Aftertreatment of the DNA microarray was conducted in accordance with the method described in Japanese Patent No. 4190899.

### 3. Preparation of labeled cDNA

### 3-1. Total RNA extraction from frozen tissue

Total RNA was extracted from the samples, which had been collected in "1-2. Method for collecting biological samples" and frozen in liquid nitrogen, using ISOGEN (Nippon Gene Co., Ltd.) in accordance with the attached protocol.

### 3-2. Total RNA extraction from FFPE

Total RNA was extracted from the FFPE tissue purchased in "1-3. Obtainment of FFPE tissue" using an ISOGEN PB Kit (Nippon Gene Co., Ltd.) in accordance with the attached protocol.

### 3-3. Purification of poly(A)+ RNA

Regarding the samples for which 125 µg or more of total RNA was successfully obtained, poly(A)+ RNA was subsequently purified therefrom using a MicroPoly(A) purist kit (Ambion, Inc.) in accordance with the attached protocol.

### 3-4. Preparation of labeled cDNA

A specimen-labeled cDNA was prepared by a reverse transcription reaction using the prepared poly(A)+ RNA or total RNA as a template. Specifically, 2.0 µg of the poly(A)+ RNA or 5.0 µg of the total RNA, SuperScript II (registered trademark) reverse transcriptase (manufactured by Thermo Fisher Scientific Inc.), and Cyanine 5-deoxyuridinetriphosphate (Cyanine 5-dUTP) (manufactured by Perkin Elmer Co., Ltd.) were used for the preparation using a nucleic acid labeling/hybridization reagent (manufactured by Micro Diagnostic Laboratories)

As a control poly(A)+ RNA or total RNA, Human Common Reference (manufactured by Micro Diagnostic Laboratories) was used. Human Common Reference is a mixture of equal amounts of poly(A)+ RNA or total RNA prepared from each of 22 kinds of human-derived cell lines (A431 cells, A549 cells, AKI cells, HBL-100 cells, HeLa cells, HepG2 cells, HL60 cells, IMR-32 cells, Jurkat cells, K562 cells, KP4 cells, MKN7 cells, NK-92 cells, Raji cells, RD cells, Saos-2 cells, SK-N-MC cells, SW-13 cells, T24 cells, U251 cells, U937 cells, and Y79 cells).

Control labeled cDNA, for which the Human Common Reference was used as a template, was prepared as in the specimen-labeled cDNA except that Cyanine 3-deoxyuridinetriphosphate (Cyanine 3-dUTP) (manufactured by Perkin Elmer Corp.) was used as a dye-labeled deoxynucleotide. A specific method for preparing the labeled cDNA was conducted in accordance with the protocol recommended by each manufacturer.

After the specimen-labeled cDNA and the control labeled cDNA were combined in the same test tube to obtain mixture labeled cDNA, the cDNA was purified using Micropure EZ (manufactured by Merck Millipore) and Microcon YM30 (registered trademark) (manufactured by Merck Millipore). A specific purification method was conducted in accordance with the protocol recommended by the company.

The purified mixture labeled cDNA was adjusted to 15 µL or 7 µL using a hybridization buffer provided with a nucleic acid labeling/hybridization reagent (manufactured by Micro Diagnostic Laboratories) and pure water to obtain a labeled cDNA solution.

### 4. Hybridization

The labeled cDNA solution was heated at 99°C for 5 minutes for heat denaturation to prepare a labeled probe. The labeled probe was added dropwise to the DNA microarray prepared in "1. Preparation of DNA microarray" and the microarray was placed in a hybridization cassette (manufactured by Micro Diagnostic Laboratories). The hybridization cassette was placed in a gas phase incubator (manufactured by Sanyo Biomedical Inc.) and incubated in a stationary state at 42°C for 20 hours.

The DNA microarray was removed from the hybridization cassette and washed with a hybridization wash solution provided with the nucleic acid labeling/hybridization reagent (manufactured by Micro Diagnostic Laboratories) in accordance with the protocol recommended by the company.

### 5. Selection of markers for differentiating basaloid squamous cell carcinoma of the esophagus

### 5-1. Measurement of the gene expression level

The expression level of each gene was determined based on fluorescence intensity of the labeled probe bound to oligo DNA immobilized to the microarray in "4. Hybridization." Fluorescence on the DNA microarray after washing was measured using a Scanner GenePix 4000B (manufactured by Axon Instruments Inc.). Thereafter, the fluorescence was optically evaluated using the analysis software GenePiacPro (manufactured by Axon Instruments Inc.) supplied with the scanner, thereby quantifying the relative value of fluorescence intensity. Specifically, fluorescence intensity (Cyanine 5 fluorescence intensity) from specimen-labeled cDNA and fluorescence intensity (Cyanine 3 fluorescence intensity) from control labeled cDNA were measured at each oligo DNA spot fixed on the DNA microarray and the Cyanine 5/Cyanine 3 fluorescence intensity ratio (logarithmic transformed relative expression ratio: log₂ ratio) at each spot was calculated, thereby correcting (scaling) the level of gene expression between spots. In addition, the background was calculated from the fluorescence intensities at sites other than the spots on the DNA microarray, and it was subtracted as the noise from the fluorescence intensity ratio of each spot.

### 5-2. Extraction of the group of genes as markers for differentiating BSCE from other esophageal cancers

Gene expression profiles were obtained from 57 surgical specimens including 7 specimens pathologically diagnosed as BSCE using a DNA microarray. From the gene expression profiles, 14400 genes were extracted which were present on both the System 1 DNA microarray and the System 2 DNA microarray in common. Next, genes for which fluorescent intensities were below the detection limit in 2 or more specimens among the 7 specimens pathologically diagnosed as basaloid squamous cell carcinoma of the esophagus were excluded. Subsequently, a group of genes meeting a requirement that the logarithmic transformed relative expression ratio was not less than 1 or not more than -1 in at least 1 out of 57 specimens was extracted. Further, the average value of logarithmic transformed relative expression ratios of all specimens was calculated for each gene, and a group of genes each having a difference between the average value and its ratio of not less than 1 or not more than - 1 for at least one specimen was extracted (7379 genes). Based on the values of the logarithmic transformed relative expression ratio for the group of genes, cluster analysis was conducted in the two-dimensional direction.

"Cluster analysis" is a statistical technique for grouping genes or samples having similar gene expression patterns. It is possible to group samples having similar gene expression patterns by defining similarity between data (e.g., Euclidean distance) and using the degree of similarity. Hierarchical cluster analysis can also be carried out using analysis software products such as "Expression View Pro" (Micro Diagnostic Laboratories), "GeneMaths XT" (Infocom, Inc.), "GeneSpring" (Agilent Technologies, Inc.), "treeview" (Stanford University), and MicroArray Data Analysis tool (Filgen, Inc.). It is possible to determine the presence or absence of BSCE contraction by constructing in advance a discriminant analysis model for differentiating BSCE from other esophageal cancers and inputting data on the gene expression profiles obtained from test subjects into the discriminant analysis model. For example, cluster analysis is conducted with the addition of gene expression data from a new test subject currently affected with an esophageal cancer to the discriminant analysis model consisting of two clusters of those having a history of an esophageal cancer, which have been found to be a BSCE group and a group of other esophageal cancers, so as to determine whether the new test subject is classified into the group of BSCE or the group of other esophageal cancers. Moreover, it is also possible to determine the presence or absence of BSCE contraction by, for example, obtaining a discriminant through discriminant analysis, associating fluorescence intensity with contraction of BSCE, and inputting a pattern of digitalized expression signals of a test subject into the discriminant.

Figure 1 shows the results. As a result of cluster analysis, 57 specimens were classified into a cluster formed with 6 out of 7 BSCE specimens (hereinafter referred to as a "BSCE cluster"), a cluster mainly formed with squamous cell carcinoma specimens (hereinafter referred to as a "squamous cell carcinoma cluster"), and a cluster mainly including normal specimens (hereinafter referred to as a "normal specimen cluster"). Note that since there was only 1 specimen of endocrine cell carcinoma, it could not be said that it was classified into a cluster.

The average measurement value of logarithmic transformed relative expression ratios of 6 specimens of basaloid squamous cell carcinoma of the esophagus (hereinafter referred to as a "BSCE specimen group") included in the BSCE cluster was calculated, and a gene group, for which the value was found to be 1 or more, was extracted. Next, among 50 specimens remaining after excluding 1 specimen of BSCE that was not included in the BSCE cluster from the remaining specimens (hereinafter referred to as a "non-BSCE specimen group"), a gene group, for which fluorescence intensity was not more than the detection limit for at least 26 specimens, was excluded. The standard deviation of the logarithmic transformed relative expression ratio for the non-BSCE specimen group was calculated, and a gene group, for which the value was found to be less than 0.5, was extracted. Next, a gene group, for which the average value of the logarithmic transformed relative expression ratio for the BSCE specimen group was larger by 1 or more than the average value of the logarithmic transformed relative expression ratio for the non-BSCE specimen group, was extracted. Further, two-group comparison between the BSCE specimen group and the non-BSCE specimen group was conducted by t-test, and a gene group, for which the P value was found to be less than 0.01, was extracted. As a result, the obtained 70 kinds of genes described in Table 1 were identified as a "group of genes for differentiating BSCE" included in the BSCE markers of the present invention.

An ROC curve was created to verify whether it would be possible for a BSCE marker to differentiate BSCE using a group of all 70 kinds of genes for differentiating BSCE as BSCE markers. Figure 2 (A) shows the results. The area of the curve, i.e., area under the curve (hereinafter referred to as "AUC") was 1.0000 for surgical specimens. In addition, a sensitivity-specificity curve was created using BSCE markers (70 genes) for verification. When the cutoff value was set to 17.2912 corresponding to a point at the intersection of sensitivity with specificity for surgical specimens, sensitivity (= specificity) was 1.0000 as shown in Figure 2 (B). These results suggest that BSCE can be differentiated with high accuracy using the BSCE markers of the present invention.

Next, the sum of the logarithmic transformed relative expression ratios of BSCE markers (a group of 70 genes) in each specimen (hereinafter referred to as "expression scores of 70 genes") were calculated, and 70 kinds of genes for differentiating BSCE were re-aligned in the ascending order of the expression scores of 70 genes. Figure 2 (C) shows the results. The results also verified that BSCE can be differentiated with high accuracy by setting the cutoff value represented by a border line in the figure using BSCE markers.

### <Example 2>

### (Object)

Efficacy for differentiating BSCE with BSCE markers is verified for biopsy specimens.

### (Method and Results)

An ROC curve was created by combining the expression scores of 70 genes for specimens (57 specimens) and the expression scores of 70 genes for biopsy specimens for verification. Regarding the expression scores of 70 genes for biopsy specimens, gene expression profiles were obtained from 312 biopsy specimens collected in Example 1 using a DNA microarray, the profiles were converted into logarithmic transformed relative expression ratios, and then, data on BSCE markers (a group of 70 genes) were extracted, thereby calculating the expression scores of 70 genes for the group of all 70 kinds of genes for differentiating BSCE. As a result, AUC was 0.9927 as shown in Figure 2 (D). In addition, a sensitivity-specificity curve was created by combining surgical specimens (57 specimens) and biopsy specimens (312 specimens) using BSCE markers (a group of 70 genes) for verification. As a result, as shown in Figure 2 (E), when the cutoff value was set to 33.1368, sensitivity was 0.9333 and specificity was 0.9915. These results revealed that BSCE in biopsy specimens can be differentiated with high accuracy using the BSCE markers of the present invention.

Further, biopsy specimens (312 specimens) and surgical specimens (57 specimens) were combined, and they were re-aligned in the ascending order of expression scores of 70 genes. As a result, as shown in Figure 2 (F), BSCE can be differentiated with high accuracy by setting the cutoff value represented by a border line in the figure using BSCE markers even when biopsy specimens and surgical specimens are combined.

Note that BSCE could not be differentiated at 100% sensitivity and specificity when biopsy specimens were used for verification. However, in the case of using biopsy specimens, specimens subjected to histopathological tests and specimens subjected to gene expression analysis were not obtained by dividing a tissue at the same site into two portions, meaning that their sites were merely adjacent to each other and thus they were different. BSCE is often covered by a portion of squamous cell carcinoma or coexists with the same lesion of squamous cell carcinoma. Therefore, even a biopsy specimen from a patient diagnosed as BSCE by histopathological tests might not contain components of BSCE, or vice versa. However, it does not mean that the method for differentiating contraction of BSCE of the present invention cannot be applied to biopsy specimens. In fact, in patients whose surgical specimens were pathologically diagnosed as having a tumor mainly composed of squamous cell carcinoma but also having BSCE at about 10%, among preoperative biopsy specimens (3 specimens), 2 specimens were diagnosed as BSCE and the remaining 1 specimen was diagnosed as squamous cell carcinoma. Further, portions in the vicinity of such specimens were verified using the expression scores of 70 genes. It was determined that one of the former specimens and the one latter specimen were found to be BSCE.

### <Example 3>

### (Object)

Efficacy for differentiating BSCE with BSCE markers was verified for FFPE tissues.

### (Method and Results)

As FFPE tissue specimens for testing BSCE markers, the above-described 20 FFPE tissue specimens purchased from USbiomax, Inc. were used. As a result of reexamining all FFPE tissues for pathological diagnosis, it was found that 13 specimens had basaloid squamous cell carcinoma of the esophagus, 2 specimens had squamous cell carcinoma, and 5 specimens had endocrine cell carcinoma.

Gene expression profiles were obtained from these FFPE tissue specimens using a DNA microarray, the profiles were converted into logarithmic transformed relative expression ratios, and then, data on BSCE markers (a group of 70 genes) were extracted, thereby calculating the expression scores of 70 genes. An ROC curve was created by combining surgical specimens (57 specimens) and FFPE tissue specimens (20 specimens) to verify whether it would be possible to differentiate BSCE in an FFPE tissue specimen using BSCE markers (a group of 70 genes). As a result, AUC was 0.9930 as shown in Figure 2 (G). In addition, a sensitivity-specificity curve was created by combining surgical specimens (57 specimens) and FFPE tissue specimens (20 specimens) using BSCE markers (a group of 70 genes) for verification. As a result, as shown in Figure 2 (H), when the cutoff value was set to 34.7723, sensitivity was 0.9000 and specificity was 0.9825. These results revealed that BSCE in FFPE tissue specimens can be differentiated with high accuracy using the BSCE markers of the present invention.

Further, FFPE tissue specimens (20 specimens) and surgical specimens (57 specimens) were combined, and they were re-aligned in the ascending order of expression scores of 70 genes. As a result, as shown in Figure 2 (I), BSCE can be differentiated with high accuracy by setting the cutoff value represented by a border line in the figure using BSCE markers even when FFPE tissue specimens and surgical specimens are combined.

### <Example 4>

### (Object)

Efficacy of differentiating BSCE based on group scatter diagrams using surgical specimens, biopsy specimens, and FFPE specimens is verified.

### (Method and Results)

A group scatter diagram was created based the expression scores of 70 genes as BSCE markers (a group of 70 genes) by combining all of surgical specimens, biopsy specimens, and FFPE tissue specimens. As a result, as shown in Figure 2 (J), the average value of the non-BSCE specimen group was 8.970, and the average value of the BSCE specimen group was 70.549. There was a statistically significant difference (P=1.43×10⁻⁹) between both groups.

Next, a group scatter diagram was created separately for each specimen type. Figure 2 (K) shows the results. For surgical specimens, the average value of the non-BSCE specimen group was 6.808, and the average value of the BSCE specimen group was 108.715. There was a statistically significant difference (P=0.0004) between both groups. For biopsy specimens, the average value of the non-BSCE specimen group was 8.878, and the average value of the BSCE specimen group was 72.764. There was a statistically significant difference (P=0.0012) between both groups. For FFPE tissue specimens, the average value of the non-BSCE specimen group was 28.389, and the average value of the BSCE specimen group was 48.635. In addition, there was a statistically significant difference (P=0.0003) between both groups.

The above results verified that BSCE can be differentiated with high accuracy by determining the expression levels of BSCE markers.

### <Example 5>

### (Object)

A group of essential genes for differentiating BSCE is selected from BSCE markers.

### (Method and Results)

Priority ranking was made using the 70 kinds of genes for differentiating BSCE identified in Example 1 in consideration of the difference in the average value between the BSCE specimen group and the non-BSCE specimen group, standard deviation, P value of the t-test, score values for the BSCE specimen group and clusters formed by cluster analysis. As a result, it was revealed that the group of 5 kinds of genes for differentiating BSCE represented by NOs: R-1 to R-5 in Table 1 can differentiate the BSCE specimen group and the non-BSCE specimen group.

After gene expression profiles were obtained using a DNA microarray, the profiles were converted into logarithmic transformed relative expression ratios, gene expression data of 5 kinds of genes for differentiating BSCE constituting the above-described group of essential genes for differentiating BSCE were extracted to calculate the sum (hereinafter referred to as "expression scores of 5 genes"), and an ROC curve was created using the sum, i.e., the values of the expression scores of 5 genes. As a result, as shown in Figure 3 (A), AUC was 1.0000 for surgical specimens alone. In addition, as shown in Figure 3 (C), AUC was 0.9533 for surgical specimens and biopsy specimens, and as shown in Figure 3 (E), AUC was 0.9737 for surgical specimens and FFPE tissue specimens.

Next, a sensitivity-specificity curve was created for verification. As a result, as shown in Figure 3 (B), both sensitivity and specificity were 1.0000 for surgical specimens alone when the cutoff value was set to 2.7064. In addition, as shown in Figure 3 (D), when the cutoff value was set to 4.1997, sensitivity was 0.9333 and specificity was 0.9718 for surgical specimens and biopsy specimens. Further, as shown in Figure 3 (F), when the cutoff value was set to 6.3559, sensitivity was 0.9500 and specificity was 0.9123 for surgical specimens and FFPE tissue specimens.

Subsequently, surgical specimens alone (Figure 3 (G)), surgical specimens and biopsy specimens (Figure 3 (H)), and surgical specimens and FFPE tissue specimens (Figure 3 (I)) were re-aligned in the ascending order of the expression scores of 5 genes using the expression scores of 5 genes for the above-described 5 kinds of genes for differentiating BSCE. As a result, it was revealed that the BSCE specimen group and the non-BSCE specimen group can be differentiated by setting a certain cutoff value regardless of specimens.

Further, a group scatter diagram was created from all analyzed specimens using the expression scores of 5 genes for the above-described 5 kinds of genes for differentiating BSCE. Figure 3 (J) shows the results. The average value of the non-BSCE specimen group was 1.052, and the average value of the BSCE specimen group was 10.496. There was a statistically significant difference (P=1.61×10⁻¹¹) between both groups.

Subsequently, a group scatter diagram was created separately for each specimen type. Figure 3 (K) shows the results. For surgical specimens, the average value of the non-BSCE specimen group was -0.967, and the average value of the BSCE specimen group was 13.200. There was a statistically significant difference (P=0.0003) between both groups. For biopsy specimens, the average value of the non-BSCE specimen group was 1.240, and the average value of the BSCE specimen group was 9.801. There was a statistically significant difference (P=0.0048) between both groups. For FFPE tissue specimens, the average value of the non-BSCE specimen group was 7.320, and the average value of the BSCE specimen group was 9.468. There was a statistically significant difference (P=0.0265) between both groups.

The above results indicated that even BSCE markers consisting of the group of 5 kinds of genes for differentiating BSCE shown in NOs: R-1 to R-5 in Table 1 can also differentiate the BSCE specimen group and the non-BSCE specimen group. Thus, the group of 5 genes for differentiating BSCE was designated as a group of essential genes for differentiating BSCE.

### <Example 6>

### (Object)

A group of genes for differentiating BSCE is narrowed down from BSCE markers. [1]

### (Method and Results)

Priority ranking was made using the 70 kinds of genes for differentiating BSCE identified in Example 1 in consideration of the difference in the average value between the BSCE specimen group and the non-BSCE specimen group, standard deviation, P value of t-test, score values for the BSCE specimen group and clusters created by cluster analysis. As a result, it was revealed that even a group of 8 kinds of genes for differentiating BSCE, including 3 kinds of selected genes for differentiating BSCE represented by NOs: R-6 to R-8 in Table 1 in addition to the group of essential genes for differentiating BSCE, can differentiate the BSCE specimen group and the non-BSCE specimen group.

The basic procedures were conducted as in Example 5. An ROC curve was created from the sum obtained by extracting gene expression data of 8 kinds of genes for differentiating BSCE to calculate the sum (hereinafter referred to as "expression scores of 8 genes"), i.e., expression scores of 8 genes. As a result, as shown in Figure 4 (A), AUC was 1.0000 for surgical specimens alone. In addition, as shown in Figure 4 (C), AUC was 0.9364 for surgical specimens and biopsy specimens, and as shown in Figure 4 (E), AUC was 0.9693 for surgical specimens and FFPE tissue specimens.

Next, a sensitivity-specificity curve was created for verification. As a result, as shown in Figure 4 (B), both sensitivity and specificity were 1.0000 for surgical specimens alone when the cutoff value was set to 2.3660. In addition, as shown in Figure 4 (D), when the cutoff value was set to 6.0307, sensitivity was 0.9333 and specificity was 0.9746 for surgical specimens and biopsy specimens. Further, as shown in Figure 4 (F), when the cutoff value was set to 9.6492, sensitivity was 0.9000 and specificity was 0.9123 for surgical specimens and FFPE tissue specimens.

Subsequently, surgical specimens alone (Figure 4 (G)), surgical specimens and biopsy specimens (Figure 4 (H)), and surgical specimens and FFPE tissue specimens (Figure 4 (I)) were re-aligned in the ascending order of the expression scores of 8 genes using the expression scores of 8 genes for the above-described 8 kinds of genes for differentiating BSCE. As a result, it was revealed that the BSCE specimen group and the non-BSCE specimen group can be differentiated by setting a certain cutoff value regardless of specimens.

Further, a group scatter diagram was created from all analyzed specimens using the expression scores of 8 genes for the above-described 8 kinds of genes for differentiating BSCE. As a result, as shown in Figure 4 (J), the average value of the non-BSCE specimen group was 2.050, and the average value of the BSCE specimen group was 14.273. There was a statistically significant difference (P=1.23×10⁻¹⁰) between both groups.

Next, Figure 4 (K) shows the results of creating a group scatter diagram separately for each specimen type. For surgical specimens, the average value of the non-BSCE specimen group was -0.623, and the average value of the BSCE specimen group was 18.805. There was a statistically significant difference (P=0.0008) between both groups. For biopsy specimens, the average value of the non-BSCE specimen group was 2.294, and the average value of the BSCE specimen group was 12.767. There was a statistically significant difference (P=0.0049) between both groups. For FFPE tissue specimens, the average value of the non-BSCE specimen group was 10.538, and the average value of the BSCE specimen group was 12.758. There was a statistically significant difference (P=0.0690) between both groups.

The above results indicated that BSCE markers consisting of the group of 8 kinds of genes for differentiating BSCE shown in NOs: R-1 to R-8 in Table 1 can also differentiate the BSCE specimen group and the non-BSCE specimen group.

### <Example 7>

### (Object)

A group of genes for differentiating BSCE is narrowed down from BSCE markers. [2]

### (Method and Results)

Priority ranking was made using the 70 kinds of genes for differentiating BSCE identified in Example 1 in consideration of the difference in the average value between the BSCE specimen group and the non-BSCE specimen group, standard deviation, P value of t-test, score values for the BSCE specimen group and clusters created by cluster analysis. As a result, it was revealed that even a group of 10 kinds of genes for differentiating BSCE, including 5 kinds of selected genes for differentiating BSCE represented by NOs: R-9 to R-13 in Table 1 in addition to the group of essential genes for differentiating BSCE, can differentiate the BSCE specimen group and the non-BSCE specimen group.

The basic procedures were conducted as in Example 5. An ROC curve was created from the sum obtained by extracting gene expression data of the 10 kinds of genes for differentiating BSCE to calculate the sum (hereinafter referred to as "expression scores of 10 genes"), i.e., expression scores of 10 genes. As a result, as shown in Figure 5 (A), AUC was 1.0000 for surgical specimens alone. In addition, as shown in Figure 5 (C), AUC was 0.9714 for surgical specimens and biopsy specimens, and as shown in Figure 5 (E), AUC was 0.9930 for surgical specimens and FFPE tissue specimens.

Next, a sensitivity-specificity curve was created for verification. As a result, as shown in Figure 5 (B), both sensitivity and specificity were 1.0000 for surgical specimens alone when the cutoff value was set to 2.2155. In addition, as shown in Figure 5 (D), when the cutoff value was set to 6.6384, sensitivity was 0.9333 and specificity was 0.9915 for surgical specimens and biopsy specimens. Further, as shown in Figure 5 (F), when the cutoff value was set to 6.5719, sensitivity was 0.9500 and specificity was 0.9825 for surgical specimens and FFPE tissue specimens.

Subsequently, surgical specimens alone (Figure 5 (G)), surgical specimens and biopsy specimens (Figure 5 (H)), and surgical specimens and FFPE tissue specimens (Figure 5 (I)) were re-aligned in the ascending order of the expression scores of 10 genes using the expression scores of 10 genes for the above-described 10 kinds of genes for differentiating BSCE. As a result, it was revealed that the BSCE specimen group and the non-BSCE specimen group can be differentiated by setting a certain cutoff value regardless of specimens.

Further, a group scatter diagram was created from all analyzed specimens using the expression scores of 10 genes for the above-described 10 kinds of genes for differentiating BSCE. As a result, as shown in Figure 5 (J), the average value of the non-BSCE specimen group was 0.311, and the average value of the BSCE group was 13.584. There was a statistically significant difference (P=1.63×10⁻¹⁰) between both groups.

Next, Figure 5 (K) shows the results of creating a group scatter diagram separately for each specimen type. For surgical specimens, the average value of the non-BSCE specimen group was -1.814, and the average value of the BSCE specimen group was 19.965. There was a statistically significant difference (P=9.87×10⁻⁵) between both groups. For biopsy specimens, the average value of the non-BSCE specimen group was 0.551, and the average value of the BSCE specimen group was 14.208. There was a statistically significant difference (P=2.52×10⁻³) between both groups. For FFPE tissue specimens, the average value of the non-BSCE group was 5.063, and the average value of the BSCE specimen group was 9.764. There was a statistically significant difference (P=9.27×10⁻⁴) between both groups.

The above results indicated that BSCE markers consisting of the group of 10 kinds of genes for differentiating BSCE shown in NOs: R-1 to R-5 and NOs: R-9 to R-13 in Table 1 can also differentiate the BSCE specimen group and the non-BSCE specimen group.

### <Example 8>

### (Object)

A group of genes for differentiating BSCE is narrowed down from BSCE markers. [3]

### (Method and Results)

It was revealed that the group of 13 kinds of genes for differentiating BSCE represented by NOs: R-1 to R-13 in Table 1 verified in Examples 5 to 7, including the group of essential genes for differentiating BSCE, also can differentiate the BSCE specimen group and the non-BSCE specimen group.

The basic procedures were conducted as in Example 5. An ROC curve was created from the sum obtained by extracting gene expression data of the 13 kinds of genes for differentiating BSCE to calculate the sum (hereinafter referred to as "expression scores of 13 genes"), i.e., expression scores of 13 genes. As a result, as shown in Figure 6 (A), AUC was 1.0000 for surgical specimens alone. In addition, as shown in Figure 6 (C), AUC was 0.9482 for surgical specimens and biopsy specimens, and as shown in Figure 6 (E), AUC was 0.9877 for surgical specimens and FFPE tissue specimens.

As shown in Figure 6 (B), also for the sensitivity-specificity curve, both sensitivity and specificity were 1.0000 for surgical specimens alone when the cutoff value was set to 2.7974. In addition, as shown in Figure 6 (D), when the cutoff value was set to 8.5372, sensitivity was 0.9333 and specificity was 0.9915 for surgical specimens and biopsy specimens. Further, as shown in Figure 6 (F), when the cutoff value was set to 9.3096, sensitivity was 0.9500 and specificity was 0.9649 for surgical specimens and FFPE tissue specimens.

Subsequently, surgical specimens alone (Figure 6 (G)), surgical specimens and biopsy specimens (Figure 6 (H)), and surgical specimens and FFPE tissue specimens (Figure 6 (I)) were re-aligned in the ascending order of the expression scores of 13 genes using the expression scores of 13 genes for the above-described 13 kinds of genes for differentiating BSCE. As a result, it was revealed that the BSCE specimen group and the non-BSCE specimen group can be differentiated by setting a certain cutoff value regardless of specimens.

Further, a group scatter diagram was created from all analyzed specimens using the expression scores of 13 genes for the above-described 13 kinds of genes for differentiating BSCE. As a result, as shown in Figure 6 (J), the average value of the non-BSCE specimen group was 1.309, and the average value of the BSCE group was 17.360. There was a statistically significant difference (P=3.94×10⁻¹⁰) between both groups.

Next, Figure 6 (K) shows the results of creating a group scatter diagram separately for each specimen type. For surgical specimens, the average value of the non-BSCE specimen group was -1.471, and the average value of the BSCE specimen group was 25.570. There was a statistically significant difference (P=0.0003) between both groups. For biopsy specimens, the average value of the non-BSCE specimen group was 1.605, and the average value of the BSCE specimen group was 17.174. There was a statistically significant difference (P=0.0029) between both groups. For FFPE tissue specimens, the average value of the non-BSCE specimen group was 8.281, and the average value of the BSCE specimen group was 13.055. There was a statistically significant difference (P=0.0035) between both groups.

The above results indicated that BSCE markers consisting of the group of 13 kinds of genes for differentiating BSCE shown in NOs: R-1 to R-13 in Table 1 can also differentiate the BSCE specimen group and the non-BSCE specimen group.

### <Example 9>

### (Object)

A group of genes for differentiating BSCE is narrowed down from BSCE markers. [4]

### (Method and Results)

Priority ranking was made using the 70 kinds of genes for differentiating BSCE identified in Example 1 in consideration of the difference in the average value between the BSCE specimen group and the non-BSCE specimen group, standard deviation, P value of t-test, score values for the BSCE specimen group and clusters created by cluster analysis. As a result, it was revealed that even a group of 7 kinds of genes for differentiating BSCE, including 2 kinds of selected genes for differentiating BSCE represented by NOs: R-14 and R-15 in Table 1 in addition to the group of essential genes for differentiating BSCE, can differentiate the BSCE specimen group and the non-BSCE specimen group.

The basic procedures were conducted as in Example 5. An ROC curve was created from the sum obtained by extracting gene expression data of the 7 kinds of genes for differentiating BSCE to calculate the sum (hereinafter referred to as "expression scores of 7 genes"), i.e., values of expression scores of 7 genes. As a result, as shown in Figure 7 (A), AUC was 1.0000 for surgical specimens alone. In addition, as shown in Figure 7 (C), AUC was 0.9648 for surgical specimens and biopsy specimens, and as shown in Figure 7 (E), AUC was 0.9904 for surgical specimens and FFPE tissue specimens.

Next, a sensitivity-specificity curve was created for verification. As a result, as shown in Figure 7 (B), both sensitivity and specificity were 1.0000 for surgical specimens alone when the cutoff value was set to 1.1 138. In addition, as shown in Figure 7 (D), when the cutoff value was set to 4.2419, sensitivity was 0.9333 and specificity was 0.9746 for surgical specimens and biopsy specimens. Further, as shown in Figure 7 (F), when the cutoff value was set to 8.2572, sensitivity was 0.9000 and specificity was 1.0000 for surgical specimens and FFPE tissue specimens.

Subsequently, surgical specimens alone (Figure 7 (G)), surgical specimens and biopsy specimens (Figure 7 (H)), and surgical specimens and FFPE tissue specimens (Figure 7 (I)) were re-aligned in the ascending order of the expression scores of 7 genes using the expression scores of 7 genes for the above-described 7 kinds of genes for differentiating BSCE. As a result, it was revealed that the BSCE specimen group and the non-BSCE specimen group can be differentiated by setting a certain cutoff value regardless of specimens.

Further, a group scatter diagram was created from all analyzed specimens using the expression scores of 7 genes for the above-described 7 kinds of genes for differentiating BSCE. As a result, as shown in Figure 7 (J), the average value of the non-BSCE specimen group was 0.256, and the average value of the BSCE specimen group was 12.531. There was a statistically significant difference (P=1.28×10⁻¹¹) between both groups.

Next, Figure 7 (K) shows the results of creating a group scatter diagram separately for each specimen type. For surgical specimens, the average value of the non-BSCE specimen group was -2.845, and the average value of the BSCE specimen group was 15.358. There was a statistically significant difference (P=0.0003) between both groups. For biopsy specimens, the average value of the non-BSCE specimen group was 0.617, and the average value of the BSCE specimen group was 12.544. There was a statistically significant difference (P=0.0037) between both groups. For FFPE tissue specimens, the average value of the non-BSCE group was 6.748, and the average value of the BSCE specimen group was 11.001. There was a statistically significant difference (P=0.0010) between both groups.

The above results indicated that BSCE markers consisting of the group of 7 kinds of genes for differentiating BSCE shown in NOs: R-1 to R-5 and NOs: R-14 and R-15 in Table 1 can also differentiate the BSCE specimen group and the non-BSCE specimen group.

### <Example 10>

### (Object)

A group of genes for differentiating BSCE is narrowed down from BSCE markers. [5]

### (Method and Results)

Priority ranking was made using the 70 kinds of genes for differentiating BSCE identified in Example 1 in consideration of the difference in the average value between the BSCE specimen group and the non-BSCE specimen group, standard deviation, P value of t-test, score values for the BSCE specimen group and clusters created by cluster analysis. It was revealed that even a group of 10 kinds of genes for differentiating BSCE, further including 2 kinds of selected genes for differentiating BSCE represented by NOs: R-14 and R-15 in Table 1, in addition to the group of 8 kinds of genes for differentiating BSCE represented by NOs: R-1 to R-8 in Table 1 described in Example 6, can differentiate the BSCE specimen group and the non-BSCE specimen group.

The basic procedures were conducted as in Example 5. An ROC curve was created from the sum obtained by extracting gene expression data of the 10 kinds of genes for differentiating BSCE to calculate the sum (hereinafter referred to as "expression scores of 10' genes"), i.e., values of expression scores of 10' genes. As a result, as shown in Figure 8 (A), AUC was 1.0000 for surgical specimens alone. In addition, as shown in Figure 8 (C), AUC was 0.9437 for surgical specimens and biopsy specimens, and as shown in Figure 8 (E), AUC was 0.9851 for surgical specimens and FFPE tissue specimens.

Next, a sensitivity-specificity curve was created for verification. As a result, as shown in Figure 8 (B), both sensitivity and specificity were 1.0000 for surgical specimens alone when the cutoff value was set to 0.7590. In addition, as shown in Figure 8 (D), when the cutoff value was set to 6.1962, sensitivity was 0.9333 and specificity was 0.9774 for surgical specimens and biopsy specimens. Further, as shown in Figure 8 (F), when the cutoff value was set to 10.2696, sensitivity was 0.9000 and specificity was 0.9474 for surgical specimens and FFPE tissue specimens.

Subsequently, surgical specimens alone (Figure 8 (G)), surgical specimens and biopsy specimens (Figure 8 (H)), and surgical specimens and FFPE tissue specimens (Figure 8 (I)) were re-aligned in the ascending order of the expression scores of 10' genes using the expression scores of 10' genes for the above-described 10 kinds of genes for differentiating BSCE. As a result, it was revealed that the BSCE specimen group and the non-BSCE specimen group can be differentiated by setting a certain cutoff value regardless of specimens.

Further, a group scatter diagram was created from all analyzed specimens using the expression scores of 10' genes for the above-described 10 kinds of genes for differentiating BSCE. As a result, as shown in Figure 8 (J), the average value of the non-BSCE specimen group was 1.254, and the average value of the BSCE specimen group was 16.308. There was a statistically significant difference (P=6.69×10⁻¹¹) between both groups.

Next, Figure 8 (K) shows the results of creating a group scatter diagram separately for each specimen type. For surgical specimens, the average value of the non-BSCE specimen group was -2.502, and the average value of the BSCE specimen group was 20.963. There was a statistically significant difference (P=0.0007) between both groups. For biopsy specimens, the average value of the non-BSCE specimen group was 1.671, and the average value of the BSCE specimen group was 15.510. There was a statistically significant difference (P=0.0040) between both groups. For FFPE tissue specimens, the average value of the non-BSCE group was 9.966, and the average value of the BSCE specimen group was 14.292. There was a statistically significant difference (P=0.0039) between both groups.

The above results indicated that BSCE markers consisting of the group of 10 kinds of genes for differentiating BSCE shown in NOs: R-1 to R-8 and NOs: R-14 and R-15 in Table 1 can also differentiate the BSCE specimen group and the non-BSCE specimen group.

### <Example 11>

### (Object)

A group of genes for differentiating BSCE is narrowed down from BSCE markers. [6]

### (Method and Results)

Priority ranking was made using the 70 kinds of genes for differentiating BSCE identified in Example 1 in consideration of the difference in the average value between the BSCE specimen group and the non-BSCE specimen group, standard deviation, P value of t-test, score values for the BSCE specimen group and clusters created by cluster analysis. It was revealed that even a group of 12 kinds of genes for differentiating BSCE, further including 2 kinds of selected genes for differentiating BSCE represented by NOs: R-14 and R-15 in Table 1 in addition to the group of 10 kinds of genes for differentiating BSCE represented by NOs: R-1 to R-5 and NOs: R-9 to R-13 in Table 1 described in Example 7, can differentiate the BSCE specimen group and the non-BSCE specimen group.

The basic procedures were conducted as in Example 5. An ROC curve was created from the sum obtained by extracting gene expression data of the 12 kinds of genes for differentiating BSCE to calculate the sum (hereinafter referred to as "expression scores of 12 genes"), i.e., values of expression scores of 12 genes. As a result, as shown in Figure 9 (A), AUC was 1.0000 for surgical specimens alone. In addition, as shown in Figure 9 (C), AUC was 0.9772 for surgical specimens and biopsy specimens, and as shown in Figure 9 (E), AUC was 0.9974 for surgical specimens and FFPE tissue specimens.

Next, a sensitivity-specificity curve was created for verification. As a result, as shown in Figure 9 (B), both sensitivity and specificity were 1.0000 for surgical specimens alone when the cutoff value was set to 1.4000. In addition, as shown in Figure 9 (D), when the cutoff value was set to 6.6782, sensitivity was 0.9333 and specificity was 0.9915 for surgical specimens and biopsy specimens. Further, as shown in Figure 9 (F), when the cutoff value was set to 6.7474, sensitivity was 0.9500 and specificity was 1.0000 for surgical specimens and FFPE tissue specimens.

Subsequently, surgical specimens alone (Figure 9 (G)), surgical specimens and biopsy specimens (Figure 9 (H)), and surgical specimens and FFPE tissue specimens (Figure 9 (I)) were re-aligned in the ascending order of the expression scores of 12 genes using the expression scores of 12 genes for the above-described 12 kinds of genes for differentiating BSCE. As a result, it was revealed that the BSCE specimen group and the non-BSCE specimen group can be differentiated by setting a certain cutoff value regardless of specimens.

Further, a group scatter diagram was created from all analyzed specimens using the expression scores of 12 genes for the above-described 12 kinds of genes for differentiating BSCE. As a result, as shown in Figure 9 (J), the average value of the non-BSCE specimen group was -0.485, and the average value of the BSCE specimen group was 15.619. There was a statistically significant difference (P=9.03 × 10⁻¹¹) between both groups.

Next, Figure 9 (K) shows the results of creating a group scatter diagram separately for each specimen type. For surgical specimens, the average value of the non-BSCE specimen group was -3.692, and the average value of the BSCE specimen group was 22.123. There was a statistically significant difference (P=0.0001) between both groups. For biopsy specimens, the average value of the non-BSCE specimen group was -0.072, and the average value of the BSCE specimen group was 16.950. There was a statistically significant difference (P=0.0024) between both groups. For FFPE tissue specimens, the average value of the non-BSCE group was 4.490, and the average value of the BSCE specimen group was 11.297. There was a statistically significant difference (P=0.0001) between both groups.

The above results indicated that BSCE markers consisting of the group of 12 kinds of genes for differentiating BSCE shown in NOs: R-1 to R-5, NOs: R-9 to R-13, and NOs: R-14 and R-15 in Table 1 can also differentiate the BSCE specimen group and the non-BSCE specimen group.

### <Example 12>

### (Object)

A group of genes for differentiating BSCE is narrowed down from BSCE markers. [7]

### (Method and Results)

Priority ranking was made using the 70 kinds of genes for differentiating BSCE identified in Example 1 in consideration of the difference in the average value between the BSCE specimen group and the non-BSCE specimen group, standard deviation, P value of t-test, score values for the BSCE specimen group and clusters created by cluster analysis. It was revealed that even a group of 15 kinds of genes for differentiating BSCE, further including 2 kinds of selected genes for differentiating BSCE represented by NOs: R-14 and R-15 in Table 1 in addition to the group of 13 kinds of genes for differentiating BSCE represented by NOs: R-1 to R-13 in Table 1 described in Example 7, can differentiate the BSCE specimen group and the non-BSCE specimen group.

The basic procedures were conducted as in Example 5. An ROC curve was created from the sum obtained by extracting gene expression data of the 15 kinds of genes for differentiating BSCE to calculate the sum (hereinafter referred to as "expression scores of 15 genes"), i.e., expression scores of 15 genes. As a result, as shown in Figure 10 (A), AUC was 1.0000 for surgical specimens alone. In addition, as shown in Figure 10 (C), AUC was 0.9556 for surgical specimens and biopsy specimens, and as shown in Figure 10 (E), AUC was 0.9939 for surgical specimens and FFPE tissue specimens.

Next, a sensitivity-specificity curve was created for verification. As a result, as shown in Figure 10 (B), both sensitivity and specificity were 1.0000 for surgical specimens alone when the cutoff value was set to 1.0559. In addition, as shown in Figure 10 (D), when the cutoff value was set to 8.8317, sensitivity was 0.9333 and specificity was 0.9915 for surgical specimens and biopsy specimens. Further, as shown in Figure 10 (F), when the cutoff value was set to 9.2942, sensitivity was 0.9500 and specificity was 0.9825 for surgical specimens and FFPE tissue specimens.

Subsequently, surgical specimens alone (Figure 10 (G)), surgical specimens and biopsy specimens (Figure 10 (H)), and surgical specimens and FFPE tissue specimens (Figure 10 (I)) were re-aligned in the ascending order of the expression scores of 15 genes using the expression scores of 15 genes for the above-described 15 kinds of genes for differentiating BSCE. As a result, it was revealed that the BSCE specimen group and the non-BSCE specimen group can be differentiated by setting a certain cutoff value regardless of specimens.

Further, a group scatter diagram was created from all analyzed specimens using the expression scores of 15 genes for the above-described 15 kinds of genes for differentiating BSCE. As a result, as shown in Figure 10 (J), the average value of the non-BSCE specimen group was 0.513, and the average value of the BSCE specimen group was 19.396. There was a statistically significant difference (P=2.11×10⁻¹⁰) between both groups.

Next, Figure 10 (K) shows the results of creating a group scatter diagram separately for each specimen type. For surgical specimens, the average value of the non-BSCE specimen group was -3.349, and the average value of the BSCE specimen group was 27.728. There was a statistically significant difference (P=0.0003) between both groups. For biopsy specimens, the average value of the non-BSCE specimen group was 0.982, and the average value of the BSCE specimen group was 19.917. There was a statistically significant difference (P=0.0028) between both groups. For FFPE tissue specimens, the average value of the non-BSCE group was 7.708, and the average value of the BSCE specimen group was 14.588. There was a statistically significant difference (P=0.0003) between both groups.

The above results indicated that BSCE markers consisting of the group of 15 kinds of genes for differentiating BSCE shown in NOs: R-1 to R-13 and NOs: R-14 to R-15 in Table 1 can also differentiate the BSCE specimen group and the non-BSCE specimen group.

## Claims

1. A method for assisting differentiating contraction of basaloid squamous cell carcinoma of the esophagus, comprising:
a measurement step of measuring expression levels of 5 genes comprising nucleotide sequences shown in SEQ ID NOs: 141 to 145, respectively, per unit amount of samples collected from a test subject and a healthy subject or a group of healthy subjects, thereby obtaining measurement values thereof;
a calculation step of, based on the measurement values obtained in the measurement step, calculating expression ratios for each gene based on the measurement values of the test subject and the healthy subject or the group of healthy subjects, thereby obtaining a sum of the expression ratios, or calculating an average measurement value from the respective measurement values of all of the genes measured in the measurement step for the test subject and the group of healthy subjects; and
a determination step of, based on the values obtained in the calculation step, determining that the test subject is highly likely to be affected with basaloid squamous cell carcinoma of the esophagus in a case in which the sum exceeds a given cutoff value resulting from an ROC curve, or in a case in which the average measurement value of the test subject is statistically significantly larger than the average measurement value of the group of healthy subjects.

2. The method according to claim 1, wherein the measurement step further comprises measuring the expression level per unit amount for one or more genes comprising the nucleotide sequences shown in SEQ ID NOs: 146 to 148, respectively.

3. The method according to claim 1 or 2, wherein the measurement step further comprises measuring the expression level per unit amount for one or more genes comprising the nucleotide sequences shown in SEQ ID NOs: 149 to 153, respectively.

4. The method according to any one of claims 1 to 3, wherein the measurement step further comprises measuring the expression level per unit amount for one or more genes comprising the nucleotide sequences shown in SEQ ID NOs: 154 to 210, respectively.

5. The method according to any one of claims 1 to 4, wherein the expression level of the gene per unit amount is measured as an absolute amount or a relative amount of mRNA of the gene or a nucleotide fragment thereof or a protein encoded by the gene or a peptide fragment thereof per unit amount.

6. The method according to any one of claims 1 to 5, wherein in the determination step the average measurement value of the test subject is twice or more than the average measurement value of the group of healthy subjects.

7. A reagent for detecting basaloid squamous cell carcinoma of the esophagus, wherein said reagent contains a group of probes consisting of nucleotide sequences shown in SEQ ID NOs: 141 to 145.

8. The reagent for detecting basaloid squamous cell carcinoma of the esophagus according to claim 7, wherein said reagent further contains one or more probes consisting of nucleotide sequences shown in SEQ ID NOs: 146 to 148.

9. The reagent for detecting basaloid squamous cell carcinoma of the esophagus according to claim 7 or 8, wherein said reagent further contains one or more probes consisting of nucleotide sequences shown in SEQ ID NOs: 149 to 153.

10. The reagent for detecting basaloid squamous cell carcinoma of the esophagus according to any one of claims 7 to 9, wherein said reagent further contains one or more probes consisting of nucleotide sequences shown in SEQ ID NOs: 154 to 210.

11. A kit for detecting basaloid squamous cell carcinoma of the esophagus, wherein said kit contains the reagent for detecting basaloid squamous cell carcinoma of the esophagus according to any one of claims 7 to 10.

## Patentansprüche

1. Verfahren zum Unterstützen der Differenzierung einer Erkrankung mit basaloidem Plattenepithelkarzinoms der Speiseröhre, umfassend:
einen Messschritt zum Messen der Expressionspegels von 5 Genen, umfassend Nukleotidsequenzen, die entsprechend in SEQ ID NO: 141 bis 145 gezeigt sind, je Einheitsmenge von Proben, entnommen von einem Testsubjekt und einem gesunden Subjekt oder von einer Gruppe gesunder Subjekte, wodurch von diesen Messwerte erhalten werden;
einen Berechnungsschritt zum Berechnen, auf der Grundlage der im Messschritt erhaltenen Messwerte, von Expressionsverhältnissen für jedes Gen auf der Grundlage der Messwerte des Testsubjekts und des gesunden Subjekts oder der Gruppe gesunder Subjekte, dadurch Erhalten einer Summe der Expressionsverhältnisse oder Berechnen eines mittleren Messwertes aus den jeweiligen Messwerten aller im Messschritt gemessenen Gene für das Testsubjekt und die Gruppe gesunder Subjekte; und
einen Bestimmungsschritt zum Bestimmen, basierend auf den im Berechnungsschritt erhaltenen Werten, dass das Testsubjekt mit hoher Wahrscheinlichkeit von basaloidem Plattenepithelkarzinom der Speiseröhre betroffen ist, in einem Fall, in dem die Summe einen bestimmten Grenzwert überschreitet, der aus einer ROC-Kurve resultiert, oder in einem Fall, in dem der durchschnittliche Messwert des Testsubjekts statistisch signifikant größer ist als der durchschnittliche Messwert der Gruppe gesunder Subjekte.

2. Verfahren nach Anspruch 1, wobei der Messschritt ferner das Messen des Expressionspegels je Einheitsmenge für ein oder mehrere Gene umfasst, umfassend die entsprechend in SEQ ID NO: 146 bis 148 gezeigten Nukleotidsequenzen.

3. Verfahren nach Anspruch 1 oder 2, wobei der Messschritt ferner das Messen des Expressionspegels je Einheitsmenge für ein oder mehrere Gene umfasst, umfassend die entsprechend in SEQ ID NO: 149 bis 153 gezeigten Nukleotidsequenzen.

4. Verfahren nach einem der Ansprüche 1 bis 3, wobei der Messschritt ferner das Messen des Expressionspegels je Einheitsmenge für ein oder mehrere Gene umfasst, umfassend die entsprechend in SEQ ID NO: 154 bis 210 gezeigten Nukleotidsequenzen.

5. Verfahren nach einem der Ansprüche 1 bis 4, wobei der Expressionspegel des Gens je Einheitsmenge als absolute Menge oder als relative Menge an mRNA des Gens oder eines Nukleotidfragments davon oder eines Proteins, für das durch das Gen kodiert wird, oder eines Peptidfragments davon je Einheitsmenge gemessen wird.

6. Verfahren nach einem der Ansprüche 1 bis 5, wobei im Bestimmungsschritt der durchschnittliche Messwert des Testsubjekts doppelt so groß ist wie der durchschnittliche Messwert der Gruppe gesunder Subjekte.

7. Reagens zum Nachweisen von basaloidem Plattenepithelkarzinom der Speiseröhre, wobei das Reagens eine Gruppe von Sonden enthält, bestehend aus Nukleotidsequenzen, die in SEQ ID NO: 141 bis 145 gezeigt sind.

8. Reagens zum Nachweisen von basaloidem Plattenepithelkarzinom der Speiseröhre nach Anspruch 7, wobei das Reagens ferner eine Gruppe von einer oder mehreren Sonden enthält, bestehend aus Nukleotidsequenzen, die in SEQ ID NO: 146 bis 148 gezeigt sind.

9. Reagens zum Nachweisen von basaloidem Plattenepithelkarzinom der Speiseröhre nach Anspruch 7 oder 8, wobei das Reagens ferner eine Gruppe von einer oder mehreren Sonden enthält, bestehend aus Nukleotidsequenzen, die in SEQ ID NO: 149 bis 153 gezeigt sind.

10. Reagens zum Nachweisen von basaloidem Plattenepithelkarzinom der Speiseröhre nach einem der Ansprüche 7 bis 9, wobei das Reagens ferner eine Gruppe von einer oder mehreren Sonden enthält, bestehend aus Nukleotidsequenzen, die in SEQ ID NO: 154 bis 210 gezeigt sind.

11. Kit zum Nachweisen von basaloidem Plattenepithelkarzinom der Speiseröhre, wobei das Kit das Reagens zum Nachweisen von basaloidem Plattenepithelkarzinom der Speiseröhre nach einem der Ansprüche 7 bis 10 enthält.

## Revendications

1. Procédé pour assister la contraction de différenciation d'un carcinome à cellules squameuses basaloïdes de l'œsophage, comprenant :
une étape de mesure où l'on mesure des niveaux d'expression de 5 gènes comprenant des séquences nucléotidiques présentées dans les SEQ ID NO: 141 à 145, respectivement, par quantité unitaire d'échantillons collectés provenant d'un sujet de test et d'un sujet sain ou d'un groupe de sujets sains, obtenant ainsi des valeurs de mesure de ceux-ci ;
une étape de calcul où l'on calcule, sur la base des valeurs de mesure obtenues dans l'étape de mesure, des rapports d'expression pour chaque gène sur la base des valeurs de mesure du sujet de test et du sujet sain ou du groupe de sujets sains, obtenant ainsi une somme des rapports d'expression, ou où l'on calcule une valeur de mesure moyenne à partir des valeurs de mesure respectives de tous les gènes mesurées dans l'étape de mesure pour le sujet de test et le groupe de sujets sains ; et
une étape de détermination où l'on détermine, sur la base des valeurs obtenues dans l'étape de calcul, que le sujet de test a une forte probabilité d'être affecté par un carcinome à cellules squameuses basaloïdes de l'œsophage dans un cas dans lequel la somme dépasse une valeur seuil donnée résultant d'une courbe ROC, ou dans un cas dans lequel la valeur de mesure moyenne du sujet de test est statistiquement significativement plus grande que la valeur de mesure moyenne du groupe de sujets sains.

2. Procédé selon la revendication 1, l'étape de mesure comprenant en outre la mesure du niveau d'expression par quantité unitaire pour un ou plusieurs gènes comprenant les séquences nucléotidiques présentées dans les SEQ ID NO: 146 à 148, respectivement.

3. Procédé selon la revendication 1 ou 2, l'étape de mesure comprenant en outre la mesure du niveau d'expression par quantité unitaire pour un ou plusieurs gènes comprenant les séquences nucléotidiques présentées dans les SEQ ID NO: 149 à 153, respectivement.

4. Procédé selon l'une quelconque des revendications 1 à 3, l'étape de mesure comprenant en outre la mesure du niveau d'expression par quantité unitaire pour un ou plusieurs gènes comprenant les séquences nucléotidiques présentées dans les SEQ ID NO: 154 à 210, respectivement.

5. Procédé selon l'une quelconque des revendications 1 à 4, le niveau d'expression du gène par quantité unitaire étant mesuré comme une quantité absolue ou une quantité relative d'ARNm du gène ou d'un fragment nucléotidique correspondant ou d'une protéine codée par le gène ou d'un fragment peptidique correspondant par quantité unitaire.

6. Procédé selon l'une quelconque des revendications 1 à 5, dans lequel dans l'étape de détermination la valeur de mesure moyenne du sujet de test est le double ou plus de la valeur de mesure moyenne du groupe de sujets sains.

7. Réactif pour la détection d'un carcinome à cellules squameuses basaloïdes de l'œsophage, ledit réactif contenant un groupe de sondes constituées de séquences nucléotidiques présentées dans les SEQ ID NO: 141 à 145.

8. Réactif pour la détection d'un carcinome à cellules squameuses basaloïdes de l'œsophage selon la revendication 7, ledit réactif contenant en outre une ou plusieurs sondes constituées de séquences nucléotidiques présentées dans les SEQ ID NO: 146 à 148.

9. Réactif pour la détection d'un carcinome à cellules squameuses basaloïdes de l'œsophage selon la revendication 7 ou 8, ledit réactif contenant en outre une ou plusieurs sondes constituées de séquences nucléotidiques présentées dans les SEQ ID NO: 149 à 153.

10. Réactif pour la détection d'un carcinome à cellules squameuses basaloïdes de l'œsophage selon l'une quelconque des revendications 7 à 9, ledit réactif contenant en outre une ou plusieurs sondes constituées de séquences nucléotidiques présentées dans les SEQ ID NO: 154 à 210.

11. Kit pour la détection d'un carcinome à cellules squameuses basaloïdes de l'œsophage, ledit kit contenant le réactif pour la détection d'un carcinome à cellules squameuses basaloïdes de l'œsophage selon l'une quelconque des revendications 7 à 10.
